(19) Europäisches Patentamt
European Patent Office
Office européen des brevets

(11) EP 2 308 858 A1

(12) EUROPEAN PATENT APPLICATION

(43) Date of publication:
**13.04.2011 Bulletin 2011/15**

(21) Application number: **10195739.7**

(22) Date of filing: **17.03.2008**

(51) Int Cl.:
*C07D 261/04* (2006.01) *C07D 413/12* (2006.01)
*A01N 43/80* (2006.01) *A01P 7/02* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **29.03.2007 JP 2007087293**

(62) Document number(s) of the earlier application(s) in accordance with Art. 76 EPC:
**08738852.6 / 2 139 874**

(71) Applicant: **Sumitomo Chemical Company, Limited
Tokyo 104-8260 (JP)**

(72) Inventors:
- **Ihara, Hideki
Osaka-shi Osaka 556-0016 (JP)**
- **Kumamoto, Koji
Toyonaka-shi Osaka 560-0021 (JP)**

(74) Representative: **Vossius & Partner
Siebertstrasse 4
81675 München (DE)**

Remarks:
This application was filed on 17-12-2010 as a divisional application to the application mentioned under INID code 62.

# (54) Isoxazoline Compounds and their Use in Pest Control

(57) The present invention relates to an isoxazoline compound represented by the formula (1):

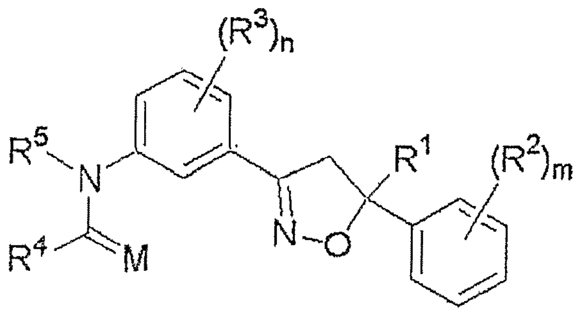

(1)

wherein the substituents are as defined in the description, which has a controlling activity against pests.

EP 2 308 858 A1

## Description

TECHNICAL FIELD

The present invention relates to an isoxazoline compound and its use in pest control.

BACKGROUND ART

International Publication No. W02005-51932 pamphlet describes a certain kind of isoxazoline compounds as the active ingredients of agricultural and horticultural fungicides and insecticides.

DISCLOSURE OF THE INVENTION

The present inventors investigated in order to find a compound having controlling activity against pests, and consequently found that an isoxazoline compound represented by the formula (1) shown below has controlling effect on pests, whereby the present invention has been accomplished.
That is, the present invention provides the following.

1. An isoxazoline compound represented by the formula (1):

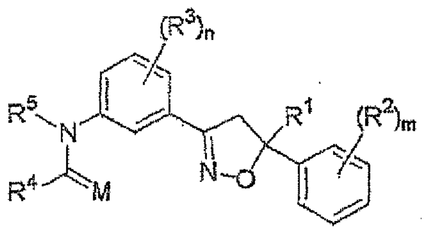

(1)

wherein $R^1$ is a $C_1$-$C_4$ haloalkyl group,
$R^2$ is a $C_1$-$C_6$ alkyl group unsubstituted or substituted with one or more halogen atoms, a $C_1$-$C_6$ alkoxy group unsubstituted or substituted with one or more halogen atoms, a halogen atom, a $C_1$-$C_6$ alkylsulfenyl groups a $C_1$-$C_5$ alkylsulfinyl group, a $C_1$-$C_6$ alkylsulfonyl group, a nitro group or a cyano group,
$R^3$ is a $C_1$-$C_6$ alkyl group, a $C_1$-$C_6$ alkoxy group, a nitro group or a halogen atom,
m is an integer of 0 to 5,
n is an integer of 0 to 4,
M is an oxygen atom or a sulfur atom,
$R^4$ is a hydrogen atom; a $C_1$-$C_{12}$ alkyl group unsubstituted or substituted with one or more halogen atoms; a ($C_2$-$C_5$ alkoxycarbonyl)$C_1$-$C_{12}$ alkyl group; a $C_2$-$C_{12}$ cyanoalkyl group; a $C_3$-$C_{12}$ cycloalkyl group unsubstituted or substituted with one or more halogen atoms; a ($C_3$-$C_6$ cycloalkyl)$C_1$-$C_6$ alkyl group; a ($C_1$-$C_6$ alkoxy)$C_2$-$C_6$ alkyl group; a $C_2$ - $C_{12}$ alkenyl group unsubstituted or substituted with one or more halogen atoms; a $C_2$-$C_{12}$ alkynyl group unsubstituted or substituted with one or more halogen atoms; a $C_1$-$C_6$ alkoxy group unsubstituted or substituted with one or more halogen atoms; a ($C_1$-$C_6$ alkoxy)$C_1$-$C_6$ alkoxy group; a $C_3$-$C_6$ alkenyloxy group unsubstituted or substituted with one or more halogen atoms; a benzyloxy group; a phenyl ($C_2$-$C_5$)alkenyl group; a ($C_1$-$C_6$ alkylamino) $C_1$-$C_6$ alkyl group; a (di($C_1$-$C_6$ alkcyl)amino)$C_1$-$C_6$ alkyl group; a group represented by the following formula:

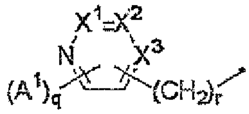

[wherein $A^1$ is a $C_1$-$C_4$ alkyl group unsubstituted or substituted with one or more halogen atoms, a $C_1$-$C_4$ alkoxy group unsubstituted or substituted with one or more halogen atoms, a halogen atom or a cyano group,
q is an integer of 0 to 3,

r is an integer of 0 to 2, and
$X^1$, $X^2$ and $X^3$ represent a combination of one nitrogen atom and two CH groups, or a combination of three CH groups] a group, represented by the following formula:

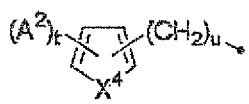

[wherein $A^2$ is a $C_1$,-$C_4$ alkyl group, a halogen atom or a nitro group,
t is an integer of 0 to 3,
u is an integer of 0 to 2, and
$x^4$ is an oxygen atom, a sulfur atom or NH] ; a group represented by the following formula:

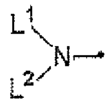

[wherein $L^1$ is a $C_1$-$C_6$ alkyl group unsubstituted or substituted with one or more halogen atoms, or a $C_3$-$C_6$ cycloalkyl group, and
$L^2$ is a hydrogen atom or a $C_1$-$C_6$ alkyl group unsubstituted or substituted with one or more halogen atoms, or $L^1$ and $L^2$, when taken together, represent a $C_2$-$C_9$ alkanediyl group, a $CH_2CH_2CH$ ($CH_3$) $CH_2CH_2$ group or a $CH_2CH_2OCH_2CH_2$ group]; a phenyl group; a phenyl($C_1$-$C_4$)alkyl group; a phenoxy group; a phenoxy ($C_1$-$C_4$) alkyl group; a phenylamino group or a phenyl ($C_1$-$C_2$) alkylamino group, wherein the benzene ring of any of said phenyl group, said phenyl ($C_1$-$C_4$) alkyl group, said phenoxy group, said phenoxy ($C_1$-$C_4$) alkyl group, said phenylamino group and said phenyl ($C_1$-$C_2$) alkylamine group may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_5$ alkylsulfinyl groups, $C_1$-$C_6$ alkylsulfonyl groups, $C_2$-$C_6$ alkoxycarbonyl groups and benzoyl group, and
$R_5$ is a hydrogen atom, a $C_1$-$C_{12}$ alkyl group, a $C_3$-$W_{12}$ cycloalkyl group, a ($C_3$-$C_6$ cycloalkyl) $C_1$-$C_6$ alkyl group, a $C_2$-$C_6$ alkoxyalkyl group, a $C_3$-$C_{12}$ alkenyl group, a $C_3$-$C_{12}$ alkynyl group, a $C_1$-$C_6$ acyl group, a $C_2$-$C_8$ cyanoalkyl group, a nitromethyl group, a $C_2$-$C_6$ alkoxycarbonyl group, a $C_3$-$C_8$ alkoxycarbonylalkyl group, a phenyl($C_1$-$C_4$) alkyl group or a benzoyl group, wherein the benzene ring of said phenyl ($C_1$-$C_4$) alkyl group or said benzoyl group may be substituted with 1 to 5 groups independently selected from the group consisting of $C_1$.$C_6$ alkyl groups unsubstituted or substituted with one or more halogen atoms, $C_1$-$C_6$ alkoxy groups unsubstituted or substituted with one or more halogen atoms, halogen atoms, nitro group and cyano group (said isoxazoline compound is hereinafter referred to as the present compound).

2. The isoxazoline compound according to the above item 1, wherein n is 0 in the formula (1).
3. The isoxazoline compound according to the above item 1, wherein in the formula (1), n is 1 and $R^3$ is a $C_1$-$C_6$ alkyl group as a substituent at the 6-position.
4. The isoxazoline compound according to the above item 1, wherein in the formula (1), n is 1 and $R^3$ is a methyl group as a substituent at the 6-position.
5. The isoxazoline compound according to the above item 1, wherein in the formula (1), n is 1 and $R^3$ is a halogen atom as a substituent at the 6-position.
6. The isoxazoline compound according to the above item 1, wherein in the formula (1), n is 1 and $R^3$ is a $C_1$-$C_4$ alkoxy group unsubstituted or substituted with one or more halogen atoms which is a substituent at the 6-position.
7. The isoxazoline compound according to any one of the above items 1 to 6, wherein in the formula (1), $R^4$ is a $C_1$-$C_{12}$ alkyl group unsubstituted or substituted with one or more halogen atoms.
8. The isoxazoline compound according to any one of the above items 1 to 6, wherein in the formula (1), $R^4$ is a $C_2$-$C_{12}$ alkenyl group unsubstituted or substituted with one or more halogen atoms.
9. The isoxazoline compound according to any one of the above items 1 to 6, wherein in the formula (1), $R^4$ is a $C_1$-$C_6$ alkoxy group unsubstituted or substituted with one or more halogen atoms.
10. The isoxazoline compound according to any one of the above items 1 to 6, wherein in the formula (1), $R^4$ is a

phenyl group unsubstituted or substituted with 1 to 5 groups independently selected from the following group X:

group X: $C_1$-$C_6$ alkyl groups unsubstituted or substituted with one or more halogen atoms, $C_1$-$C_6$, alkoxy groups or substituted with one or more halogen atoms, halogen atoms, nitro group, cyano group, $C_1$-$C_6$ alkylsulfenyl groups. $C_1$-$C_6$ alkylsulfinyl groups, $C_1$-$C_6$ alkylsulfonyl groups, $C_2$-$C_6$ alkoxycarbonyl groups and benzoyl group.

11. The isoxazoline compound according to the above item 1, wherein in the formula (1) , $R^1$ is a trifluoromethyl group, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom.
12. The isoxazoline compound according to the above item 1, wherein in the formula (1), $R^1$ is a trifluoromethyl group, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_5$ alkoxy groups unsubstituted or substituted with one or more halogen atoms, halogen atoms, nitro group, cyano group, $C_1$-$C_6$ alkylsulfenyl groups. $C_1$-$C_6$ alkylsulfinyl groups, $C_1$-$C_6$ alkylsulfonyl groups, $C_2$-$C_6$ alkoxycarbonyl groups and benzoyl group.

13. The isoxazoline compound according to the above item 1, wherein in the formula (1), $R^1$ is a trifluoromethyl group, m is 2, $R^2$S are chlorine atoms as substituents at the 3-position and 5-position, respectively, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom.
14. The isoxazoline compound according to the above item 1, wherein in the formula (1), $R^1$- is a trifluoromethyl groups, m is 2, $R^2$S are chlorine atoms as substituents at the 3-position and 5-position, respectively, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups. $C_1$-$C_6$ alkylsulfinyl groups, $C_1$-$C_6$ alkylsulfonyl groups, $C_2$-$C_5$ alkoxycarbonyl groups and benzoyl groups.

15. The isoxazoline compound according to the above item 1, wherein in the formula (1), $R^1$ is a trifluoromethyl group, n is 0 or 1, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom .
16. The isoxazoline compound according to the above item 1, wherein in the formula (1), $R^1$ is a trifluoromethyl group, n is 0 or 1, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups. $C_1$-$C_6$ alkylsulfinyl groups, $C_1$-$C_6$ alkylsulfonyl groups, $C_2$-$C_6$ alkoxycarbonyl groups and benzoyl group.

17. A composition for controlling pests, characterized by containing an isoxazoline compound represented by the formula (1) according to any one of the above items 1 to 16 as an active ingredient.
18. A method for controlling pests, characterized by applying an effective amount of an isoxazoline compound represented by the formula (1) according to any one of the above items 1 to 16 to the pests or a locus where the pests inhabit.
19. Use of an isoxazoline compound represented by the formula (1) according to any one of the above items 1 to 16 for controlling pests.
20. Use of an isoxasoline compound represented by the formula (1) according to any one of the above items 1 to 16 for producing a composition for controlling pests.

Advantages of the Invention

The present compound is useful as the active ingredient of a composition for controlling pests because of its controlling activity against pests.

BEST MODE FOR CARRYING OUT THE INVENTION

In the present invention, the $C_{1-}C_4$ haloalkyl group represented by $R^1$ includes, for example, trifluoromethyl group, difluoromethyl group, chlorodifluoromethyl group and pentafluoroethyl group.
The $C_1$-$C_6$ alkyl group unsubstituted or substituted with one or more halogen atoms which is represented by $R^2$ includes, for example, methyl group, ethyl group, propyl group, isopropyl group, tert-butyl group and trifluoromethyl groups.
The $C_1$-$C_6$ alkoxy group unsubstituted or substituted with one or more halogen atoms which is represented by $R^2$ includes, for example, methoxy group, ethoxy group, propoxy group, isopropoxy group, tert-butoxy group, trifluoromethoxy group and difluoromethoxy group.
The halogen atom represented by $R^2$ includes fluorine atom, chlorine atom, bromine atom and iodine atom.
The $C_1$-$C_6$ alkylsulfenyl group represented by $R^2$ includes, for example, methylthio group, ethylthio group, propylthio group and isopropylthio group.
The $C_1$-$C_6$ alkylsulfinyl group represented by $R^2$ includes, for example, methylsulfinyl group, ethylsulfinyl group, propylsulfinyl group and isopropylsulfinyl group.
The $C_{1-}C_6$ alkylsulfonyl group represented by $R^2$ includes, for example, methylsulfonyl group, ethylsulfonyl group, propylsulfonyl group and isopropylsulfonyl group.
The $C_1$-$C_6$ alkyl group represented by $R^3$ includes, for example, methyl group, ethyl group, propyl group and isopropyl group.
The $C_1$-$C_6$ alkoxy group represented by $R^3$ includes, for example, methoxy group, ethoxy group, propoxy group and isopropoxy group.
The halogen atom represented by $R^3$ includes fluorine atom, chlorine atom, bromine atom and iodine atom.
The $C_1$-$C_{12}$ alkyl group unsubstituted or substituted with one or more halogen atoms which is represented by $R^4$ includes, for example, methyl group, ethyl group, propyl group, isopropyl group, butyl group, isobutyl group, sec-butyl group, tart-butyl group, pentyl group, 1-methylbutyl group, 2-methylbutyl group, 3-methylbutyl group, I-ethylpropy, group, 1,1-dimethylpropyl group, 1,2-dimethylpropyl group, 2,2-group, hexyl group, 1-methylpentyl group, 2-methylpentyl group, 1,1-dimethylbutyl group, 1,3-dimethylbutyl group, heptyl group, octyl group, nonyl group, decyl group, undecyl group, dodecyl group, fluoromethyl group, chloromethyl group, bromomethyl group, iodomethyl group, difluoromethyl group, chlorofluoromethyl group, dichloromethyl group bromofluoromethyl group, trifluoromethyl group, chlorodifluoromethyl group, dichlorofluoromethyl group, trichloromethyl group, bromodifluoromethyl group, bromochlorofluoromethyl group, difluoroiodomethyl group, 2-fluoroethyl group, 2-chloroethyl group, 2-bromoethyl group, 2,2-difluoroethyl group, 2-chloro-2-fluoroethyl group, 2,2-dichloroethyl group, 2-bromo-2-fluoroethyl group, 2,2,2-trifluoroethyl group, 2-chloro-2,2-difluoroethyl group, 2,2-dichloro-2-fluoroethyl group, 2,2,2-trichloroethyl group, 2-bromo-2,2-difluoroethyl group, 1,1,2,2-tetrafluoroethyl group, 1,1,2,2,2-pentafluoroethyl group, 1-chloro-1,2,2,2-tetrafluoroethyl group, 2-chloro-1,1,2,2-tetrafluoroethyl group, 1,2-dichloro-1,2,2-trifluoroethyl group, 1-bromo-1,2,2,2-tetrafluoroethyl group, 2-bromo-1,1,2,2-tetrafluoroethyl group, 2-fluoropropyl group, 2-chloropropyl group, 2,3-dichloropropyl group, 3,3,3-trifluoropropyl group, 3-bromo-3,3-difluoropropyl group, 2,2,3,3-tetrafluoropropyl group, 2,2,3,3,3-pentafluoropropyl group, 1,1,2,3,3,3-hexafluoropropyl group, 1,1,2,2,3,3,3-heptafluoropropyl group, 2,3-diehloro-1,1,2,3,3-pentafluoropropyl group, 2-fluoro-1-methylethyl group, 2-chloro-l-methylethyl group, 2-bromo-1-methylethyl group, 2,2,2-trifluoro-1-(trifluoromethyl)ethyl group, 1,2,2,2-tetrafluoro-1-(trifluoromethyl)ethyl group and 1,1,2,2,3;3,4,4,4-nonafluorobutyl group.
The ($C_2$-$C_5$ alkoxycarbonyl) $C_1$-$C_{12}$ alkyl group represented by $R^4$ includes, for example, methoxycarbonylmethyl group, ethoxycarbonylmethyl group, propoxycarbonylmethyl group, isopropoxycarbonylmethyl group and tert-butoxycarbonylmethyl group.
The $C_2$-$C_{12}$ cyanoalkyl group represented by $R^4$ includes, for example, cyanomethyl group.
The $C_3$-$C_{12}$ cycloalkyl group unsubstituted or substituted with one or more halogen atoms which is represented by $R^4$ includes, for example, cyclopropyl group, 1-methylcyclopropyl group, 2-methylcyclopropyl group, 2,2-dimethylcyclopropyl group, 2,2,3,3-tetramethylcyclopropyl group, cyclobutyl group, cyclopentyl group, 1-methylcyclopentyl group, 2-methylcyclopentyl group, 3-methylcyclopentyl group, cyclohexyl group, 1-methylcyclohexyl group, 2-methylcyclohexyl group, 3-methylcyclohexyl group, 4-methylcyclohexyl group, cycloheptyl group, cyclooctyl group, 3-fluorocyclopropyl group, 2-chlorocyclopropyl group, 2,2-difluorocyclopropyl group, 2,2-dichlorocyclopropyl group, 2,2-dibromocyclopropyl group, 2,2-difluoro-1-methylcyclopropyl group, 2,2-dichloro-1-methylcyclopropyl group, 2,2,3,3-tetrafluorocyclobutyl group and 2-chloro-2,3,3-trifluorocyclobutyl group.
The ($C_3$-$C_6$ cycloalkyl) $C_{1-}C_6$ alkyl group represented by $R^4$ includes, for example, cyclopropylmethyl group,

(1-methylcyclopropyl)methyl group, cyclobutylmethyl group, cyclopentylmethyl group and cyclohexylmethyl group.

The ($C_1$-$C_6$ alkoxy)$C_2$-$C_6$ alkyl group represented by $R^4$ includes, for example, 2-methoxyethyl group, 2-ethoxyethyl group, 2-propoxyethyl group, 2-isopropoxyethyl group, 2-butoxyethyl group, 2-isobutoxyethyl group, 2-(sec-butoxy)ethyl group and 2-(tert-butoxy)ethyl groups.

The $C_2$-$C_{12}$ alkenyl group unsubstituted or substituted with one or more halogen atoms which is represented by $R^4$ includes, for example, vinyl group, 1-propenyl group, 2-propenyl group, 1-methylethenyl group, 2-butenyl group, 1-methyl-2-propenyl group, 2-methyl-2-propenyl group, 2-pentenyl group, 2-methyl-2-butenyl group, 3-methyl-2-butenyl group, 2-ethyl-2-propenyl group, 1,1-dimethyl-2-propenyl group, 2-hexenyl group, 2-methyl-2-pentenyl group, 2,4-dimethyl-2,6-heptadienyl group, 3,7-dimethyl-2,6-octadienyl group, 2,2-dichlorovinyl group, 2-fluoro-2-propenyl group, 2-chloro-2-propenyl group, 3-chloro-2-propenyl group, 2-bromo-2-propenyl group, 3-bromo-2-propenyl group, 3,3-difluoro-2-propenyl group, 2,3-dichloro-2-propenyl group, 3,3-dichloro-2-propenyl group, 2,3-dibromo-2-propenyl group, 2,3,3-trifluoro-2-propenyl group, 2,3,3-trichloro-2-propenyl group', 1-(trifluaromethyl) ethenyl group, 3-chloro-2-butenyl group, 3-bromo-2-butenyl group, 4,4-difluoro-3-butenyl group, 3,4,4-trifluoro-3-butenyl group, 3-chloro-3,4,4-trifluoro-2-butenyl group and 3-bromo-2-methyl-2-propenyl group.

The $C_2$-$C_{12}$ alkynyl group unsubstituted or substituted with one or more halogen atoms which is represented by $R^4$ includes, for example, ethynyl group, 1-propynyl group, 2-propynyl group, 2-butynyl group, 1-methyl-2-propynyl group, 2-pentynyl group, 1-methyl-2-butynyl group, 1,1-dimethyl-2-propynyl group, 2-hexynyl group, 2-chloroethynyl group, 2-bromoethynyl group, 2-iodoethynyl group, 3-chloro-2-propynyl group, 3-bromo-2-propynyl group and 3-iodo-2-propynyl group.

The $C_1$-$C_6$ alkoxy group unsubstituted or substituted with one or more halogen atoms which is represented by $R^4$ includes, for example, methoxy group, ethoxy group, propoxy group, isopropoxy group, butoxy group, isobutoxy group, sec-butoxy group, tert-butoxy group, pentyloxy group, hexyloxy group, difluoromethoxy group, trifluoromethoxy group, chlorodifluoromethoxy group, bromodifluoromethoxy group, 2-fluoroethoxy group, 2-chloroethoxy group, 2,2,2-trifluoroethoxy group, 1,1,2,2-tetrafluoroethoxy group, 2-chloro-1,1,2-trifluoroethoxy group, 2-bromo-1,1,2-trifluoroethoxy group, 1,1,2,2,2-pentafluoroethoxy group, 2,2-dichloro-1,1,2-trifluoroethoxy group, 2,2,2-trichloro-1,1-difluoroethoxy group, 2-bromo-1,1,2,2-tetrafluoroethoxy group, 2,2,3,3-tetrafluoropropoxy group, 1,1,2,3,3,3-hexafluoropropoxy group, 2,2,2-trifluoro-1-(trifluoromethyl)ethoxy group, 1,1,2,2,3,3,3-heptafluoropropoxy group and 2-bromo-1,1,2,3,3,3-hexafluoropropoxy group.

The ($C_1$-$C_6$ alkoxy) $C_1$-$C_6$ alkoxy group represented by $R^4$ includes, for example, 2-methoxyethoxy group, 2-ethoxyethoxy group, 2-propoxyethoxy group, 2-isopropoxyethoxy group and 2-tert-butoxyethoxy group.

The $C_3$-$C_5$ alkenyloxy group unsubstituted or substituted with one or more halogen atoms which is represented by $R^4$ includes, for example, 2-propenyloxy group.

The phenyl ($C_2$-$C_6$) alkenyl group represented by $R^4$ includes, for example, 2-phenylvinyl group.

The ($C_1$-$C_6$ alkylamino) $C_1$-$C_6$ alkyl group represented by $R^4$ includes, for example, (methylamino) methyl group, (ethylamino) methyl group, (methylamino)ethyl group and (ethylamino) ethyl group.

The (di($C_1$-$C_6$ alkyl) amino) $C_1$-$C_6$ alkyl group represented by $R^4$ includes, for example, (dimethylamino)methyl group, (diethylamino)methyl groups, (dimethylamino) ethyl group and (diethylamino)ethyl group.

The group for $R^4$ represented by the formula:

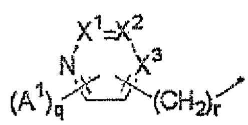

wherein $A^1$, q, r, $X^1$, $X^2$ and $X^3$ are as defined above, includes, for example, 2-pyridyl group, 3-methyl-2-pyridyl group, 4-methyl-2-pyridyl group, 5-methyl-2-pyridyl group, 6-methyl-2-pyridyl group. 3-fluoro-2-pyridyl group, 4-fluoro-2-pyridyl group, 5-fluoro-2-pyridyl group, 6-fluoro-2-pyridyl group, 3-chloro-2-pyridyl group, 4-chloro-2-pyridyl group, 5-chloro-2-pyridyl group, 6-chloro-2-pyridyl group, 3-bromo-2-pyridyl group, 4-bromo-2-pyridyl group, 5-bromo-2-pyridyl group, 6-bromo-2-pyridyl group, 3-pyridyl group, 2-methyl-3-pyridyl group, 4-methyl-3-pyridyl group, 5-methyl-3-pyridyl group, 6-methyl-3-pyridyl group, 2-fluoro-3-pyridyl group, 4-fluoro-3-pyridyl group, 5-fluoro-3-pyridyl group, 6-fluoro-3-pyridyl group, 2-chloro-3-pyridyl group, 4-chloro-3-pyridyl group, 5-chloro-3-pyridyl group, 6-chloro-3-pyridyl group, 2-methylthio-3-pyridylgroup, 4-methylthio-3-pyridyl group, 5-methylthio-3-pyridyl group, 6-methylthio-3-pyridyl group, 2-trifluoromethyl-3-pyridyl group, 4-trifluoromethyl-3-pyridyl group, 5-trifluoromethyl-3-pyridyl group, 6-trifluoromethyl-3-pyridyl group, 5,6-dichloro-3-pyridyl group, 2,6-dichloro-3-pyridyl group, 6-cyano-3-pyridyl group, 4-pyridyl group, 2-fluoro-4-pyridyl group, 3-fluoro-4-pyridyl group, 2-chloro-4-pyridyl group, 3-chloro-4-pyridyl group, 2,6-dichloro-4-pyridyl group, 3,5-dichloro-4-pyridyl group, 2-pyridylmethyl group, 3-pyridylmethyl group and 4-pyridylmethyl group.

The group for $R^4$ represented by the formula:

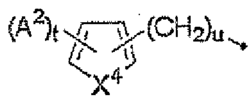

wherein $A^2$, t, u and $X^4$ are as defined above, includes, for example, 2-fury group, 2-methyl-2-furyl group, 5-bromo-2-furyl group, 5-nitro-2-furyl group, 3,4-dibromo-2-furyl group, 3-furyl group, 2-methyl-3-furyl group and 2,5-dimethyl-3-furyl group.

The group for $R^4$ represented by the formula:

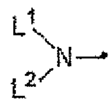

wherein $L^1$ and $L^2$ are as defined above, includes, for example, methylamino group, ethylamino group, propylamino group, isopropylamino group, butylamino group, isobutylamino group, tert-butylamino group, dimethylamino group, ethyl (methyl)amino group, diethylamino group, propyl(methyl)amino group, isopropyl(methyl)amino group, dipropylamino group, butyl (methyl) amino group, isobutyl (methyl) amino group, tert-butyl (methyl) amino group, pyrrolidin-1-yl group, 2-methylpyrrolidin1-yl group, 2-ethylpyrrolidin-1-y1 group, 2-propylpyrrolidin-1-yl group, 2-isopropylpyrrolidin-1-yl group, 2-tert-butylpyrrolidin-1-yl group, 3-methylpyrrolidin-1-yl group, 3-ethylpyrrolidin- 1-yl group, 2,5-dimethylpyrrolidin-1-yl group, 3,4-dimethylpyrrolidin-1-yl group, 3,3-dimethylpyrrolidin-1-yl group, piperidino group, 2-methylpiperidino group, 2-ethylpiperidino group, 2-propylpiperidino group, 2-isopropylpiperidino group, 2-tert-butylpiperidino group, 2-sec-butylpiperidino group, 3-methylpiperidino group, 3-ethylpiperidino group, 3-propylpiperidino group, 3-isopropylpiperidino group, 3-tert-butylpiperidino group, 3-sec-butylpiperidino group, 4-methylpiperidino group, 4-ethylpiperidino group, 4-propylpiperidino group, 4-isopropylpiperidino group, 4-tert-butylpiperidino group, 4- (trifluoromethyl) piperidino group, 2,6-dimethylpiperidino group, 2,4-dimethylpiperidino group, 2,5-dimethylpiperidino group, 3,5-dimethylpiperidino group, 2,2-dimethylpiperidino group, 3,3-dimethylpiperidino group, 4,4-dimethylpiperidino group, 3-ethyl-6-methylpiperidino group, 3-ethyl-5-methylpiperidino group, 3,5-diethylpiperidino group, 2,3 -dimethylpiperidino group, 3,3,5-trimethylpiperidino group, 2,3,5,6-tetramethylpiperidino group, 3,3,5,5-tetramethylpiperidino group, hexamethyleneimino group, 2-methylhexamethyleneimino group, 2-ethylhexamethyleneimino group, 3-methylhexamethyleneimino group, 3-ethylhexamethyleneimino group, 4-methylhexamethyleneimino group, 4-ethylhexamethyleneimino group, heptamethyleneimino group and morpholino group.

As the phenyl group represented by $R^4$ which may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group, there are exemplified phenyl group, 2-methylphenyl group, 3-methylphenyl group, 4-methylphenyl group, 2-(trifluoromethyl)phenyl group, 3-(trifluoromethyl)phenyl group, 4-(trifluoromethyl)phenyl group, 2-fluorophenyl group, 3-fluorophenyl group, 4-fluorophenyl group, 2-chlorophenyl group, 3-chlorophenyl group, 4-chlorophenyl group, 2-bromophenyl group, 3-bromophenyl group, 4-bromophenyl group, 2-iodophenyl group, 3-iodophenyl group, 4-iodophenyl group, 2-cyanophenyl group, 3-cyanophenyl group, 4-cyanophenyl group, 2-nitrophenyl group, 3-nitrophenyl group, 4-nitrophenyl group, 2-methoxyphenyl group, 3-methoxyphenyl group, 4-methoxyphenyl group, 2-(trifluoromethoxy)phenyl group, 3-(trifluoromethoxy)phenyl group, 4 (trifluoromethoxy)phenyl group, 2,3-dichlorophenyl group, 2,4-dichlorophenyl group, 2,5-dichlorophenyl group, 2,6-dichlorophenyl group, 3,4-dichlorophenyl group, 3,5-dichlorophenyl group, 2,3,4-trichlorophenyl group, 2,3,5-trichlorophenyl group, 3,4,5-trichlorophenyl group, 2,4,6-trichlorophenyl group, 2,3-difluorophenyl group, 2,4-difluorophenyl group, 2,5-difluorophenyl group, 2,6-difluorophenyl group, 3,4-difluorophenyl group, 3,5-difluorophenyl group, 3,4,5-trifluorophenyl group, 2,3,5,6-tetrafluorophenyl group, 2,3,4,5,6-pentafluorophenyl group, 3,5-bis(trifluoromethyl)phenyl group, 2,3-dimethoxyphenyl group, 2,4-dimethoxyplienyl group, 2,5-dimethoxyphenyl group, 2,6-dimethoxyphenyl group, 3,4-dimethoxyphenyl group, 3,5-dimethoxyphenyl group, 2-methanesulfonylphenyl group, 3-methanesulfonylphenyl group, 4-methanesulfonylphenyl group, 4-methoxycarbonylphenyl group, 4-ethoxycarbonylphenyl group and 4-benzoylphenyl group.

As the phenyl ($C_1$-$C_4$) alkyl group represented by $R^4$ which may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano

group, $C_1$-$C_6$ alkylsulfenyl groups, $C_1$-$C_6$ alkylsulfinyl groups, $C_1$-$C_6$ alkylsulfonyl groups, $C_2$ - $C_6$ alkoxycarbouyl groups and benzoyl group, there are exemplified phenylmethyl group, (2-methylphenyl)methyl group, (3-methylphenyl)methyl group, (4-methylphenyl)methyl group, {2-(trifluoromethyl)phenyl} methyl group, {3-(trifluoromethyl)phenyl}methyl group, {4-(trifluoromethyl)phenyl}methyl groups, (2-fluorophenyl)methyl group, (3-fluorophenyl)methyl group, (4-fluorophenyl) methyl group, (2-chlorophenyl) methyl group, (3-chlorophenyl) methyl group, (4-chlorophenyl)methyl group, (2-bromophenyl)methyl group, (3-bromophenyl)methyl group, (4-bromophenyl)methyl group, (2-iodophenyl)methyl group, (3-iodophenyl)methyl group, (4-iodophenyl)methyl group, (2-cyanophenyl)methyl group, (3-cyanophenyl)methyl group, (4-cyanophenyl)methyl group, (2-nitrophenyl)methyl group, (3-nitrophenyl) methyl group, (4-nitrophenyl)methyl group, (2-methoxyphenyl)methyl group, (3-methoxyphenyl)methyl group, (4-methoxyphenyl) methyl group, (2-trifluoromethoxyphenyl) methyl group, (3-trifluoromethoxyphenyl) methyl group, (4-trifluoromethoxyphenyl) methyl group, (2,3-dichlorophenyl) methyl group, (2,4-dichlorophenyl) methyl group, (2,5-dichlorophenyl) methyl group, (2,6-dichlorophenyl) methyl group, (3,4-dichlorophenyl) methyl group, (3,5-dichlorophenyl) methyl group, (2,3-difluorophenyl) methyl group, (2,4-difluorophenyl) methyl group, 12 group, (2,6-difluorophenyl)methyl group, (3,4-difluorophenyl)methyl group, (3,5-difluoroohenyl) methyl group, (2,3,4-trifluorophenyl)methyl group, (3,4,5-trifluorophenyl)methyl group, (2,4,6-trifluorophenyl) methyl group, (2,3,5,6-tetrafluorophenyl)methyl group, (2,3,4,5,6-pentafluorophenyl) methyl group, {3,5-bis(trifluoromethyl)phenyl}methyl group, (2,3-dimethoxyphenyl)methyl group, (2,4-dimethoxyphenyl)methyl group, (2,5-dimethoxyphenyl)methyl group, (2,6-dimethoxyphenyl) methyl group, (3,4-dimethoxyphenyl)methyl group, (3,5-dimethoxyphenyl) methyl group, 1-phenylethyl group, 1-(2-methylphenyl)ethyl group, 1-(3-methylphenyl)ethyl group, 1-(4-methylphenyl) ethyl group, 1 -{2 - (trifluoromethyl)phenyl} ethyl group, 1-{3-(trifluoromethyl)phenyl}ethyl group, 1-{4-(trifluoromethyl) phenyl}ethyl group, 1-(2-fluorophenyl)ethyl group, 1-(3-fluorophenyl)ethyl group, 1-(4-fluorophenyl)ethyl group, 1-(2-chlorophenyl)ethyl group, 1- (3-chlorophenyl)ethyl group, 1-(4-chlorophenyl)ethyl group, 1-(2-bromophenyl) ethyl group, 1-(3-bromophenyl)ethyl group, 1-(4-bromophenyl)ethyl group, 1- (2-iodophenyl) ethyl group, 1- (3-iodophenyl)ethyl group, 1-(4-iodophenyl) ethyl group, 1- (2-cyanophenyl) ethyl group, 1- (3-cyanophenyl) ethyl group, 1- (4-cyanophenyl) ethyl group, 1- (2-nitrophenyl) ethyl group, 1-(3-nitrophenyl)ethyl group, 1-(4-nitrophenyl)ethyl group, 1-(2-methoxyphenyl)ethyl group, 1-(3-methoxyphenyl)ethyl group, 1-(4-methoxyphenyl)ethyl group, 1- (2-trifluoromethoxyphenyl) ethyl group, 1-(3-trifluoromethoxyphenyl) ethyl group, 1- (4-trifluoromethoxyphenyl)ethyl group, 1-(2,3-dichlorophenyl) ethyl group, 1-(2,4-dichlorophenyl) ethyl group, 1-(2,5 - dichlorophenyl) ethyl group, 1- (2,6-dichlorophenyl) ethyl group, 1-(3,4-díchlorophenyl)ethyl group, 1-(3,5-dichlorophenyl)ethyl group, 1-(2,3,4-trichlorophenyl) ethyl group, 1- (3,4,5 - trichlorophenyl)ethyl group, 1-(2,4,6-trichlorophenyl)ethyl group, 1-(2,3-difluorophenyl)ethyl group, 1-(2,4-difluorophenyl)ethyl group, 1 -(2,5 -difluorophenyl) ethyl group, 1-(2,6-difluorophenyl) ethyl group, 1-(3,4-difluorophenyl)ethyl group, 1- (3,5-difluorophenyl) ethyl group, 1-(2,3,4-trifluorophenyl) ethyl group, 1-(3,4,5-trifluorophenyl)ethyl group, 1-(2,4,6-trifluorophenyl)ethyl group, 1-(2,3,5,6-tetrafluorophenyl)ethyl group, 1- (2,3,4,5,6-penta fluorophenyl)ethyl group, 1 -{3,5 - bis (trifluoromethyl) phenyl) ethyl group, 1-(2,3-dimethoxyphenyl) ethyl group, 1- (2,4 - dimethoxyphenyl) ethyl group, 1-(2,5-dimethoxyphenyl) ethyl group, 1-(2,6-dimethoxyphenyl) ethyl group, 1=(3,4 - dimethoxyphenyl) ethyl group, 1-(3,5-dimethoxyphenyl) ethyl group, 2-phenylethyl group, 2-(2-methylphenyl) ethyl group, 2-(3-methylphenyl)ethyl group, 2-(4-methylphenyl) ethyl group, 2-{2 - (trifluoromethyl)phenyl}ethyl group, 2-(3-(trifluoromethyl)phenyl}ethyl group, 2-{4-(trifluoromethyl)phenyl}ethyl group, 2-(2-fluorophenyl)ethyl group, 2 -(3 -fluorophenyl)ethyl group, 2 -(4 -fluorophenyl)ethyl group, 2- (2-chlorophenyl)ethyl group, 2-(3-chlorophenyl)ethyl group, 2 - (4 - chlorophenyl) ethyl group, 2 - (2-bromophenyl)ethyl group, 2-(3-bromophenyl)ethyl group, 2-(4-brornophenyl)ethyl group, 2-(2-iodophenyl)ethyl group, 2-(3-iodophenyl)ethyl group, 2-(4-iodophenyl)ethyl group, 2-(2-cyanophenyl)ethyl group, 2-(3-cyanophenyl)ethyl group, 2-(4-cyanophenyl)ethyl group, 2-(2-nitrophenyl)ethyl group, 2-(3-nitrophenyl)ethyl group, 2-(4-nitrophenyl)ethyl group, 2 -(2-methoxyphenyl)ethyl group, 2 -(3 - methoxyphenyl)ethyl group, 2- (4-methoxyphenyl) ethyl group, 2- (2-trifluoromethoxyphenyl} ethyl group, 2-(3-trifluoromethoxyphenyl) ethyl group, 2-(4-trifluoromethoxyphenyl)ethyl group, 2-(2,3-dichlorophenyl)ethyl group, 2- (2,4-dichlorophenyl) ethyl group, 2-(2,5-dichlorophenyl)ethyl group, 2-(2,6-dichlorophenyl)ethyl group, 2 -(3,4-dichlorophenyl)ethyl group, 2-(3,5-dichlorophenyl)ethyl group, 2-(2,3,4-trichlorophenyl) ethyl group, 2-(3,4,5-trichlorophenyl)ethyl group, 2-(2,4,6-trichlorophenyl)ethyl group, 2-(2,3-difluorophenyl)ethyl group, 2-(2,4-difluorophenyl)ethyl group, 2-(2,5-difluorophenyl)ethyl group, 2 -(2,6 - difluarophenyl)ethyl group, 2-(3,4-difluorophenyl) ethyl group, 2-(3,5-difluorophenyl)ethyl group, 2-(2,3,4-triflucrophenyl)ethyl group, 2-(3,4,5-trifluorophenyl)ethyl group, 2-(2,4,6-trifluorophenyl)ethyl group, 2-(2,3,5,6-tetrafluorophenyl)ethyl group, 2-(2,3,4,5,6-pentafluorophenyl)ethyl group, 2-{3,S-bis(trifluorometliyl)pheriyl}ethyl group, 2-(2,3-dimethoxyphenyl)ethyl group, 2-(2,4-dimethoxyphenyl)ethyl group, 2-(2,5-dimethoxyphenyl)ethyl group, 2-(2,6-dimethoxyphenyl)ethyl group, 2-(3,4-dimethoxyphenyl)ethyl group and 2-(3,5-dimethoxyphenyl)ethyl group.

As the phenoxy ($C_1$-$C_4$) alkyl group represented by $R^4$ which may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group, there are exemplified phenoxymethyl group, (2-fluorophenoxy) methyl group, (3-fluorophenoxy) methyl group, (4-fluorophenoxy)methyl group, (2-chlorophenoxy)methyl group, (3-chlorophenoxy)methyl group, (4-chlorophenoxy)methyl group, (2-bromophenoxy) methyl group, (3-bromophenoxy) methyl group, (4-bromophenoxy)methyl group, (2-methoxyphenoxy)methyl group, (3-methoxyphenoxy) methyl group, (4-methoxyphenoxy) methyl group, (2,3-difluorophenoxy)methyl group, (2,4-difluorophenoxy)methyl group, (2,5-di fluorophenoxy)methyl group, (2,6-difluorophenoxy)methyl group, (3,4-difluorophenoxy) methyl group, (3,5-difluorophenoxy)methyl group, (2,3-dichlorophenoxy)methyl group, (2,4-dichlorophenoxy) methyl group, (2,5-dichlorophenoxy) methyl group, (2,6-dichlorophenoxy) methyl group, (3,4-dichlorophenoxy) methyl group, (3,5-dichlorophenoxy) methyl group, 1-phenoxyethyl group, 1-(2-fluorophenoxy)ethyl group, 1-(3-fluorophenoxy)ethyl group, 1-(4-fluorophenoxy)ethyl group, 1-(2-chlorophenoxy) ethyl group, 1-(3-chlorophenoxy)ethyl group, 1-(4-chlorophenoxy)ethyl group, 1-(2-bromophenoxy) ethyl group, 1- (3-bromophenoxy) ethyl group, 1- (4-bromophenoxy)ethyl group, 1-(2-me thoxyphenoxy) ethyl group, 1- (3-methoxyphenoxy)ethyl group, 1- (4-methoxyphenoxy) ethyl group, 1- (2,3-difluorophenoxy)ethyl group, 1- (2,4-difluorophenoxy) ethyl group, 1- (2,5-difluorophenoxy)ethyl group, 1-(2,6-difluorophenoxy)ethyl group, 1-(3,4-difluorophenoxy) ethyl group, 1-(3,5-difluorophenoxy) ethyl group,1-(2,3-dichlorophenoxy) ethyl group, 1-(2,4-dichlorophenoxy) ethyl group, 1-(2,5-dichlorophenexy) ethyl group, 1-(2,6-dichlorophenoxy)ethyl group, 1-(3,4-dichlorophenoxy) ethyl group, 1-(3,5-dichlorophenoxy) ethyl group, 2-phenoxyethyl group,2-(2-fluorophenoxy) ethyl group, 2-(3-fluorophenoxy) ethyl group, 2-(4-fluorophenoxy) ethyl group, 2-(2 chlorophenoxy) ethyl group, 2-(3-chlorophenoxy) ethyl group, 2-(4-chlorophenoxy)ethyl group, 2-(2-bromophenoxy) ethyl group, 2-(3-bromophenoxy) ethyl group, 2-(4-bromophenoxy) ethyl group, 2-(2-methoxyphenoxy) ethyl group, 2-(3-methoxyphenoxy) ethyl group,2-(4-methoxyphenoxy)ethyl group, 2-(2,3-difluorophenoxy) ethyl group, 2-(2,4-difluorophenoxy) ethyl group, 2-(2,5-difluorophenoxy) ethyl group, 2-(2,6-difluorophenoxy) ethyl group, 2-(3,4-difluorophenoxy)ethyl group, 2-(3,5-difluorophenoxy) ethyl group, 2-(2,3 dichlorophenoxy) ethyl group, 2-(2,4-dichlorophenoxy) ethyl group, 2-(2,5-dichlorophenoxy) ethyl group, 2-(2,6-dichlorophenoxy)ethyl group, 2-(3,4-dichlorophenoxy) ethyl group and 2-(3,5-dichlorophenoxy)ethyl group.

As the phenyl- ($C_1$-$C_2$) alkylamino group represented by $R^4$ which may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group, there are exemplified benzylamino group, 2-methylbenzylamino group, 3-methylbenzylamino group, 4-methylbenzylamino group, {2-(trifluoromethyl)phenyl}methylamino group, {3-(trifluoromethyl)phenyl}methylamino group, {4 - (trifluoromethyl)pheryl}methylamino group, 2-fluorobenzylamino group, 3-fluorobenzylamino group, 4-fluorobenzylamino group, 2-chlorobenzylamino group, 3-chlorobenzylamino group, 4-chlorobenzylamirio group, 2-bromobenzylamino group, 3-bromobenzylamino group, 4-bromobenzylamino group, (2,3-difluorophenyl)methylamino group, (2,4-difluorophenyl)methyla!nino group, (2,5-difluorophenyl) methylamino group, (2,6-difluorophenyl) methylamino group, (3,4-difluorophenyl)methylamino group, (3,5-difluorophenyl)methylamino group, (2,3-dichlorophenyl)methylamino group, (2,4-dichlorophenyl)methylamino group, (2,5-dichlorophenyl)methylamino group, (2,6-dichlorophenyl) methylamino group, (3,4-dichlorophenyl)methylamino group, (3,5-dichlorophenyl)methylamino group, (2,3-dimethoxyphenyl)methylamino group, (2,4-dimethoxyphenyl) methylamino group, (2,5-dimethoxyphenyl) methylamino group, (2,6-dimethoxyphenyl) methylamino group, (3,4-dimethoxyphenyl) methylamino group, (3,5-dimethoxyhenyl)methylamino group, 1-phenylethylamino group and 2-phenylethylamino group.

The $C_1$-$C_{12}$ alkyl group represented by $R^5$ includes, for example, methyl group, ethyl group, propyl group, isopropyl group, butyl group, isobutyl group, sec-butyl group, tert-butyl group, pentyl group, 1-methylbutyl group, 2-methylbutyl group, 3-methylbutyl group, 1-ethylpropyl group, 1,1-dimethylpropyl group, 1,2-dimethylpropyl group, 2,2-dimethylpropyl group, hexyl group, 1-methylpentyl group, 2-methylpentyl group, 1,1-dimethylbutyl group, 1,3-dimethylbutyl group, heptyl group, octyl group, nonyl group, decyl group, undecyl group and dodecyl group.

The $C_3$-$C_{12}$ cycloalkyl group represented by $R^5$ includes, for example, cyclopropyl group, 1-methylcyclopropyl group, 2-methylcyclopropyl group, 2,2-dimethylcyclopropyl group, 2,2,3,3-tetramethylcyclopropyl group, cyclobutyl group, cyclopentyl group, 1-methylcyclopentyl group, 2-methylcyclopentyl group, 3-methylcyclopentyl group, cyclohexyl group, 1-methylcyclohexyl group, 2-methylcyclohexyl group, 3-methylcyclohexyl group, 4-methylcyclohexyl group, cycloheptyl group and cyclooctyl group.

The ($C_3$-$C_6$ cycloalkyl)$C_1$-$C_6$ alkyl group represented by $R^5$ includes, for example, cyclopropylmethyl group, (1-methylcyclopropyl)methyl group, cyclobutylmethyl group, cyclopentylmethyl group, cyclohexylmethyl group and cycloheptylmethyl group.

The $C_2$-$C_6$ alkoxyalkyl group represented by $R^5$ includes, for example, methoxymethyl group, 1-methoxyethyl group, 2-methoxyethyl group, 1-ethoxyethyl group, 2-ethoxyethyl group, 1-propoxyethyl group, 2-propoxyethyl group, isopropoxymethyl group, 1-butoxyethyl group, 2-butoxyethyl group, 1-isobutoxyethyl group, 2-isobutoxyethyl group, 1-(sec-butoxy)ethyl group, 2-(sec-butoxy)ethyl group, 1-methoxypropyl group, 2-methoxypropyl group and 3-methoxypropyl group.

The $C_3$-$C_{12}$ alkenyl group represented by $R^5$ includes, for example, 1-propenyl group, 2-propenyl group, 1-methylethenyl group, 2-butenyl group, 1-methyl-2-propenyl group, 2-methyl-2-propenyl group, 2-pentenyl group, 2-methyl-2-butenyl group, 3-methyl-2-butenyl group, 2-ethyl-2-propenyl group, 1,1-dimethyl-2-propenyl group, 2-hexenyl

group, 2-methyl-2-pentenyl group, 2.,4-dimethyl-2,6-heptedienyl group and 3,7-dimethyl-2,6-octadienyl group.

The $C_3$-$C_{12}$ alkynyl group represented by $R^5$ includes, for example, 1-propynyl group, 2-propynyl group, 2-butynyl group, 1-methyl-2-propynyl group, 2-pentynyl group, 1-methyl-2-butynyl group, 1,1-dimethyl-2-propynyl group and 2-hexynyl group.

The $C_1$-$C_6$ acyl group represented by $R^5$ includes, for example, formyl group, acetyl group, propionyl group, isobutyroyl group and trimethylacetyl group.

The $C_2$-$C_8$ cyanoalkyl group represented by $R^5$ includes, for example, cyanomethyl group and 2-cyanoethyl group.

The $C_2$-$C_6$ alkoxycarbonyl group represented by $R^5$ includes, for example, methoxycarbonyl group and ethoxycarbonyl group.

The $C_3$-$C_8$ alkoxycarbonylalkyl group represented by $R^5$ includes, for example, 1-(methoxycarbonyl) ethyl group, 1-(ethoxycarbonyl) ethyl group, 1 2-(methoxycarbonyl)ethyl group and 2-(ethoxycarbonyl)ethyl group.

As the phenyl ($C_1$-$C_4$) alkyl group represented by $R^5$ which may be substituted with 1 to 5 groups independently selected from the group consisting of $C_1$-$C_6$ alkyl groups unsubstituted substituted with one or more halogen atoms, $C_1$-$C_6$ alkoxy groups unsubstituted or substituted with one or more halogen atoms, halogen atoms, nitro group and cyano group, there are exemplified benzyl group, 1-phenylethyl group and 2-phenylethyl groups.

As the benzoyl group represented by $R^5$ which may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group and cyano group, there are exemplified benzoyl group, 2-fluorobenzoyl group, 3-fluorobenzoyl group, 4-fluorobenzoyl group, 2-chlorobenzoyl group, 3-chlorobenzoyl group, 4-chlorobenzoyl group, 2-methoxybenzoyl group, 3-methoxybenzoyl group, 4-methoxybenzoyl group, 2-cyanobenzoyl group, 3-cyanobenzoyl group, 4-cyanobenzoyl group, 2-nitrobenzoyl group, 3-nitrobenzoyl group and 4-nitrobenzoyl group.

As the present compound, the following isoxazoline compounds are exemplified:

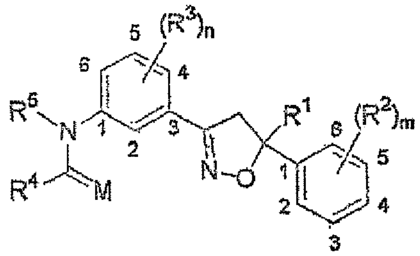

(1)

isoxazoline compounds of the formula (1) in which $R^1$ is a trifluoromethyl group;
isoxazoline compounds of the formula (1) in which m is 2 and each of $R^2$ is a halogen atom;
isoxazoline compounds of the formula (1) in which m is 2 and each of $R^2$ is a chlorine atom;
isoxazoline compounds of the formula (1) in which m is 2 and $R^2$s are chlorine atoms as substituents at the 3-position and 5-position, respectively;
isoxazoline compounds of the formula (1) in which n is 0 or 1;
isoxazoline compounds of the formula (1) in which n is 0;
isoxazoline compounds of the formula (1) in which n is 1;
isoxazoline compounds of the formula (1) in which n is 1 and $R^3$ is a $C_1$-$C_6$ alkyl group as a substituent at the 6-position;
isoxasoline compounds of the formula (1) in which n is 1 and $R^3$ is a methyl group as a substituent at the 6-position;
isoxazoline compounds of the formula (1) in which $R^4$ is a hydrogen atom;
isoxazoline compounds of the formula (1) in which $R^4$ is a $C_1$-$C_{12}$ alkyl group unsubstituted or substituted with one or more halogen atoms;
isoxazoline compounds of the formula (1) in $R^4$ is a $C_2$-$C_{12}$ alkenyl group unsubstituted or substituted with one or more halogen atoms;
isoxasoline compounds of the formula (1) in which $R^4$ is a $C_1$-$C_6$ alkoxy group unsubstituted or substituted with one or more halogen atoms;
isoxazoline compounds of the formula (1) in which $R^4$ is a phenyl group which may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group (hereinafter, the group consisting of $C_1$-$C_6$ alkyl groups unsubsti-

tuted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group is referred to as the group X in some cases);

isoxazoline compounds of the formula (1) in which $R^1$ is a trifluoromethyl group, $R^4$ is a hydrogen atom, a $C_1$-$C_{12}$ alkyl group unsubstituted or substituted with one or more halogen atoms, a ($C_2$-$C_5$ alkoxycarbonyl) $C_1$-$C_{12}$ alkyl group, a $C_2$-$C_{12}$ cyanoalkyl group, a $C_3$-$C_{12}$ cycloalkyl group unsubstituted or substituted with one or more halogen atoms, a ($C_3$-$C_6$ cycloalkyl)$C_1$-$C_6$ alkyl group, a ($C_1$-$C_6$ alkoxy)$C_2$-$C_6$ alkyl group, a $C_2$-$C_{12}$ alkenyl group unsubstituted or substituted with one or more halogen atoms, a $C_2$-$C_{12}$ alkynyl group unsubstituted or substituted with one or more halogen atoms, a $C_1$-$C_6$ alkoxy group unsubstituted or substituted with one or more halogen atoms, a ($C_1$-$C_6$ aLkoxy)$C_1$-$C_6$ alkoxy group, a $C_3$-$C_6$ alkenyloxy group unsubstituted or substituted with one or more halogen atoms, a benzyloxy group, a phenyl ($C_2$-$C_6$) alkenyl group, a ($C_1$-$C_6$ alkylamino)$C_1$-$C_6$ alkyl group, or a (di($C_1$-$C_6$ alkyl) amino)$C_1$-$C_6$ alkyl group, and $R^5$ is a hydrogen atom;

isoxazoline compounds of the formula (1) in which $R^1$ is a trifluoromethyl group, $R^4$ is a phenyl group which may be substituted with 1 to 5 groups independently selected from above-mentioned group X, and $R^5$ is a hydrogen atom;

isoxazoline compounds of the formula (1) in which $R^1$ is a trifluoromethyl group, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom;

isoxazoline compounds of the formula: (1) in which $R^1$ is a trifluoromethyl group, m is 2, $R^2$s are chlorine atoms as substituents at the 3-position and 5-position, respectively, $R^4$ is a hydrogen atom, a $C_1$-$C_{12}$ alkyl group unsubstituted or substituted with one or more halogen atoms, a ($C_2$-$C_5$ alkoxycarbonyl)$C_1$-$C_{12}$ alkyl group, a $C_2$-$C_{12}$ cyanoalkyl group, a $C_3$-$C_{12}$ cycloalkyl group unsubstituted or substituted with one or more halogen atoms, a ($C_3$-$C_6$ cycloalkyl) $C_1$-$C_6$ alkyl group, a ($C_1$-$C_6$ alkoxy) $C_2$-$C_6$ alkyl group, a $C_2$-$C_{12}$ alkenyl group unsubstituted or substituted with one or more halogen atoms, a $C_2$-$C_{12}$ alkynyl group unsubstituted or substituted with one or more halogen atoms, a $C_1$-$C_6$ alkoxy group unsubstituted or substituted with one or more halogen atoms, a ($C_1$-$C_6$ alkoxy)$C_1$-$C_6$ alkoxy group, a $C_3$-$C_6$ alkenyloxy group unsubstituted or substituted with one or more halogen atoms, a benzyloxy group, a phenyl($C_2$-$C_6$) alkenyl group, a ($C_1$-$C_6$ alkylamino) $C_1$-$C_6$ alkyl group, or a (di ($C_1$-$C_6$ alkyl) amino) $C_1$-$C_6$ alkyl group, and $R^5$ is a hydrogen atom;

isoxazoline compounds of the formula (1) in which $R^1$ is a trifluoromethyl group, m is 2, $R^2$s are chlorine atoms as substituents at the 3-position and 5-position, respectively, $R^4$ is a phenyl group which may be substituted with 1 to 5 groups independently selected from the above-mentioned group X, and $R^5$ is a hydrogen atom;

isoxazoline compounds of the formula (1) in which $R^1$ is a trifluoromethyl group, m is 2, $R^2$s are chlorine atoms as substituents at the 3-position and 5-position, respectively, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom;

isoxazoline compounds of the formula (1) in which $R^1$ is a trifluoromethyl group, n is 0 or 1, $R^4$ is a hydrogen atom, a $C_1$-$C_{12}$ alkyl group unsubstituted or substituted with one or more halogen atoms, a ($C_2$-$C_5$ alkoxycarbonyl)$C_1$-$C_{12}$ alkyl group, a $C_2$-$C_{12}$ cyanoalkyl group, a $C_3$-$C_{12}$ cycloalkyl group unsubstituted or substituted with one or more halogen atoms, a ($C_3$-$C_6$ cycloalkyl) $C_1$-$C_6$ alkyl group, a ($C_1$-$C_6$ alkoxy)$C_2$-$C_6$ alkyl group, a $C_2$-$C_{12}$ alkenyl group unsubstituted or substituted with one or more halogen atoms, a $C_2$-$C_{12}$ alkynyl group unsubstituted or substituted with one or more halogen atoms, a $C_1$-$C_6$ alkoxy group unsubstituted or substituted with one or more halogen atoms, a ($C_1$-$C_6$ alkoxy)$C_1$-$C_6$ alkoxy group, a $C_3$-$C_6$ alkenyloxy group unsubstituted or substituted with one or more halogen atoms, a benzyloxy group, a phenyl($C_2$-$C_6$)alkenyl group, a ($C_1$-$C_6$ alkylamino)$C_1$-$C_6$ alkyl group, or a (di($C_1$-$C_6$ alkyl)amino)$C_1$-$C_6$ alkyl group, and $R^5$ is a hydrogen atom;

isoxazoline compounds of the formula (1) in which $R^1$ is a trifluoromethyl group, n is 0 or 1, $R^4$ is a phenyl group which may be substituted with 1 to 5 groups independently selected from the above-mentioned group X, and $R^5$ is a hydrogen atom; and

isoxazoline compounds of the formula (1) in which $R^1$ is a trifluoromethyl group, m is 2, $R^2$s are chlorine atoms as substituents at the 3-position and 5-position, respectively, n is 0, $R^4$ is a phenyl group unsubstituted or substituted with 1 to 5 groups independently selected from the above-mentioned group X, and $R^5$ is a hydrogen atom;

isoxazoline compounds of the formula (1) in which $R^1$ is a trifluoromethyl group, m is 2, $R^2$s are chlorine atoms as substituents at the 3-position and 5-position, respectively, n is 1, $R^3$ is a $C_1$-$C_6$ alkyl group as a substituent at the 6-position, $R^4$ is a phenyl group unsubstituted or substituted with 1 to 5 groups independently selected from the above-mentioned group X, and $R^5$ is a hydrogen atom;

isoxazoline compounds of the formula (I) in which $R^1$ is a trifluoromethyl group, m. is 2, $R^2$s are chlorine atoms as substituents at the 3-position and 5-position, respectively, n is 0, $R^4$ is a phenyl group which may be substituted

with 1 to 5 groups independently selected from the group consisting of $C_1$,-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfonyl groups, $C_2$-$C_6$ alkoxycarbonyl groups and benzoyl group (the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfonyl groups, $C_2$-$C_6$ alkoxycarbonyl groups and benzoyl group is hereinafter referred to as group Y in same cases), and $R^5$ is a hydrogen atom;
isoxazoline compounds of the formula (1) in which $R^1$ is a trifluoromethyl group, m is 2, $R^2$s are chlorine atoms as substituents at the 3-position and 5-position, respectively, n is 1, $R^3$ is a $C_1$-$C_6$ alkyl group as a substituent at the 6-position, $R^4$ is a phenyl group unsubstituted or substituted with 1 to 5 groups independently selected from the above-mentioned group Y, and $R^5$ is a hydrogen atom;
isoxazoline compounds of the formula (1) in which $R^1$ is a trifluoromethyl group, n is 0 or 1, $R^4$ is a hydrogen atom, a $C_1$-$C_{12}$ alkyl group unsubstituted or substituted with one or more halogen atoms, a $C_7$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom.

Next, the production of the present compound is explained below.

(Production Process 1)

The present compound can be produced by reacting a compound represented by the formula (2) with a compound represented by the formula (3).

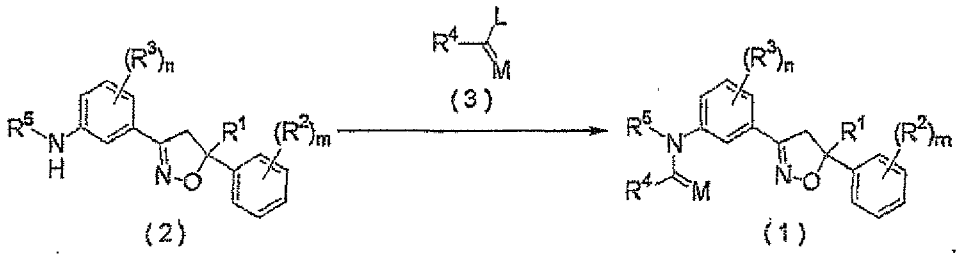

wherein $R^1$, $R^2$, $R^3$, $R^4$, $R^5$, M, m and n are as defined above, and L is a hydroxyl group or a chlorine atom.
The reaction is usually carried out in a solvent.
The solvent used in the reaction includes, for example, ethers such as tetrahydrofuran, diethyl ether, tert-butyl methyl ether, ethylene glycol dimethyl ether, 1,4-dioxane, etc.; acid amides such as N,N-dimethylformamide, etc.; nitriles such as acetonitrile, etc.; hydrocarbons such as toluene, etc.; esters such as ethyl acetate, etc.; sulfoxides such as dimethyl sulfoxide, etc.; and mixtures thereof.
When L is a chlorine atom, the reaction is carried out in the presence of a base.
The base used in the reaction in such a case includes, for example, alkali metal hydrides such as sodium hydride, etc.; carbonates such as potassium carbonate, etc. alkali metal alkoxides such as potassium tert-butoxide, etc.; and organic amines such as triethylamine, pyridine, etc.
When L is a hydroxyl group, the reaction is carried out in the presence of a condensing agent.
The condensing agent used in the reaction in such a case includes, for example, dicyclohexylcarbodiimide and 1-(3-dimethylaminapropyl)-3-ethylcarbodiimide hydrochloride.
As to the amounts of the reactants used in the reaction, the amount of the compound of the formula (3) is usually 1 to 2 moles per mole of the compound of the formula (2), and the amount of the base or the condensing agent is usually 1 to 2 moles per mole of the compound of the formula (2).
The reaction temperature ranges usually from 0 to 80°C, and the reaction time ranges usually from 0.5 to 24 hours.
After completion of the reaction, the present compound can be isolated by extracting the reaction mixture with an organic solvent and carrying out after-treatments such as drying and concentration. The present compound isolated can be further purified by operations such chromatography, recrystallization and the like.

(Production Process 2)

The present compound can be produced also by reacting a compound represented by the formula (4) with a base at first and then reacting- the reaction product with a compound represented by the formula (5) .

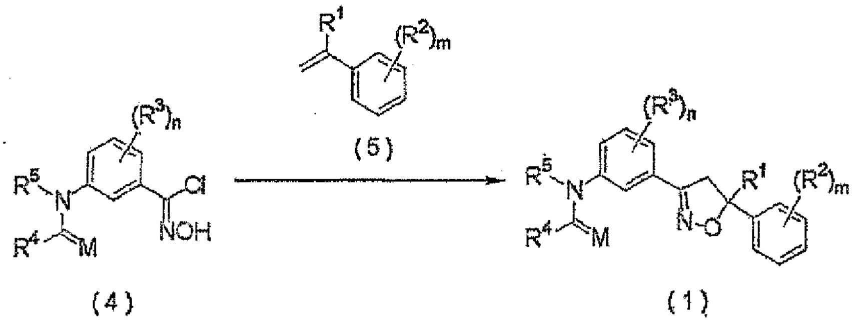

(4) (5) (1)

wherein $R^1$, $R^2$, $R^3$, $R^4$, $R^5$, M, m and n are as defined above.

The reaction is usually carried out in a solvent.

The solvent used in the reaction includes, for example, ethers such as tetrahydrofuran, diethyl ether, tert-butyl methyl ether, ethylene glycol dimethyl ether, 1,4-dioxane, etc.; acid amides such as N,N-dimethylformamide, etc.; nitriles such as acetonitrile, etc. ; hydrocarbons such as toluene, etc.; esters such as ethyl acetate, etc.; sulfoxides such as dimethyl sulfoxide, etc.; and mixtures thereof.

The base used in the reaction includes, for example, alkali metal hydrides such as sodium hydride, etc. carbonates such as potassium carbonate, etc.; alkali metal alkoxides such as potassium tert-butoxide, etc. and organic amines such as triethylamine, pyridine, etc.

As to the amounts of the reactants used in the reaction, the amount of the compound of the formula (5) is usually 1 to 2 moles per mole of the compound of the formula (4), and the amount of the base is usually 1 to 2 moles per mole of the compound of the formula (4).

In the step of reacting the compound of the formula (4) with the base at first, the reaction temperature ranges usually from 0 to 80°C and the reaction time ranges usually from 0.5 to 24 hours

The reaction mixture obtained in the step of reacting the compound of the formula (4) with the base can be used as it is in the step of the reaction with the compound of the formula (5). In this step, the reaction temperature ranges usually from 0 to 80°C and the reaction time ranges usually from 0.5 to 24 hours

After completion of the reaction, the present compound can be isolated by extracting the reaction mixture with an organic solvent and carrying out after-treatments such as drying and concentration. The present compound isolated can be further purified by operations such as chromatography, recrystallization and the like.

(Production Process 3)

A compound represented by the formula (1-1) , i.e., the present compound in which $R^4$ is a $C_1$-$C_6$- alkoxy group unsubstituted or substitute with one or more halogen atoms, or the like, can be produced by reacting a compound represented by the formula (2) with a carbonylating agent at first and then reacting the reaction produce with a compound represented by the formula (13).

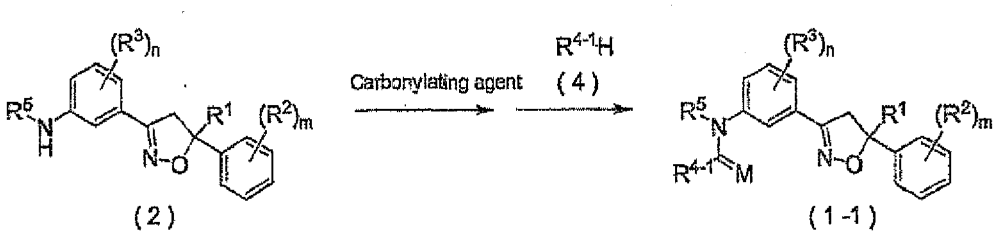


(2) (1-1)

wherein $R^{4-1}$ is a $C_1$-$C_6$ alkoxy group unsubstituted or substituted with one or more halogen atoms, a ($C_1$-$C_6$) alkoxy) C2-CG alkoxy group, a $C_3$-$C_6$ alkenyloxy group unsubstituted or substituted with one or more halogen atoms, a benzyloxy

group, a group represented by the following formula:

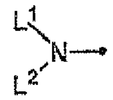

a phenoxy group unsubstituted or substituted with 1 to 5 groups independently selected from the group X, a phenylamino group unsubstituted or substituted, with 1 to 5 groups independently selected from the group X, or a phenyl ($C_1$-$C_2$) alkylamino group unsubstituted or substituted with 1 to 5 groups independently selected from the group X, and $R^1$, $R^2$, $R^3$ , $R^5$, $L^1$, $L^2$, M, m and n are as defined above.

The reactions are usually carried out in a solvent.

The solvent used in the reaction includes, for example ethers such as tetrahydrofuran, diethyl ether, tert-butyl methyl ether, ethylene glycol dimethyl ether, 1,4-dioxane, etc . ; acid amides such as N,N-dimethylformamide,etc. ; nitriles such as acetonitrile, etc. ; hydrocarbons such as toluene, etc. ; esters such as ethyl acetate, etc.; sulfoxides such as dimethyl sulfoxide, etc.; and mixtures thereof.

The carbonylating agent used in the reaction includes, for example, phosgene, triphosgene and trichloromethyl chloroformate in the case of M being an oxygen atom, and thiophosgene in the case of M being- an sulfur atoms.

As to the amounts of the reactants used in the reaction, the amount of the compound of the formula (13) is usually 1 to 200 moles per mole of the compound of the formula (2) , and the amount of the carbonylating agent is usually 1 to 4 moles per mole of the compound of the formula (2).

In the step of reacting the compound of the formula. (2) with the carbonylating agent at first, the reaction temperature ranges usually from 0 to 100°C and the reaction time ranges usually from 0.5 to 24 hours

The reaction mixture obtained in the step of reacting the compound of the formula (2) with the carbonylating agent can be used as it is in the step of the reaction with the compound of the formula (13). In this step, the reaction temperature ranges usually from 0 to 150°C and the reaction time ranges usually from 0.5 to 24 hours

After completion of all the reactions, the present compound can be isolated by extracting the reaction mixture with an organic solvent and carrying out after-treatments such as drying and concentration. The compound of the formula (1-1) isolated can be further purified by operations such as chromatography, recrystallization and the like.

Next, a process for producing each intermediate used for producing the present compound is explained below.

The compound represented by the formula (4) can be produced by reacting a compound represented by the formula (6) with a chlorinating agent.

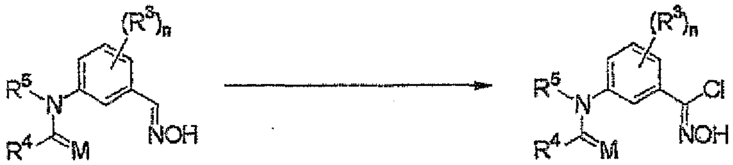

(6) (4)

wherein $R^3$, $R^4$, $R^5$, M and n are as defined above.

The reaction is usually carried out in a solvent.

The solvent used in the reaction includes, for example, ethers such as tetrahydrofuran, diethyl ether, tert-butyl methyl ether, ethylene glycol dimethyl ether, 1,4-dioxane, etc.; hydrocarbons such as toluene, etc.; esters such as ethyl acetate, etc.; acid amides such as N,N-dimethylformamide, etc.; nitriles such as acetonitrile, etc.; sulfoxides such as dimethyl sulfoxide, etc.; and mixtures thereof.

The chlorinating agent used in the reaction includes, for example, chlorine gas and N-chlorosuccinimide.

As to the amounts of the reactants used in the reaction, the amount of the chlorinating agent is usually 1 to 2 moles per mole of the compound of the formula (63.

The reaction temperature ranges usually from -20 to 80°C, and the reaction time ranges usually from 0.5 to 24 hours.

After completion of the reaction, the compound of the formula (4) can be isolated by extracting the reaction mixture with an organic solvent and carrying out after-treatments such as drying and concentration. The compound of the formula (4) isolated can be further purified by operations such as chromatography, recrystallization and the like.

The compound represented by the formula (6) can be produced by reacting a compound represented by the formula (7) with hydroxylamine.

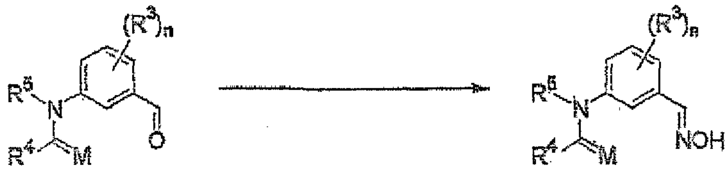

(7) (6)

wherein $R^3$, $R^4$, $R^5$, M and n are as defined above.

The reaction is usually carried out in a solvent.

The solvent used in the reaction includes, for example, ethers such as tetrahydrofuran, diethyl ether, tert-butyl methyl ether, ethylene glycol dimethyl ether, 1,4-dioxane, etc.; hydrocarbons such as toluene, etc.; esters such as ethyl acetate, etc.; acid amides such as N,N-dimethylformamide, etc.; alcohols such as ethanol, methanol, etc.; nitriles such as acetonitrile, etc.; sulfoxides such as dimethyl sulfoxide, etc.; water; and mixtures there of.

As the hydroxylamine used in the reaction, there are exemplified salts of hydroxylamine with a mineral acid, such as hydroxylamine hydrochloride and hydroxylamine sulfate, which can give hydroxy lamine in the reaction system. In such a case, the reaction is carried out in the presence of a base. The base used in such a case includes, for example, organic amines such as triethylamine, etc.; carbonates such as sodium carbonate, etc.; and alkali metal hydroxides such as sodium hydroxide, etc.

As to the amounts of the reactants used in the reaction, the amount of hydroxylamine is usually to 2 moles per mole of the compound of the formula (7), and the amount of the base used in the case of using the salt of hydroxylamine with a mineral acid is usually 1 to 2 moles per mole of the salt of hydroxylamine with a mineral acid.

The reaction temperature ranges usually from 0 to 80°C, and the reaction time ranges usually from 0.5 to 24 hours. After completion of the reaction, the compound of the formula (6) can be isolated by extracting the reaction mixture with an organic solvent and carrying out after-treatments such as drying and concentration. The compound of the formula (6) isolated can be further purified by operations such chromatography, recrystallization and the like.

A compound represented by the formula (2-1), i.e., a compound represented by the formula (2) in which $R^5$ is a hydrogen atom, can be produced by reducing a compound represented by the formula (8) by any of the following methods (i) to (iii) .

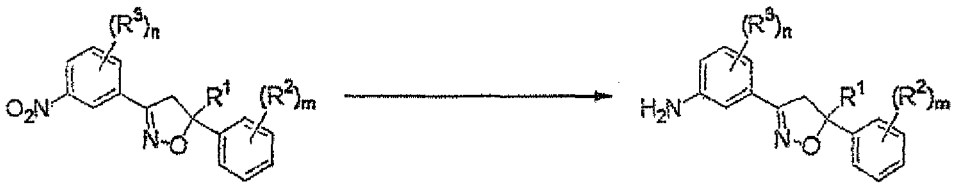

(8) (2-1)

$R^1$, $R^2$, $R^3$, M, m and n are as defined above.

(i) A method of reacting the compound of the formula (8) with hydrogen gas in the presence of a transition metal catalyst

This reaction is carried out in a solvent.

The solvent used in the reaction includes, for example, esters such as ethyl acetate, etc.; alcohols such as ethanol, methanol, etc. water; acetic acid; hydrochloric acid; and mixtures thereof.

The transition metal catalyst used in the reaction includes, for example, Raney nickel, palladium-carbon, and platinum dioxide.

The amount of the transition metal catalyst used in the reaction is usually 0.01 to 0.5 mole per mole of the compound of the formula (8).

The reaction temperature ranges usually from to 80°C, and the reaction time ranges usually from 0.5 to 24 hours.

After completion of the reaction, the compound of the formula (2-1) can be isolated by filtering the reaction

mixture and, if necessary, carrying out after-treatments such as extraction with an organic solvent, drying and concentration. The compound of the formula (2-1) isolated can be further purified by operations such chromatography, recrystallization and the like.

(ii) A method of reacting the compound of the formula (8) with hydrazine in the presence of a base

This reaction is carried out in a solvent.
The solvent used in the reaction includes, for example, ethers such as diethylene glycol, triethylene glycol, etc., water, and mixtures thereof.
The base used in the reaction includes, for example, alkali metal hydroxides such as potassium hydroxide, etc.
The hydrazine used in the reaction includes, for example, hydrazine hydrated.
As to the amounts of the reactants used in the reaction, the amount of the base is usually 1 to 10 moles per mole of the compound of the formula (8), and the amount of the hydrazine is usually 1 to 10 moles per mole of the compound of the formula (8).
After completion of the reaction, the compound of the formula (2-1) can be isolated by extracting the reaction mixture with an organic solvent and carrying out after-treatments such as drying and concentration. The compound of the formula (2-1) isolated can be further purified, by operations such chromatography, recrystallization and the like.

(iii) A method of reacting the compound of the formula (8) with a metal in the presence of an acid

This reaction is usually carried out in a solvent.
The solvent used in the reaction includes, for example, alcohols such as ethanol, etc., water, and mixtures thereof .
The metal used in the reaction includes, for example, iron, tin and stannous chloride.
The acid used in the reaction includes, for example, acetic acid, hydrochloric acid and sulfuric acid.
As to the amounts of the reactants used in the reaction, the amount of the metal is usually 2 to 20 moles per mole of the compound of the formula (8), and the amount of the acid is usually 0.1 to 2 moles per mole of the compound of the formula (8).
The reaction temperature ranges usually from to 100°C, and the reaction time ranges usually from 0.5 to 12 hours.
After completion of the reaction, the compound of the formula (2-1) can be isolated by filtering the reaction mixture and, if necessary, carrying out after-treatments such as extraction with an organic solvent, drying and concentration. The compound of the formula (2-1) isolated can be further purified by operations such chromatography, recrystallization and the like.
The compound represented by the formula (8) can be produced by reacting a compound represented by the formula (9) with a base at first and then reacting the reaction product with a compound represented by the formula (5).

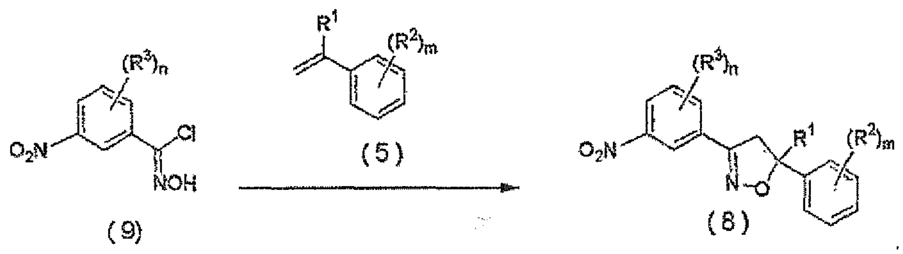

wherein $R^1$, $R^7$, $R^3$, m and n are as defined above.
The reaction is usually carried out in a solvent.
The solvent used in the reaction includes, for example, ethers such as tetrahydrofuran, diethyl ether, tert-butyl methyl ether, ethylene glycol dimethyl ether, 1,4-dioxane, etc.; acid amides such as N,N-dimethylformamide, etc.; nitriles such as acetonitrile, etc.; hydrocarbons such as toluene, etc.; esters such as ethyl acetate, etc.; sulfoxides such as dimethyl sulfoxide, etc.; and mixtures thereof.
The base used in the reaction includes, for example, alkali metal hydrides such as sodium hydride, etc.; carbonates such as potassium carbonate, etc.; alkali metal alkoxides such as potassium tert-butoxide, etc.; and organic amines such as triethylamine, pyridine, etc.
As to the amounts of the reactants used in the reaction, the amount of the compound of the formula (5) is usually 1 to 2 moles per mole of the compound of the formula (9), and the amount of the base is usually 1 to 2 moles

per mole of the compound of the formula (9).

In the step of reacting the compound of the formula (9) with the base at first, the reaction temperature ranges usually from 0 to 80°C and the reaction time ranges usually from 0.5 to 24 hours.

The reaction mixture obtained in the step of reacting the compound of the formula (9) with the base can be used as it is in the step of the reaction with the compound of the formula (5). In this step, the reaction temperature ranges usually from to 80°C and the reaction time ranges usually from 0.5 to 24 hours

After completion of the reaction, the compound of the formula (8) can be isolated by extracting the reaction mixture with an organic solvent and carrying out after-treatments such as drying and concentration. The compound of the formula (8) isolated can be further purified by operations such as chromatography, recrystallization and the like.

The compound represented by the formula (9) can be produced by reacting a compound represented by the formula (10) with a chlorinating agent.

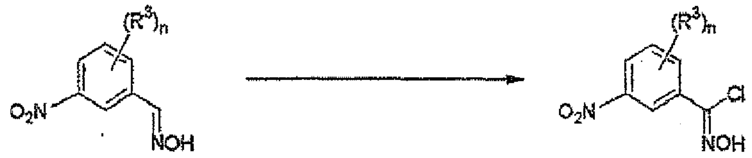

(10) (9)

wherein $R^3$ and n are as defined above.

The reaction is usually carried out in a solvent.

The solvent used in the reaction includes, for example, ethers such as tetrahydrofuran, diethyl ether, tert-butyl methyl ether, ethylene glycol dimethyl ether, 1,4-dioxane, etc.; hydrocarbons such as toluene, etc.; esters such as ethyl acetate, etc.; acid amides such as N,N-dimethylformamide, etc.; nitriles such as acetonitrile, etc.; sulfoxides such as dimethyl sulfoxide, etc.; and mixtures thereof.

The chlorinating agent used in the reaction includes, for example, chlorine gas and N-chlorosuccinimide.

As to the amounts of the reactants used in the reaction, the amount of the chlorinating agent is usually 1 to 2 moles per mole of the compound of the formula (10).

The reaction temperature ranges usually from -20 to 80°C, and the reaction time ranges usually from 0.5 to 24 hours.

After completion of the reaction, the compound of the formula (9) can be isolated by extracting the reaction mixture with an organic solvent and carrying out after-treatments such as drying and concentration. The compound of the formula (9) isolated can be further purified by operations such as chromatography, recrystallization and the like.

The compound represented by the formula (10) can be produced by reacting a compound represented by the formula (11) with hydroxylamine.

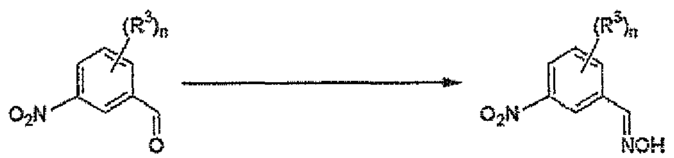

(11) (10)

The reaction is usually carried out in a solvent.

The solvent used in the reaction includes, for example, ethers such as tetrahydrofuran, diethyl ether, tert-butyl methyl ether, ethylene glycol dimethyl ether, 1,4-dioxane, etc.; hydrocarbons such as toluene, etc.; esters such as ethyl acetate, etc.; acid amides such as N,N-dimethylformamide, etc.; alcohols such as ethanol, methanol, etc.; nitriles such as acetonitrile, etc.; sulfoxides such as dimethyl sulfoxide, etc.; water; and mixtures thereof.

As the hydroxylamine used in the reaction, there are exemplified salts of hydroxylamine with a mineral acid, such as hydroxylamine hydrochloride and hydroxylamine sulfate, which can give hydroxylamine in the reaction system. In such a case, the reaction is carried out in the presence of a base. The base used in such a case includes, for example, organic amines such as triethylamine, etc.; carbonates such as sodium carbonate, etc.; and alkali metal hydroxides such as sodium hydroxide, etc.

As to the amounts of the reactants used in the reaction, the amount of hydroxylamine is usually 1 to 2 moles

per mole of the compound of the formula (11), and the amount of the base used in the case of using the salt of hydroxylamine with a mineral acid is usually 1 to 2 moles per mole of the salt of hydroxylamine with a mineral acid.

The reaction temperature ranges usually from 0 to 80°C, and the reaction time ranges usually from 0.5 to 24 hours.

After completion of the reaction, the compound of the formula (10) can be isolated by extracting the reaction mixture with an organic solvent and carrying out after-treatments such as drying and concentration. The compound of the formula (10) isolated can be further purified by operations such chromatography, recrystallization and the like.

Specific examples of the present compound are given below:

isoxazoline compounds represented by the formula (i):

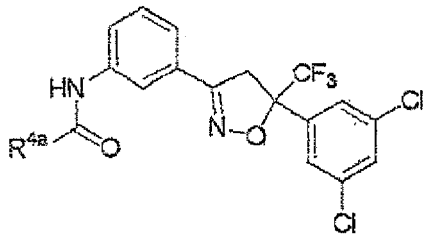

(i)

wherein $R^{4a}$ is any group selected from the group (A) described hereinafter;
isoxazoline compounds represented by the formula (ii):

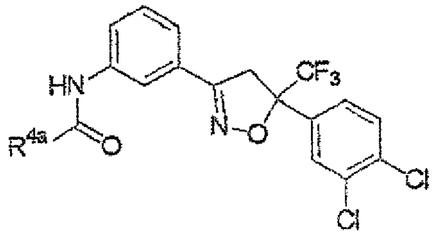

(ii)

wherein $R^{4a}$ is any group selected from the group (A) described hereinafter;
isoxazoline compounds represented by the formula (iii):

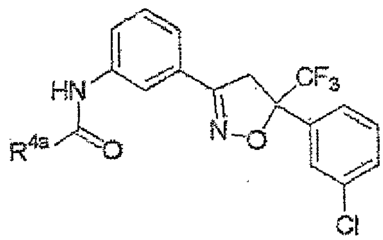

(iii)

wherein $R^{4a}$ is any group selected from the group (A) described hereinafter;
isoxazoline compounds represented by the formula (iv):

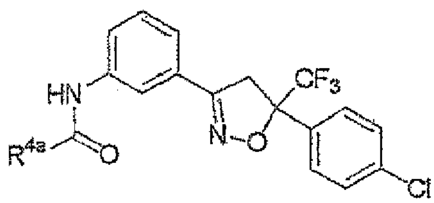

(iv)

wherein $R^{4a}$ is any group selected from the group (A) described hereinafter; isoxasolina compounds represented by the formula (v):

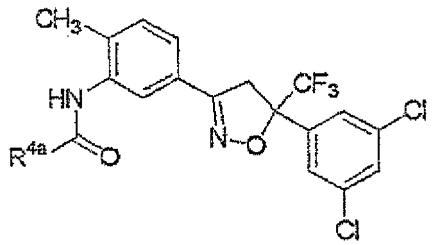

(v)

wherein $R^{4a}$ is any group selected from the group (A) described hereinafter; isoxazoline compounds represented by the formula (vi):

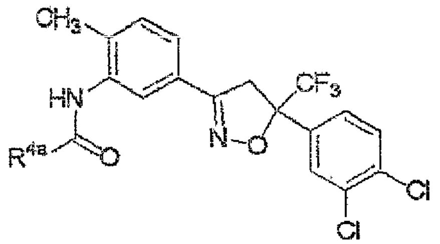

(vi)

wherein $R^{4a}$ is any group selected from the group (A) described hereinafter; isoxazoline compounds represented by the formula (vii):

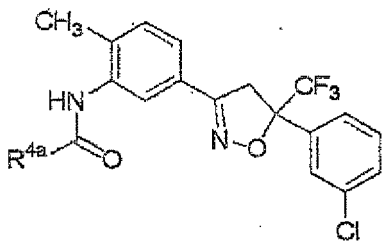

(vii)

wherein $R^{4a}$ is any group selected from the group (A) described hereinafter; isoxazoline compounds represented by the formula (viii):

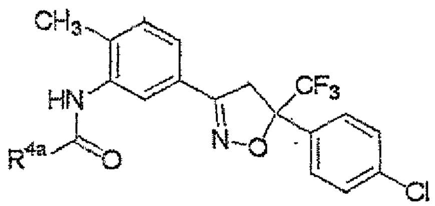

(viii)

wherein $R^{4a}$ is any group selected from the group (A) described hereinafter; isoxazoline compounds represented by the formula (ix):

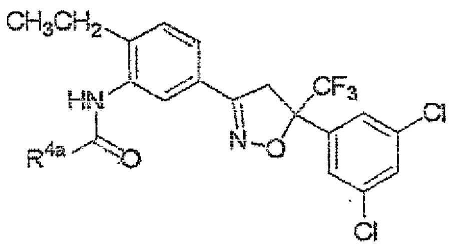

( ix )

wherein $R^{4a}$ is any group selected from the group (A) described hereinafter; isoxazoline compounds represented by the formula (x):

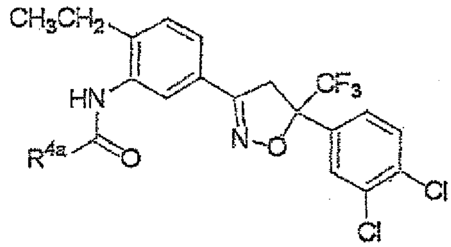

( x )

wherein $R^{4a}$ is any group selected from the group (A) described hereinafter; isoxazoline compounds represented by the formula (xi):

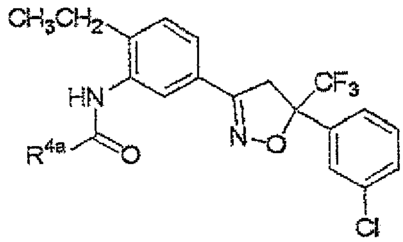

( xi )

wherein $R^{4a}$ is any group selected from the group (A) described hereinafter; isoxazoline compounds represented by the formula (xii):

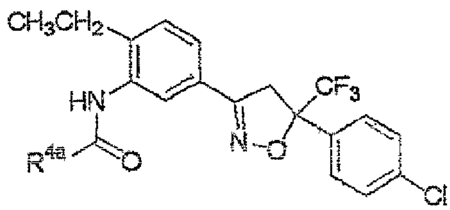

( xii )

wherein $R^{4a}$ is any group selected from the group (A) described hereinafter; isoxazoline compounds represented by the formula (xiii):

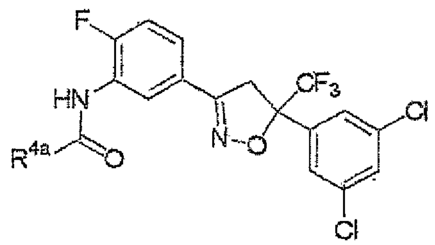

( xiii )

wherein $R^{4a}$ is any group selected from the group (A) described hereinafter; isoxazoline compounds represented by the formula (xiv):

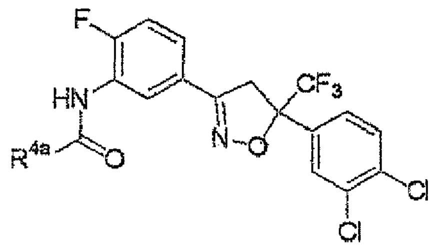

( xiv )

wherein $R^{4a}$ is any group selected from the group (A) described hereinafter; isoxazoline compounds represented by the formula (xv):

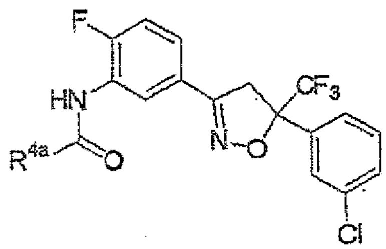

( xv )

wherein $R^{4a}$ is any group selected from the group (A) described hereinafter; isoxazoline compounds represented by the formula (xvi):

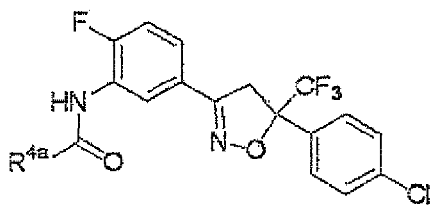

( xvi )

wherein $R^{4a}$ is any group selected from the group (A) described hereinafter; isoxazoline compounds represented by the formula (xvii):

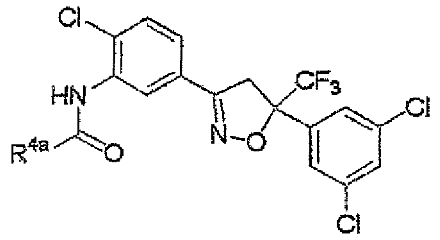

( xvii )

wherein $R^{4a}$ is any group selected from the group (A) described hereinafter; isoxazoline compounds represented by the formula (xviii).

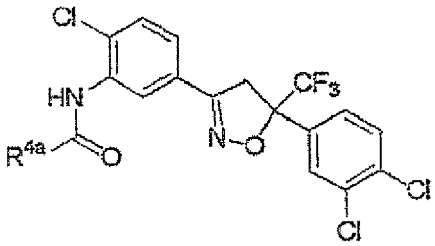

( xviii )

wherein $R^{4a}$ is any group selected from the group (A) described hereinafter; isoxazoline compounds represented by the formula (xix):

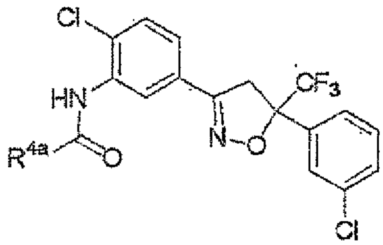

( xix)

wherein $R^{4a}$ is any group selected from the group (A) described hereinafter; isoxazoline compounds represented by the formula (xx):

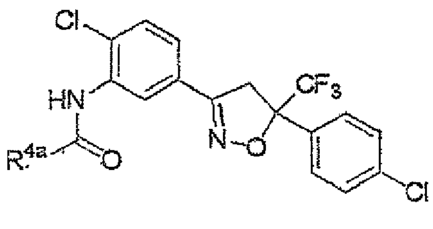

( xx )

wherein $R^{4a}$ is any group selected from the group (A) described hereinafter; isoinazoline compounds represented by the formula (xxi):

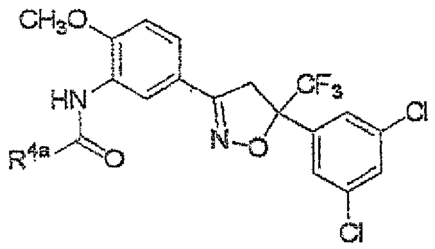

( xxi )

wherein $R^{4a}$ is any group selected from the group (A) described hereinafter;
isoxazoline compounds represented by the formula (xxii) :

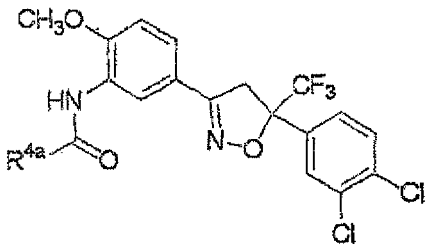

( xxii )

wherein $R^{4a}$ is any group selected from the group (A) described hereinafter;
isoxazoline compounds represented by the formula (xxiii);

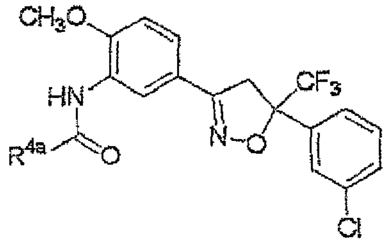

( xxiii)

wherein $R^{4a}$ is any group selected from the group (A) described hereinafter; and
isoxazoline compounds represented by the formula (xxiv) :

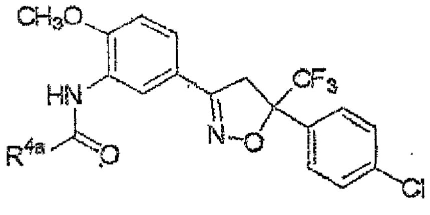

( xxiv )

wherein $R^{4a}$ is any group selected from the group (A) described hereinafter.

Group (A):

Methyl group, ethyl group, propyl group, isopropyl group, butyl group, isobutyl group, sec-butyl group, tert-butyl group, pentyl group, 1-methylbutyl group, 2-methylbutyl group, 3-methylbutyl group, 1-ethylpropyl group, 1,1-dimethylpropyl group, 1,2-dimethylpropyl group, 2,2-dimethylpropyl group, hexyl group, 1-methylpentyl group, 2-methylpentyl group, 1,1-dimethylbutyl group, 1,3-dimethylbutyl group, heptyl group, octyl group, nonyl group, decyl group, undecyl

group, dodecyl group, methoxycarbonylmethyl group, ethoxycarbonylmethyl group, propoxycarbonylmethyl group, isopropoxycarbonylmethyl group, tert-butoxycarbonylmethyl group; cyanomethyl group, 2-phenylvinyl group, 2-methoxyethoxy group, 2-ethoxyethoxy group, 2-propoxyethoxy group, 2-isopropoxyethoxy group, 2-tert-butoxyethoxy group, benzyloxy group, allyloxy group, fluoromethyl group, chloromethyl group, bromomethyl group, iodomethyl group, difluoromethyl group, chlorofluoromethyl group, dichloromethyl group, bromofluoromethyl group, trifluoromethyl group, chlorodifluoromethyl group, dichlorofluoromethyl group, trichloromethyl group, bromodifluoromethyl group, bromochlorofluoromethyl group, difluoroiodomethyl group, 2-fluoroethyl group, 2-chloroethyl group, 2-bromoethyl group, 2,2-difluoroethyl group, 2-chloro-2-fluoroethyl group, 2,2-dichloroethyl group, 2-bromo-2-fluoroethyl group, 2,2,2-trifluoroethyl group, 2-chloro-2,2-difluoroethyl group, 2,2-dichloro-2-fluoroethyl group, 2,2,2-trichloroethyl group, 2-bromo-2,2-difluoroethyl group, 1,1,2,2-tetrafluoroethyl group, 1,1,2,2,2-pentafluoroethyl group, 1-chloro-1,2,2,2-tetrafluoroethyl group, 2-chloro-1,1,2,2-tetrafluoroethyl group, 1,2-dichloro-1,2,2-trifluoroethyl group, 1-bromo-1,2,2,2-tetrafluoroethyl group, 2-bromo-1,1,2,2-tetrafluoroethyl group, 2-fluoropropyl group, 2-chloropropyl group, 2,3-dichloropropyl group, 3,3,3-trifluoropropyl group, 3-bromo-3,3-difluoropropyl group, 2,2,3,3-tetrafluoropropyl group, 2,2,3,3,3-pentafluoropropyl group, 1,1,2,3,3,3-hexafluoropropyl group, 1,1,2,2,3,3,3-heptfluoropropyl group, 2,3-dichloro-1,1,2,3,3-pentafluoropropyl group, 2-fluoro-1-methylethyl group, 2-chloro-1-methylethyl group, 2-bromo-1-methylethyl group, 2,2,2-trifluoro-1-(trifluoromethyl)ethyl group, 1,2,2,2-tetrafluaro-1-(trifluoromethyl)ethyl group, 1,1,2,2,3,3,4,4,4-nonafluorobutyl group, cyclopropyl group, 1-methylcyclopropyl group, 2-methylcyclopropyl group, 2,2-dimethylcyclopropyl group, 2,2,3,3-tetramethylcyclopropyl group, cyclobutyl group, cyclopentyl group, 1-methylcyclopentyl group, 2-methylcyclopentyl group, 3-methylcyclopentyl group, cyclohexyl group, 1-methylcyclohexyl group, 2-methylcyclohexyl group, 3-methylcyclohexyl group, 4-methylcyclohexyl group, cycloheptyl group, cyclooctyl group, 2-fluorocyclopropyl group, 2-chlorocyclopropyl group, 2,2-difluorocyclopropyl group, 2,2-dichlorocyclopropyl group, 2,2-dibromocyclopropyl group, 2,2-difluoro-1-methylcyclopropyl group, 2,2-dichloro-1-methylcyclopropyl group, 2,2,3,3-tetrafluorocyclobutyl group, 2-chloro-2,3,3-trifluorocyclobutyl group, cyclopropylmethyl group, 1-methylcyclopropylmethyl group, cyclobutylmethyl group, cyclopentylmethyl group, cyclohexylmethyl group, cycloheptylmethyl group, 2-methoxyethyl group, 2-ethoxyethyl group, 2-propoxyethyl group, 2-isopropoxyethyl group, 2-butoxyethyl group, 2-isobutoxyethyl group, 2-(sec-butoxy)ethyl group, 2-(tert-butoxy)ethyl group, vinyl group, 1-propenyl group, 2-propenyl group, 1-methylethenyl group, 2-butenyl group, 1-methyl-2-propenyl group, 2-methyl-2-propenyl group, 2-pentenyl group, 2-methyl-2-butenyl group, 3-methyl-2-butenyl group, 2-ethyl-2-propenyl group, 1,1-dimethyl-2-propenyl group, 2-hexenyl group, 2-methyl-2-pentenyl group, 2,4-dimethyl-2,6-heptadienyl group, 3,7-dimethyl-2,6-octadienyl group, 2,2-dichlorovinyl group, 2-fluoro-2-propenyl group, 2-chloro-2-propenyl group, 3-chloro-2-propenyl group, 2-bromo-2-propenyl group, 3-bromo-2-propenyl group, 3,3-difluoro-2-propenyl group, 2,3-dichloro-2 -propenyl group, 3,3-dichloro-2-propenyl group, 2,3-dibromo-2-propenyl group, 2,3,3-trifluoro-2-propenyl group, 2,3,3-trichloro-2-propenyl group, 1-(trifluoromethyl)ethenyl group, 3-chloro-2-butenyl group, 3-bromo-2-butenyl group, 4,4-difluoro-3-butenyl group, 3,4,4-trifluoro-3-butenyl group, 3-chloro-4,4,4-trifluoro-2-butenyl group, 3-bromo-2-methyl-2-propenyl group, ethynyl group, 1-propynyl group, 2-propynyl group, 2-butynyl group, 1-methyl-2-propynyl group, 2-pentynyl group, 1-methyl-2-butynyl group, 1,1-dimethyl-2-propynyl group, 2-hexynyl group, 2-chloroethynyl group, 2-bromoethynyl group, 2-iodoethynyl group, 3-chloro-2-propynyl group, 3-bromo-2-propynyl group, 3-iodo-2-propynyl group, methoxy group, ethoxy group, propoxy group, isopropoxy group, butoxy group, isobutoxy group, sec-butoxy group, tert-butoxy group, pentyloxy group, hexyloxy group, difluoromethoxy group, trifluoromethoxy group, chlorodifluoromethoxy group, bromodifluoromethoxy group, 2-fluoroethoxy group, 2-chloroethoxy group, 2,2,2-trifluoroethoxy group, 1,1,2,2-tetrafluoroethoxy group, 2-chloro-1,1,1-trifluoroethoxy group, 2-bromo-1,1,2-trifluoroethoxy group, 1,1,2,2,2-pentafluoroethoxy group, 2,2-dichloro-1,1,2-trifluoroethoxy group, 2,2,2-trichloro-1,1-difluoroethoxy group, 2-bromo-1,1,2,2-tetrafluoroethoxy group, 2,2,3,3-tetrafluoropropoxy group, 1,1,2,3,3,3-hexafluoropropoxy group, 2,2,2-trifluoro-1-(trifluoromethyl)ethoxy group, 1,1,2,2,3,3,3-heptafluoropropoxy group, 2-bromo-1,1,2,3,3,3-hexafluoropropoxy group, 2-pyridyl group, 3-methyl-2-pyridyl group, 4-methyl-2-pyridyl group, 5-methyl-2-pyridyl group, 6-methyl-2-pyridyl group, 3-fluoro-2-pyridyl group, 4-fluoro-2-pyridyl group, 5-fluoro-2-pyridyl group, 6-fluoro-2-pyridyl group, 3-chloro-2-pyridyl group, 4-chloro-2-pyridyl group, 5-chloro-2-pyridyl group, 6-chloro-2-pyridyl group, 3-bromo-2-pyridyl group, 4-bromo-2-pyridyl group, 5-bromo-2-pyridyl group, 6-bromo-2-pyridyl group, 3-pyridyl group, 2-methyl-3-pyridyl group, 4-methyl-3-pyridyl group, 5-methyl-3-pyridyl group, 6-methyl-3-pyridyl group, 2-fluoro-3-pyridyl group, 4-fluoro-3-pyridyl group, 5-fluoro-3-pyridyl group, 6-fluoro-3-pyridyl group, 2-chloro-3-pyridyl group, 4-chloro-3-pyridyl group, 5-chloro-3-pyridyl group, 6-chloro-3-pyridyl group, 2-methylthio-3-pyridyl group, 4-methylthio-3-pyridyl group, 5-methylthio-3-pyridyl group, 6-methylthio-3-pyridyl group, 2-trifluoromethyl-3-pyridyl group, 4-trifluoromethyl-3-pyridyl group, 5-trifluoromethyl-3-pyridyl group, 6-trifluoromethyl-3-pyridyl group, 5,6-dichloro-3-pyridyl group, 2,6-dichloro-3-pyridyl group, 6-cyano-3-pyridyl group, 4-pyridyl group, 2-fluoro-4-pyridyl group, 3-fluoro-4-pyridyl group, 2-chloro-4-pyridyl group, 3-chloro-4-pyridyl group, 2,6-dichloro-4-pyridyl group, 3,5-dichloro-4-pyridyl group, 2-pyridylmethyl group, 3-pyridylmethyl group, 4-pyridylmethyl group, 2-furyl group, 2-methyl-2-furyl group, 5-bromo-2-furyl group, 5-nitro-2-furyl group, 3,4-dibromo-2-furyl group, 3-furyl group, 2-methyl-3-furyl group, 2,5-dimethyl-3-furyl group, methylamino group, ethylamino group, propylamino group, isopropylamino group, butylamino group, isobutylamino group, tert-butylamino group, dimethylamino group, ethyl(methyl)amino group, diethylamino group, propyl (methyl) amino group, isopropyl (methyl) amino group,

dipropylamino group, butyl(methyl)amino group, isobutyl(methyl)amino group, tert-butyl(methyl)amino group, pyrrolidin-1-yl group, 2-methylpyrrolidin-1-yl group, 2-ethylpyrrolidin-1-yl group, 2-propylpyrrolidin-1-yl group, 2-isopropylpyrrolidin-1-yl group, 2-tert-butylpyrrolidin-1-yl group, 3-methylpyrrolidin-1-yl group, 3-ethylpyrrolidin-1-yl group, 2,5-dimethylpyrrolidin-1-yl group, 3,4-dimethylpyrrolidin-1-yl group, 3,3-diraethylpyrrolidin-1-yl group, piperidino group, 2-methylpiperidino group, 2-ethylpiperidino group, 2-propylpiperidino group, 2-isopropylpiperidino group, 2-tert-butylpiperidino group, 2-sec-butylpiperidino group, 3-methylpiperidino group, 3-ethylpiperidino group, 3-propylpiperidino group, 3-isopropylpiperidino group, 3-tert-butylpiperidino group, 3-sec-butylpiperidino group, 4-methylpiperidino group, 4-ethylpiperidino group, 4-propylpiperidino group, 4-isopropylpiperidino group, 4-tert-butylpiperidino group, 4-(trifluoromethyl)piperidino group, 2,6-dimethylpiperidino group, 2,4-dimethylpiperidino group, 2,5-dimethylpiperidino group, 3,5-dimethylpiperidino group, 2,2-dimethylpiperidino group, 3,3-dimethylpiperidino group, 4,4-dimethylpiperidino group, 3-ethyl-6-methylpiperidino group, 3-ethyl-5-methylpiperidino group, 3,5-diethylpiperidino group, 2,3-dimethylpiperidino group, 3,3,5-trimethylpiperidino group, 2,3,5,6-tetramethylpiperidino group, 3,3,5,5-tetramethylpiperidino group, hexamethyleneimino group, 2-methylhexamethyleneimino group, 2-ethylhexamethyleneimino group, 3-methylhexamethyleneimino group, 3-ethylhexamethyleneimino group, 4-methylhexamethyleneimino group, 4-ethylhexamethyleneimino group, heptamethyleneimino group, morpholino group, phenyl group, 2-methylphenyl group, 3-methylphenyl group, 4-methylphenyl group, 2-(trifluoromethyl)phenyl group, 3-(trifluoromethyl)phenyl group, 4-(trifluoromethyl)phenyl group, 2-fluorophenyl group, 3-fluorophenyl group, 4-fluorophenyl group, 2-chlorophenyl group, 3-chlorophenyl group, 4-chlorophenyl group, 2-bromophenyl group, 3-bromophenyl group, 4-bromophenyl group, 2-iodophenyl group, 3-iodophenyl group, 4-iodophenyl group, 2-cyanophenyl group, 3-cyanophenyl group, 4-cyanophenyl group, 2-nitrophenyl group, 3-nitrophenyl group, 4-nitrophenyl group, 2-methoxyphenyl group, 3-methoxyphenyl group, 4-methoxyphenyl group, 2-trifluoromethoxyphenyl group, 3-trifluoromethoxyphenyl group, 4-trifluoromethoxyphenyl group, 2,3-dichlorophenyl group, 2,4-dichlorophenyl group, 2,5-dichlorophenyl group, 2,6-dichlorophenyl group, 3,4-dichlorophenyl group, 3,5-dichlorophenyl group, 2,3,4-trichlorophenyl group, 2,4,6-trichlorophenyl group, 3,4,5-trichlorophenyl group, 2,3-difluorophenyl group, 2,4-difluorophenyl group, 2,5-difluorophenyl group, 2,6-difluorophenyl group, 3,4-difluorophenyl group, 3,5-difluorophenyl group, 2,3,4-trifluorophenyl group, 2,4,6-trifluorophenyl group, 3,4,5-trifluorophenyl group, 2,3,5,6-tetrafluorophenyl group, 2,3,4,5,6-pentafluorophenyl group, 3,5-bis(trifluoromethyl)phanyl group, 2,3-dimethoxyphenyl group, 2,4-dimethoxyphenyl group, 2,5-dimethoxyphenyl group, 2,6-dimethoxyphenyl group, 3,4-dimethoxyphenyl group, 3,5-dimethoxyphenyl group, 2-methanesulfonylphenyl group, 3-methanesulfonylphenyl group, 4-methanesulfonylphenyl group, 4-benzoylphenyl group, 4-methoxycarbonylphenyl group, 4-ethoxycarbonylphenyl group, phenylmethyl group, (2-methylphenyl)methyl group, (3-methylphenyl)methyl group, (4-methylphenyl)methyl group, {2-(trifluoromethyl)phenyl)methyl group, {3-(trifluoromethyl)phenyl}methyl group, {4-(trifluoromethyl)phenyl}methyl group, (2-fluorophenyl)methyl group, (3-fluorophenyl)methyl group, (4-fluorophenyl)methyl group, (2-chlorophenyl)methyl group, (3-chlorophenyl)methyl group, (4-chlorophenyl)methyl group, (2-bromophenyl)methyl group, (3-bromophenyl)methyl group, (4-bromophenyl)methyl group, (2-iodophenyl)methyl group, (3-iodophenyl)methyl group, (4-iodophenyl)methyl group, (2-cyanophenyl)methyl group, (3-cyanophenyl)methyl group, (4-cyanophenyl) methyl group, (2-nitrophenyl)methyl group, (3-nitrophenyl)methyl group, (4-nitrophenyl)methyl group, (2-methoxyphenyl)methyl group, (3-methoxyphenyl)methyl group, (4-methoxyphenyl)methyl group, (2-trifluoromethoxyphenyl)methyl group, (3-trifluoromethoxyphenyl)methyl group, (4-trifluoromethoxyphenyl) methyl group, (2,3-dichlorophenyl) methyl group, (2,4-dichlorophenyl)methyl group, (2,5-dichlorophenyl)methyl group, (2,6-dichlorophenyl) methyl group, (3,4-dichlorophenyl)methyl group, (3,5-dichlorophenyl)methyl group, (2,3,4-trichlorophenyl)methyl group, (3,4,5-trichlorophenyl)methyl group, (2,4,6-trichlorophenyl)methyl group, (2,3,5,6-tetrachlorophenyl)methyl group, (2,3-difluorophenyl)methyl group, (2,4-difluorophenyl)methyl group, (2,5-difluorophenyl)methyl group, (2,6-difluorophenyl) methyl group, (3,4-difluorophenyl)methyl group, (3,5-difluorophenyl)methyl group, (2,3,4-trifluorophenyl)methyl group, (3,4,5-trifluorophenyl)methyl group, (2,4,6-trifluorophenyl)methyl group, (2,3,5,6-tetrafluorophenyl)methyl group, (2,3,4,5,6-pentafluorophenyl)methyl group, {3,5-bis(trifluoromethyl)phenyl}methyl group, (2,3-dimethoxyphenyl)methyl group, (2,4-dimethoxyphenyl)methyl group, (2,5-dimethoxyphenyl)methyl group, (2,6-dimethoxyphenyl) methyl group, (3,4-dimethoxyphenyl)methyl group, (3,5-dimethoxyphenyl)methyl group, 1-phenylethyl group, 2-phenylethyl group, 1-(2-methylphenyl)ethyl group, 2-(2-methylphenyl)ethyl group, 1-(3-methylphenyl)ethyl group, 2-(3-methylphenyl)ethyl group, 1-(4-methylphenyl) ethyl group, 2-(4-methylphenyl)ethyl group, 1-{2-(trifluoromethyl)phenyl} ethyl group, 2-{2-(trifluoromethyl)phenyl}ethyl group, 1-{3-(trifluoromethyl)phenyl}ethyl group, 2-{3-(trifluoromethyl)phenyl}ethyl group, 1-{4-(trifluoromethyl)phenyl}ethyl group, 2-{4-(trifluoromethyl)phenyl)ethyl group, 1-(2-fluorophenyl)ethyl group, 2-(2-fluorophenyl)ethyl group, 1-(3-fluorophenyl)ethyl group, 2-(3-fluorophenyl)ethyl group, 1-(4-fluorophenyl) ethyl group, 2-(4-fluorophenyl)ethyl group, 1-(2-chlorophenyl)ethyl group, 2-(2-chlorophenyl)ethyl group, 1-(3-chlorophenyl)ethyl group, 2-(3-chlorophenyl)ethyl group, 1-(4-chlorophenyl)ethyl group, 2-(4-chlorophenyl)ethyl group, 1-(2-bromophenyl)ethyl group, 2-(2-bromophenyl)ethyl group, 1-(3-bromophenyl)ethyl group, 2-(3-bromophenyl)ethyl group, 1-(4-bromophenyl)ethyl group, 2-(4-bromophenyl)ethyl group, 1-(2-iodophenyl)ethyl group, 2-(2-iodophenyl)ethyl group, 1-(3-iodophenyl)ethyl group, 2-(3-iodophenyl)ethyl group, 1-(4-iodophenyl)ethyl group, 2-(4-iodophenyl)ethyl group, 1-(2-cyanophenyl)ethyl group, 2-(2-cyanophenyl)ethyl group, 1-(3-cyanophenyl)ethyl group, 2-(3-cyanophenyl)ethyl group, 1-(4-cyanophenyl)ethyl group, 2-(4-cyanophenyl)ethyl group, 1-(2-nitrophenyl)ethyl group, 2-(2-nitrophenyl)ethyl

group, 1-(3-nitrophenyl)ethyl group, 2-(3-nitrophenyl)ethyl group, 1-(4-nitrophenyl)ethyl group, 2-(4-nitrophenyl)ethyl group, 1-(2-methoxyphenyl)ethyl group, 2-(2-methoxyphenyl)ethyl group, 1-(3-methoxyphenyl)ethyl group, 2-(3-methoxyphenyl)ethyl group, 1-(4-methoxyphenyl)ethyl group, 2-(4-methoxyphenyl)ethyl group, 1-{2-(trifluoromethoxy)phenyl}ethyl group, 2-{2-(trifluoromethoxy)phenyl}ethyl group, 1-{3-(trifluoromethoxy)phenyl}ethyl group, 2-{3-(trifluoromethoxy)phenyl}ethyl group, 1-{4-(trifluoromethoxy)phenyl}ethyl group, 2-{4-(trifluoromethoxy)phenyl}ethyl group, phenoxymethyl group, (2-fluorophenoxy)methyl group, (3-fluorophenoxy)methyl group, (4-fluorophenoxy)methyl group, (2-chlorophenoxy)methyl group, (3-chlorophenoxy) methyl group, (4-chlorophenoxy)methyl group, (2-bromophenoxy)methyl group, (3-bromophenoxy)methyl group, (4-bromophenoxy)methyl group, (2-methoxyphenoxy)methyl group, (3-methoxyphenoxy)methyl group, (4-methoxyphenoxy)methyl group, 1-phenoxyethyl group, 2-phenoxyethyl group, 1-(2-fluorophenoxy)ethyl group, 2-(2-fluorophenoxy)ethyl group, 1-(3-fluorophenoxy)ethyl group, 2-(3-fluorophenoxy) ethyl group, 1-(4-fluorophenoxy)ethyl group, 2-(4-fluorophenoxy)ethyl group, 1-(2-chlorophenoxy)ethyl group, 2-(2-chlorophenoxy)ethyl group, 1-(3-chlorophenoxy)ethyl group, 2-(3-chlorophenoxy)ethyl group, 1-(4-chlorophenoxy)ethyl group, 2-(4-chlorophenoxy)ethyl group, 1-(2-bromophenoxy)ethyl group, 2-(2-bromophenoxy)ethyl group, 1-(3-bromophenoxy) ethyl group, 2-(3-bromophenoxy)ethyl group, 1-(4-bromophenoxy)ethyl group, 2-(4-bromophenoxy)ethyl group, 1-(2-methoxypbenoxy) ethyl group, 2-(2-methoxyphenoxy)ethyl group, 1-(3-methoxyphenoxy)ethyl group, 2-(3-methoxyphenoxy) e thyl group, 1-(4-methoxyphenoxy)ethyl group, 2-(4-methoxyphenoxy) ethyl group, benzylamino group, (2-methylphenyl)methylamino group, (3-methylphenyl)methylamino group, (4-methylphenyl)methylamino group, {2-(trifluoromethyl)phenyl}methylamino group, {3-(trifluoromethyl)phenyl}methylamino group, {4-(trifluoromethyl)phenyl}methylamino group, (2-fluorophenyl)methylamino group, (3-fluorophenyl)methylamino group, (4-fluorophenyl)methylamino group, (2-chlorophenyl)methylamino group, (3-chlorophenyl)methylamino group, (4-chlorophenyl)methylamino group, (2-bromophenyl)methylamino group, (3-bromophenyl)methylamino group, (4-bromophenyl)methylamino group, (2-methoxyphenyl)methylamino group, (3-methoxyphenyl)methylamino group, (4-methoxyphenyl)methylamino group, (1-phanylethyl)amino group, (2-phenylethyl)amino group, (methylamino)methyl group, (dimethylamino)methyl group, (ethylamino)methyl group, (diethylamino)methyl group, (methylamino)ethyl group, (dimethylamino)ethyl group, (ethylamino) ethyl group and (diethylamino)ethyl group.

As the pests against which the present compound is effective, there are exemplified injurious arthropods (e.g., injurious insects and injurious acarines) and nematodes belonging to round worms. Specific examples of the pests are as follows.

Hemiptera

Planthoppers (Delphacidae) such as small brown planthopper (Laodelphax striatellus), brown planthopper (Nilaparvata lugens) and white-backed rice planthopper (Sogatella furcifera); leafhoppers (Deltocephalidae) such as green rice leafhopper (Nephotettix cincticeps), green rice leafhopper (Nephotettix virescens) and tea green leafhopper (Empoasca onukii); aphids (Aphididae) such as cotton aphid (Aphis gossypii), green peach aphid (Mysus persicae), turnip aphid (Brevicoryne brassicae), apple aphid (Aphis spiraecola), (Macrosiphum euphorbiae), foxglove aphid (Aulacorthum solani), grain aphid (Rhopalosiphum padi), tropical citrus aphid (Toxoptera citricidus) and mealy plum aphid Hyalopterus pruni); bugs (Pentatomidae) such as common green stink bug (Nezara antennata), bean bug (Riptortus clavetus), (Leptocorisa chinensis), (Eysarcoris parvus) and brown-marmorated stink bug (Halyomorpha mista); whiteflies (Aleyrodidae) such as greenhouse whitefly (Trialeurodes vaporariorum), sweet potato whitefly (Bemisia tabaci) , silver leaf whitefly (Bemisia argentifolii), citrus whitefly (Dialeurodes citri) and citrus spiny whitefly (Aleurocanthus spiniferus); scales (Coccidae) such as California red scale (Aonidiella aurantii), San Jose scale (Comstockaspis perniciosa), citrus snow scale (Unaspis cirri), red wax scale (Ceroplastes rubens), cottony cushion scale (Icerya purchasi), (Planococcus kraunhiae), (Pseudococcus longispinis) and white peach scale (Pseudaulacaspis pentagona); lace bugs (Tingidae); psyllids (Psyllidae); etc.

Lepidoptera

Pyralid moths (Pyralidae) such as rice stem borer (Chilo ouppressalis), yellow rice borer (Tryporyza incertulas), rice leaf roller (Cnaphalocrocis medinalis), cotton pyralid (Notarcha derogate), (Plodia interpunctella), European corn borer (Ostrinia furnacalis), (Hellula undalis) and shibatsutoga (Pediasia teterrellus); Noctuidae such as tobacco cutworm (Spodoptera litura), beet armyworm (Spodoptera exigua), rice armyworm (Pseudaletia separata), cabbage armyworm (Mamestra brassicae), black cutworm (Agrotis ipsilon), cabbage looper (Plusia nigrisigna), Tricoplucia spp., Heliothis spp.and Helicoverpa spp.; Pieridae such as common cabbageworm (Pieris rapae); tortricid moths (Tortricidae) such as Adoxophyes spp., oriental fruit moth (Grapholita molesta), soybean pod borer (Leguminivora glycinivorella), (Matsumuraeses azukivora), summer fruit tortrix (Adoxophyes orana fasciata), small tea tortrix (Adoxophyes sp.), tea tortrix (Homona magnanima), apple tortrix (Archips fuscocupreanus) and codling moth (Cydia pomonella); Gracillariidae such as tea leaf roller (Caloptilia theivora) and apple leaf miner (Phyllonorycter ringoneella); Carposinidae such as peach

fruit moth (Carposina niponensis); lyonetiid moths (Lyonetiidae) such as Lyonetra sp.; tussock moths (Lymantriidae) such as Lymantria sp. and Euproctis sp. ; yponomeutid moths (Yponomeutidae) such as diamondback moth (Plutella xylostella); Gelechiidae such as wataakamimushi (Pectinophora gossypiella) and potato tuber worm (Phthorimaea operculella); Arctiidae such as fall webworm (Rypnantria cunea) ; clothes moths (Tineidae) such as casemaking clothes moth (Tinea translucens) and webbing clothes moth (Tineola bisselliella); etc.

Thysanoptera

Western flower thrips (Frankliniella occidentalis), (Thrips parmi), tea thrips (Scirtothrips dorsalis), (Thrips tabaci), (Frankliniella intonsa), etc.

Diptera

Housefly (Musca domestica), common mosquito (Culex popiens pallens), ushiabu (Tabanus trioronus), onion maggot (Hylemya antigua), seedcorn maggot (Hylemya platura), (Anopheles sinensis), rice leaf miner (Agromyza oryzae), small rice leaf miner (Hydrellia griseola), (Chlorops oryzae), melon fly (Dacus cucurbitae), (Ceratitis capitata), pea leaf miner (Liriomyza trifolii), (Liriomyza sativae), (Chromatomyia horticola), etc.

Coleoptera

Twenty-eight-spotted ladybird (Epilachna vigintioctopunctata), cucurbit leaf beetle (Aulacophora femoralis), striped flea beetle (Phyllotreta striolata), rice leaf beetle (Oulema oryzae), rice plant weevil (Echinocnemus squameus), rice water weevil (Lissorhoptrus oryzophilus), boll weevil (Anthonomus grandis), adzuki bean weevil (Callosobruchus chinensis), (Sphenophorus venatus), Japanese beetle (Popillia japonica). Cupreous chafer (Anomala cuprea), corn rootworms (Diabrotica spp.), Colorado potato beetle (Leptinotarsa decemlineata), click beetles (Agriotes spp.), tobacco beetle (Lasioderma serricorne), (Anthrenus verbasci), rust-red flour beetle (Tribolium castaneum), powderpost beetle (Lyctus brunneus), white spotted longicorn (Angplophora malasiaca), pine shoot beetle (Tomicus piniperda), etc.

Orthoptera

Migratory locust (Locusta migratoria), mole crickets (Gryllotalpa africana), (Oxya yazoensis), (Oxya japonica), etc.

Hymenoptera

Cabbage sawfly (Athalia rosae), (Acromyrmex spp.), fire ant (Solenopsis spp.), etc.

Nematoda

Rice white tip nematode (Aphelenchoides besseyi), strawberry bud nematode (Nothotylenchus acris), southern root-knot nematode (Meloidogyne incognita), northern, root-knot nematode (Meloidogyne hapla), Javanese root-knot nematode (Meloidogyne javanica), soy bean cyst nematode (Heterodera glycines), potato cyst nematode (Globodera rostochiensis), coffee root-lesion nematode (Pratylenchus coffeae), wheat root-lesion nematode (Pratylenchus neglects), etc.

Dictyoptera

German cockroach (Blattella germanica), smokybrown cockroach (Periplaneta fuliginosa), American cockroach (Periplaneta americana), brown cockroach (Periplaneta brunnea), oriental cockroach (Blatta orientalis), etc.

Acarina

Spider mites (Tetranychidae) such as two-spotted spider mite (Tetranychus urticae), Kanzawa spider mite (Tetranychus kanzawai), citrus red mite (Panonychus citri), European red mite (Panonychus ulmi) and Oligonychus sp.; eriophyid mites (Eriophyidae) such as pink citrus rust mite (Aculops pelekassi), (Phyllocoptruta citri), tomato rust mite (Aculops lycopersici), purple tea mite (Calacarus carinatus), pink tea rust mite (Acaphylla theavagrans) and (Eriophyes chibaensis); tarsonemid mites (Tarsonemidae) such as broad mite (Polyphagotarsonemus latus); false spider mites (Tenuipalpidae) such as (Brevipalpus phoenicis); Tuckerellidae; Ixodidae such as (Haemaphysalis longicornis), (Haemaphysalis flava), (Dermacentor taiwanicus), (Ixodes ovatus), Schulze tick (Ixodes persulcatus), (Boophilus microplus) and (Rhipicephalus sanguineus) ; acarid mites (Acaridae) such as mold mite (Tyrophagus putrescentiae) and (Tyrgphagus similis); Pyroglyphidae such as (Dermatophagoides farinae) and (Dermatophagoides ptrenyssnus); Cheyletidae such as (Cheyletus eruditus), (Cheyletus malaccensis) and (Cheyletus moorei); Arachnida; etc.

Although the composition for controlling pests of the present invention may be the present compound itself, the present compound is usually formulated into various formulations, for example, emulsifiable concentrates, oil formulations, dusts, granules, wettable powders, flowable concentrates microcapsules, aerosols, smoking formulation, poisonous baits, resin formulations or the like, by mixing the present compound with an inert carrier such as a solid carrier, liquid carrier and gaseous carrier and optionally adding a surfactant or other auxiliaries for formulation. These formulations usually contain the present compound in an amount of 0.01 to 95% by weight.

The solid carrier used for formulation includes, for example, fine powders and granules of clays (e.g., kaolin clay, diatomaceous earth, bentonite, fubasami clay and acid clay), synthetic hydrated silicon dioxide, talcs, ceramics, and other inorganic minerals (e.g., sericite, quartz, sulfur, activated carbon, calcium carbonate and hydrated silica), and chemical fertilizers (e.g., ammonium sulfate, ammonium phosphate, ammonium nitrate, urea and ammonium chlorine).

The liquid carrier includes, for example, water, alcohols (e.g., methanol, ethanol, isopropyl alcohol, butanol, hexanol, benzyl alcohol, ethylene glycol, propylene glycol and phenoxyethanol), ketones (e.g., acetone, methyl ethyl ketone and cyclohexanone), aromatic hydrocarbons (e.g., toluene, xylene, ethylbenzene, dodecylbenzene, phenylxylylethane and methylnaphthalene), aliphatic hydrocarbons (e.g., hexane, cyclohexane, kerosene and light oil), esters (e.g., ethyl acetate, butyl acetate, isopropyl myristate, ethyl oleate, diisopropyl adipate, diisobutyl adipate and propylene glycol monomethyl ether acetate), nitriles (e.g., acetonitrile and isobutyronitrile), ethers (e.g., diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether and 3-methoxy-3-methyl-l-butanol), acid amides (e.g., N,N-dimethylformamide and N,N-dimethylacetamide), halogenated hydrocarbons (e.g., dichloromethane, trichloroethane and carbon tetrachloride), sulfoxides (e.g., dimethyl sulfoxide), propylene carbonate, and vegetable oils (e.g., soybean oil and cotton seed oil).

The gaseous carrier includes, for example, fluorocarbon, butane gas, LPG (liquefied petroleum gas), dimethyl ether and carbon dioxide.

The surfactant includes, for example, nonionic surfactants such as polyoxyethylene alkyl ethers, polyoxyethylene alkyl aryl ethers, polyethylene glycol fatty acid esters, etc.; and anionic surfactants such as alkllsulforlates, alkylbensenesulfonates, alkyl sulfates, etc.

The other auxiliaries for formulation include, for example, adhesive agents, dispersants, coloring agents and stabilizers. Specific examples thereof are casein, gelatin, saccharides (e.g., starch, gum arabic, cellulose derivatives and alginic acid), lignin derivatives, bentonite, synthetic water-soluble polymers [e.g., poly(vinyl alcohols)s, poly(vinylpyrrolidone)s and poly (acrylic acid)sl, PAP (acidic isopropyl phosphate), BHT (2,6-di-tert-batyl-4-methylphenol), BHA (a mixture of 2-tert-butyl-4-methoxyphenol and 3-tert-butyl-4-methoxyphenol), etc.

In the method for controlling pests of the present invention, the pests are usually controlled by applying the composition for controlling pests of the present invention either to the pests directly or to a locus (e.g., a plant, soil, the interior of a house, and an animal) where the pests inhabit.

In the method for controlling pests of the present invention, the present compound can be used as it is, though as said method, a Method is usually adopted in which the present compound is formulated into the above-mentioned form of the composition for controlling pests of the present invention and the formulation obtained is applied to the pests or their habitat by the same method as in the case of conventional pesticidal compositions to bring the compound into contact with the aforesaid pests which appear or to allow the pests two take the compound.

As the habitat of the pests in the present invention, there are exemplified paddy fields, upland fields, orchards, non-crop lands and houses.

Such an application method includes, for example, spray treatment, soil treatment, seed treatment, and hydroponic solution treatment.

Specifically, the spray treatment in the present invention is a treating method in which controlling effect on the pests is obtained by treating the surface of a plant or the pests themselves with the active ingredient (the present compound), for example, by foliage application or trunk application.

The soil treatment is a treatment method in which soil, watering solution or the like is treated with the active ingredient in order to infiltrate the active ingredient through the root or the like into a crop plant to be protected against damages (e.g. eating) from the pests and thus the crop plant is protected against the damages from the pests. Specific examples of the soil treatment are planting pit treatments (e.g., planting pit application and incorporation of the active ingredient into planting pit treatment soil), plant foot treatments (e.g., plant foot application, incorporation of the active ingredient into plant foot soil, plant foot watering, and plant foot treatment in the latter half of raising-of-seedling time), planting furrow treatments (e.g., planting furrow application and incorporation of the active ingredient into planting furrow soil), row treatments (e.g., row application, incorporation of the active ingredient into row soil, and growing-season row

application), sowing-time row treatments (e.g., sowing-time row application, and sowing-time incorporation of the active ingredient into row soil), overall treatments (e.g., overall soil application, and overall incorporation of the active ingredient into soil), soil applications (e.g., growing-season granules foliar application, application under tree crown or around trunk, soil surface application, incorporation of the active ingredient into soil surface, sowing hole application, levee ground surface application, and crop space application), drench treatments (e.g., soil drench, raising-of-seedling time drench, liquid chemical injection, ground's edge drench, liquid chemical drip irrigation, and chemigation), nursery box treatments (e.g., nursery box application, and nursery box drench), nursery tray treatments (e.g., nursery tray application, and nursery tray drench), nursery bed treatments (e.g., nursery bed application, nursery bed drench, lowland nursery bed application, and seedling soaking), bed soil incorporation treatments (e.g., incorporation of the active ingredient into bed soil, and pre-sowing incorporation of the active ingredient into bed soil), and other treatments (e.g., incorporation of the active ingredient into molding, plowing-in, incorporation of the active ingredient into surface soil, incorporation of the active ingredient into soil in rainfall area, planting position treatment, granules corolla application, and incorporation of the active ingredient into a paste fertilizer).

The seed treatment is a treating method in which controlling' effect on the pests is obtained by directly treating seeds, seed tubers, bulbs or the like of a crop plant to be protected against damages (e.g. eating) from the pests with the active ingredient or by treating their vicinity with the active ingredient. Specific examples of the seed treatment are blow application, coating treatment, soaking treatment, impregnation treatment, painting treatment, film coat treatment, pellet coat treatment, etc.

The hydroponic solution treatment is a treatment method in which a hydroponic solution or the like is treated with the active ingredient in order to infiltrate the active ingredient through the root or the like into a crop plant to be protected against damages (e.g. eating) from the pests and thus the crop plant is protected against the damages from the pests. Specific examples of the hydroponic solution treatment are incorporation of the active ingredient into the hydroponic solution, and mixing of the active ingredient with the hydroponic solution.

When the composition for controlling- pests of the present invention is used for controlling pests in the agriculture field, its applying dosage is usually 1 to 10,000 g (in terms of the present compound) per 10,000 $m^2$. When the composition for Controlling pests of the present invention is a formulation such as an emulsifiable concentrate, a wettable powder, a flowable concentrate or the like, it is usually applied after having been diluted so as to have an active ingredient concentration of 0.01 to 10,000 ppm. When the composition for controlling pests is a formulation such as granules, dust or the like, it is applied as it is.

Such a formulation or an aqueous dilution of the formulation may be directly applied to the pests or plants such as crops to be protected against the pests, or the soil of an arable land may be treated with the formulation or the aqueous dilution in order to control pests living in the soil.

The composition for controlling pests of the present invention can be applied also by a method such as winding of a sheet-shaped or string-shaped resin formulation of the composition around a crop, stretching of the resin formulation around the crop, or spreading of the resin formulation on plant foot soil.

When the composition for controlling pests of the present invention is used for controlling pests (e.g., flies, mosquitoes and cockroaches) living in houses, its applying dosage is usually 0.01 to 1,000 mg (in terms of the present compound) per $m^2$ of treatment area in the case of application on a plane, and is usually 0.01 to 500 mg (in terms of the present compound) per $m^3$ of treatment space in the case of application in a space. When the composition for controlling pests of the present invention is a formulation such as an emulsifiable concentrate, a wettable powder, a flowable concentrate or the like, it is usually applied after having been diluted so as to have an active ingredient concentration of 0.1 to 1,000 ppm. When the composition for controlling pests is a formulation such as an oil formulation, an aerosol, a smoking formulation, a poisonous bait or the like, it is applied as it is.

The present compound can be used as an insecticide for crop lands such as upland field, paddy field, lawn, orchard, etc. and non-crop lands. In some cases, the present compound can control insect pests, for example, in crop lands where the following "crops" or the like are cultivated, without inflicting chemical injury on the crops and the like.

Field crops: corn, rice, wheat, barley, rye, oat, sorghum, cotton, soybean, peanut, buckwheat, beet, rape, sunflower, sugar cane, tobacco, etc.

Vegetables: Solanaceae (e.g., eggplant, tomato, green pepper, pepper and potato), Cucurbotaceae (e.g., cucumber, pumpkin, zucchini, watermelon and melon), Cruciferae (e.g., Japanese radish, turnip, horseradish, kohlrabi, Chinese cabbage, cabbage, leaf mustard, broccoli and cauliflower), Compositae (e.g., edible burdock, garland chrysanthemum, globe artichoke and lettuce), Liliacede (e.g., Welsh onion, onion, garlic and asparagus), Umbelliferae (e.g., carrot, parsley, celery and Pstinaca), Chenopodiales (e.g., spinach and chard), Lamiaceae (e.g., perilla, mint and basil), strawberry, sweet potato, Chinese yam, taro, etc.

Flowers and ornamental plants.

Ornamental foliage plants.

Fruit trees: pomaceous fruits (e.g., apple, pear, Japanese pear, Chinese quince and quince), stone fruits (e.g., peach, plum, nectarine, Japanese apricot, cherry, apricot and prune), citrus fruits (e.g., satsuma mandarin, orange,

lemon, lime and grapefruit), nut trees (e.g., chestnut, walnut, hazel, almond, pistachio, cashew nut and macadamia nut), berries (e.g., blueberry, cranberry, blackberry and raspberry), grape, Japanese persimmon, olive, loquat, banana, coffee, date palm, coconut palm, etc.

Trees other than fruit trees: tea, mulberry, flowering trees and shrubs, street trees (Japanese ash, birch, flowering dogwood, blue gum, ginkgo, lilac, maple, oak, poplar, Chinese redbud, Formosa sweet gum, plane tree, zelkova, Japanese arborvitae, fir, Japanese hemlock, needle juniper, pine, Japanese spruce and Japanese yew), etc.

The above-mentioned "crops" also include crops having resistance to herbicides such as HPFD inhibitors (e.g., isoxaflutol), ALS inhibitors (e.g., imazetapyl and thifensulflonmethyl), EPSP synthetase inhibitors, glutamine synthetase inhibitors, bromoxynil, etc. which has been imparted by a classic breeding method or a gene recombination technology.

As the "crops" having the resistance imparted by the classic breeding method, there are exemplified Clearfield (registered trade name) resistant to imidazolinone herbicides (e.g., imazetapyl) and STS soybean resistant to sulfonylurea ALS inhibition type herbicides (e.g., canola and thifensulflonmethyl). As crops having the resistance imparted by the gene recombination technology, corn cultivars resistant to glyphosate and glyfosinate are exemplified and are already on the market under the trade names of RoundupReady (registered trade name) and LibertyLink (registered trade name) .

The above-mentioned "crops" also include crops which a gene recombination technology has enabled to synthesize a selective toxin known in the case of, for example, Bacillus.

As toxins produced in such genetically modified plants, there are exemplified insecticidal proteins derived from Bacillus cereus and Bacillus popilliae; insecticidal proteins such as δ-endotoxins (e.g., CrylAb, CrylAc, CrylF, CrylFa2, Cry2Ab, Cry3A, Cry3Bbl and Cry9C), Viz1, VIP2, VIP3, VIP3A, etc. , which are derived from Bacillus thuringiensis; toxins derived from nematodes; toxins produced by animals, such as scorpion toxin, spider toxin, bee toxin, insect-specific neurotoxins, etc.; filamentous fungi toxins; plant lectins; agglutinin; protease inhibitors such as trypsin inhibitors, serine protease inhibitors, patatin, cystatin, papain inhibitors, etc.; ribosome-inactivating proteins (RIP) such as ricin, corn-RIP, abrin, ruin, sapolin, priodin, etc. ; steroid metabolic enzymes such as 3-hydroxysteroid oxidase, ecdysteroid-UDP-glucosyltransferase, cholesterol oxidase, etc.; ecdysone inhibitors; HMG-COA reductase; ion channel inhibitors such as sodium channel inhibitors, calcium channel inhibitors, etc. ; juvenile hormone esterase; diuretic hormone receptors; stilbene synthetase; bibenzyl synthetase; chitinase; and glucanase.

The toxins produced in such genetically modified crops also include hybrid toxins, partly deficient toxins and modified toxins of insecticidal proteins such as δ-endotoxin proteins (e.g., CrylAb, CrylAc, CrylF, CrylFa2, Cry2Ab, Cry3A, Cry3Bbl and Cry9C), VIP1, VIP2, VIP3, VIP3A, etc . The hybrid toxins are produced by a novel combination of the different domains of such a protein by adopting a recombination technology. As the partly deficient toxin, CrylAb deficient in a part of the amino acid sequence is known. In the modified toxins, one or more amino acids of a natural toxin have been replaced.

Examples of such toxins and genetically modified plants capable of synthesizing such toxins are described in EP-A-0 374 753, WO 93/0727B, WO 95/34656, EP-A-0 427 529, EP-A-451 878, WO 03/052073, etc.

The toxins contained in such genetically modified plants impart resistance to insect pests of Coleoptera, insect pests of Diptera and insect pests of Lepidoptera to the plants.

Genetically modified plants containing one or more insecticidal insect-resistant genes and capable of producing one or more toxins have already been known, and some of them are on the market. As these genetically modified plants, there are exemplified YieldGard (a registered trade name) (a corn cultivar capable of producing CrylAb toxin), YieldGard Rootworm (a registered trade name) (a corn cultivar capable of producing Cry3Bb1 toxin), YieldGard Plus (a registered trade name) (a corn cultivar capable of producing CrylAb and cry3Bb1 toxins), Herculex I (a registered trade name) (a corn cultivar capable of producing phosphinotrysin N-acetyltransferase (PAT) for imparting resistance to CrylFa2 toxin and Glyfosinate), NuCOTN33B (a registered trade name) (a cotton cultivar capable of producing CrylAc toxin), Bollgard I (a registered trade name) (a cotton cultivar capable of producing Cry lay toxin), Bollgard II (a registered trade name) (a cotton cultivar capable of producing crylac and Cry2Ab toxins), VIPCOT (a registered trade name) (a cotton cultivar capable of producing VIP toxin), NewLeaf (a registered trade name) (a potato cultivar capable of producing Cry3A toxin), NatureGard (a registered trade name), Agrisure (a registered trade name), GT Advantage (GA21 glyphosate-resistant properties), Agrisure (a registered trade name), CB Advantage (Btll corn borer (CB) properties), and Protecia (a register trade name).

The above-mentioned "crops" also include crops having an ability to produce an anti-pathogenic substance having- selective action which has been imparted by a gene recombination technology. As examples of the anti-pathogenic substance, PR proteins and the like are known (PRPs, EP-A-0 392 225). Such anti-pathogenic substances and genetically modified plants capable of producing them are described in EP-A-0 392 225, WO 95/33818, EP-A-0 353 191, etc.

As such anti-pathogenic substances produced by the genetically modified plants, there are exemplified ion channel inhibitors such as sodium channel inhibitors, calcium channel inhibitors (for example, KPI, KP4 and KP6 toxins produced by viruses are known), etc., stilbene synthases; bibenzyl synthases; chitinase; glucanase; PR proteins; and anti-pathogenic substances produced by microorganisms, such as peptide antibiotics, antibiotics having a heterocyclic

ring, protein factors concerned in resistance to plant diseases (which are called plant-disease-resistant genes and are described in WO 03/000906), etc.

The composition for controlling pests of the present invention may contain other agents for controlling injurious arthropods, acaricides, nematicides, fungicides, herbicides, plant growth regulators, synergists, fertilizers, soil conditioners, feed for animals, etc.

It is also possible to produce a mixed formulation properly by blending the present compound with, for example, any of other agents for controlling pests, such as insecticides, acaricides, nematicides, fungicides, plant hormone preparations, plant growth regulators and herbicides (these agents include their isomers and salts), synergists, phytotoxity-reducing agents, pigments and fertilizers, and use the mixed formulation in spray treatment, soil treatment and hydroponic solution treatment.

As the active ingredients of the above-mentioned other agents for controlling injurious arthropods, acaricides and/or nematicides, the following are exemplified.

(1) Organophosphorus compounds

Acephate, aluminium phosphide, butathiofos, cadusafos, chlorethoxyfos, chlorfenvinphos, chlorpyrifos, chlorpyrifos-methyl, cyanophos:CYAP, diazinon, DCIP (dichlorodiisopropyl ether), dichlofenthion:ECP, dichlorvos:DDVP, dimethoate, dimethylvinphos, disulfoton, EPN, ethion, ethoprophos, etrimfos, fenthion:MPP, fenitrothion:MEP, fosthiazate, formothion, hydrogen phosphide, isofenphos, isoxathion, malathion, mesulfenfos, methidathion:DMTP, monocrotophos, naled:BRP, oxydeprofos:ESP, parathion, phosalone, phosmet: PMP, pirimiphos-methyl, pyridafenthion, quinalphos, phenthoate:PAP, profenofos, propaphos, prothiofos, pyraclorfos, salithion, sulprofos, tebupirimfos, temephos, tetrachlorvinphos, terbufos, thiometon, trichlorphon:DEP, vamidothion, etc.

(2) Carbamate compounds

Alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb:MIPC, metolcarb, methomyl, methiocarb, NAC, oxamyl, pirimicarb, propoxur:PHC, XMC, thiodicarb, xylylcarb, etc.

(3) Synthetic pyrethroid compounds

Acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, 2,3,5,6-tetrafluoro-4- (methoxymethyl) benzyl (EZ) - (1RS,3RS;1RS,3SR) -2,2-dinethyl-3 -prop-1-enylcyclopropanecarboxylate, 2,3,5,6-tetrafluoro-4-methylbenzyl (EZ)-(1RS,3RS;1RS,3SR)-2,2-dimethyl-3-prop-1-enylcyclopropanecarboxylate, 2,3,5,6-tetrafluoro-4-(methoxymethyl)benzyl (1RS, 3RS;1RS, 3SR) - 2,2-dimethyl-3-(2-methyl-l-propenyl)cyclopropanecarboxylate, etc.

(4) Nereistoxin compounds

Cartap, bensultap, thiocyclam, monosultap, bisultap, etc.

(5) Neonicotinoide compounds

Imidacloprid, nitenpyram, acetamiprid, thiamethoxam, thiacloprid, dinotefuran, clothianidin, etc.

(6) Benzoylurea compounds

Chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, etc.

(7) Phenylpyrazole compounds

Acetoprole, ethiprole, fipronil, vaniliprole, pyriprole, pyrafluprole, etc.

(8) Bt toxin insecticides

Fresh spores and produced crystalline toxin derived from Bacillus thuringiensis, and a mixture thereof.

(9) Hydrazine compounds

Chromafenozide, halofenozide, methoxyfenozide, tebufenozide, etc.

(10) Organic chlorine-containing compounds

Aldrin, dieldrin, dienochlor, endosulfan, methoxychlor, etc.

(11) Natural insecticides

Machine oil, nicotine-sulfate, etc.

(12) Other insecticides

Avermectin-B, bromopropylate, buprofezin, chlorphenapyl, cyromazine, D-D (1,3-dichloropropene), emamectin-benzoate, fenazaquin, flupyrazofos, hydroprene, indoxacarb, metoxadiazone, milbemycin-A, pymetroziae, pyridalyl, pyriproxyfen, spinosad, sulfluramid, tolfenpyrad, triazamate, flubendiamide, SI-0009, cyflumetofen, arsenic acid, benclothiaz, calcium cyanamide, calcium polysulfide, chlordane, DDT, DSP, flufenerim, flonicamid, flurimfen, formetanate, metam-ammonium, metam-sodium, methyl bromide, nidinotefuran, potassium oleate, protrifenbute, spiromesifen, sulfur, metaflumizone, spirotetramat, etc.

Acaricides:

Aaequinocyl, amitraz, benzoximate, bromopropylate, chinomethionat, chlorobenzilate, CPCES (chlorfenson), clofentezine, dicofol, etoxazole, fenbutatin oxide, fenothiocarb, fenpyroximate, fluacrypyrim, fluproxyfen, hexythiazox, propargite:BPPS, polynactins, pyridaben, pyrimidifen, tebufenpyrad, tetradifon, spirodiclofen, amidoflumet, bifenazate, cyflumetofen, etc.

Nematicides (nematicidal active ingredients)

DCIP, fosthiazate, levamisol, methylisothiocyanate, morantel tartarate, etc.
The present invention is summarized by the following preferred embodiments:

1. An isoxasoline compound represented by the formula (1) :

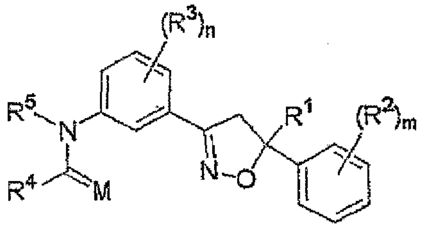

(1)

wherein $R^1$ is a $C_1$-$C_4$ haloalkyl group,
$R^2$ is a $C_1$-$C_6$ alkyl group unsubstituted or substituted with one or more halogen atoms, a $C_1$-$C_6$ alkoxy group unsubstituted or substituted with one or more halogen atoms, a halogen atom, a $C_1$-$C_6$ alkylsulfenyl group, a $C_1$-$C_6$ alkylsulfinyl group, a $C_1$-$C_6$ alkylsulfonyl group, a nitro group or a cyano group,
$R^3$ is a $C_1$-$C_6$ alkyl group, a $C_1$-$C_6$ alkoxy group, a nitro group or a halogen atom,
m is an integer of 0 to 5,
n is an integer of 0 to 4,
M is an oxygen atom or a sulfur atom,
$R^4$ is a hydrogen atom; a $C_1$-$C_{12}$ alkyl group unsubstituted or substituted with one or more halogen atoms; a

(C2-$C_5$ alkoxycarbonyl)$C_1$-$C_{12}$ alkyl group; a $C_2$-$C_{12}$ cyanoslky1 group; a $C_3$-$C_{12}$ cycloalkyl group unsubstituted or substituted with one or more halogen atoms; a ($C_3$-$C_6$ cycloalkyl) $C_1$-$C_6$ alkyl group; a ($C_1$-$C_6$ alkoxy)$C_2$-$C_6$ alkyl group; a $C_2$-$C_{12}$ alkenyl group unsubstituted or substituted with one or more halogen atoms; a $C_2$-$C_{12}$ alkynyl group unsubstituted or substituted with one or more halogen atoms; a $C_1$-$C_6$ alkoxy group unsubstituted or substituted with one or more halogen atoms; a ($C_1$-$C_6$ alkoxy)$C_1$-$C_6$ alkoxy group; a $C_3$-$C_5$ alkenyloxy group unsubstituted or substituted with one or more halogen atoms; a benzyloxy group; a phenyl($C_2$-$C_6$)alkenyl group; a ($C_1$-$C_6$ alkylamino)$C_1$-$C_5$ alkyl group; a (di($C_1$-$C_6$ alkyl)amino)$C_1$-$C_6$ alkyl group; a group represented by the following formula:

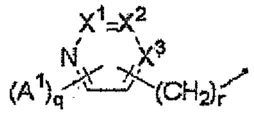

[wherein $A^1$ is a $C_1$-$C_4$ alkyl group unsubstituted or substituted with one or more halogen atoms, a $C_1$,-$C_4$ alkoxy group unsubstituted or substituted with one or more halogen atoms, a halogen atom or a cyano group,
q is an integer of 0 to 3,
r is an integer of 0 to 2, and
$X^1$, $X^2$ and $X^3$ represent a combination of one nitrogen atom and two CH groups, or a combination of three CH groups]; a group represented by the following formula:

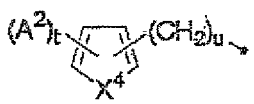

[wherein $A^2$ is a $C_1$-$C_4$ alkyl group a halogen atom or a nitro group,
t is an integer of 0 to 3,
u is an integer of 0 to 2, and
$X^4$ is an oxygen atom, a sulfur atom or NH]; a group represented by the following formula:

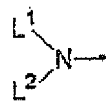

[wherein $L^1$ is a $C_1$-$C_6$ alkyl group unsubstituted or substituted with one or more halogen atoms, or a $C_3$-$C_8$ cycloalkyl group, and
$L^2$ is a hydrogen atom or a $C_1$ -$C_5$ alkyl group unsubstituted or substituted with one or more halogen atoms, or $L^1$ and $L^2$, when taken together, represent a $C_2$-$C_9$ alkanediyl group, a $CH_2CH_2CH$ ($CH_3$) $CE_2CH_2$ group or a $CH_2CH_2OCH_2CH_2$ group] ; a phenyl group; a phenyl ($C_1$-$C_4$)alkyl group; a phenoxy group; a phenoxy($_1$-$C_4$) alkyl group; a phenylamino group or a phenyl($C_1$-$C_3$)alkylamino group, wherein the benzene ring of any of said phenyl group, said phenyl ($C_1$-$C_4$) alkyl group, said phenoxy group, said phenoxy($C_1$-$C_4$)alkyl group, said phenylamino group and said Phenyl($C_1$,-$C_2$)alkylamino group may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group, and
$R^5$ is a hydrogen atom, a $C_1$-$C_2$ alkyl group, a $C_3$-$C_{12}$ cycloalkyl group, a ($C_3$-$C_6$ cycloalkyl)$C_1$-$C_6$ alkyl group, a $C_2$-$C_6$ alkoxyalkyl group, a $C_3$-$C_{12}$ alkenyl group, a $C_3$-$C_{12}$ alkynyl group, a $C_1$-$C_6$, acyl group, a $C_2$-$C_8$ cyanoalkyl group, a nitromethyl group, a $C_2$-$C_6$ alkoxycarbonyl group, a $C_3$-$C_8$ alkoxycarbonylalkyl group, a phenyl($C_1$-$C_4$)alkyl group or a benzoyl group, wherein the benzene ring of said phenyl ($C_1$-$C_4$) alkyl group or said benzoyl group may be substituted with 1 to 5 groups independently selected from the group consisting of $C_1$-$C_6$ alkyl groups unsubstituted or substituted with one or more halogen atoms, $C_1$-$C_6$ alkoxy groups unsub-

stituted or substituted with one or more halogen atoms, halogen atoms, nitro group and cyano group.

2. The isoxazoline compound according to item 1, wherein n is 0 in the formula (1).
3. The isoxazoline compound according to item 1, wherein in the formula (1), n is 1 and $R^3$ is a $C_1$-$C_6$ alkyl group as a substituent at the 6-position.
4. The isoxazoline compound according to item 1, wherein in the formula (1), n is 1 and $R^3$ is a methyl group as a substituent at the 6-position.
5. The isoxazoline compound according to item 1, wherein in the formula (1), n is 1 and $R^3$ is a halogen atom as a substituent at the 6-position.
6. The isoxazoline compound according to item 1, wherein in the formula (1), n is 1 and $R^3$ is a $C_1$-$C_4$ alkoxy group unsubstituted or substituted with one or more halogen atoms which is a substituent at the 6-position.
7. The isoxazoline compound according to any one of items 1 to 6, wherein in the formula (1), $R^4$ is a $C_1$-$C_{12}$ alkyl group unsubstituted or substituted with one or more halogen atoms.
8. The isoxazoline compound according to any one of items 1 to 6, wherein in the formula. (1), $R^4$ is a $C_2$-$C_{12}$ alkenyl group unsubstituted or substituted with one or more halogen atoms.
9. The isoxazoline compound according to any one of items 1 to 6, wherein in the formula (1), $R^4$ is a $C_1$-$C_6$ alkoxy group unsubstituted or substituted with one or more halogen atoms.
10. The isoxazoline compound according to any one of items 1 to 6, wherein in the formula (1) , $R^4$ is a phenyl group unsubstituted or substituted with 1. to 5 groups independently selected from the following group X:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group.

11. The isoxazoline compound according to item 1, wherein in the formula (1), $R^1$ is a trifluoromethyl group, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom.
12. The isoxazoline compound according to item 1, wherein in the formula (1), $R^1$ is a trifluoromethyl group, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl groups.

13. The isoxazoline compound according to item 1., wherein in the formula (1), $R^1$ is a trifluoromethyl group, m is 2, $R^2$S are chlorine atoms as substituents at the 3-position and 5-position, respectively, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom.
14. The isoxazoline compound according to item 1, wherein in the formula (1), $R^1$ is a trifluoromethyl group, m is 2, $R^2$s are chlorine atoms as substituents at the 3-position and 5-position, respectively, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ a-lkylsulfenyl groups, $C_1$-$C_6$ alkylsulfinyl groups, $C_1$-$C_6$, alkylsulfonyl groups, $C_2$-$G_6$ alkoxycarbonyl groups and benzoyl group.

15. The isoxazoline compound according to item 1, wherein in the formula (1), $R^1$ is a trifluoromethyl group, n is 0 or 1, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom.
16. The isoxazoline compound according to item 1, wherein in the formula (1), $R^1$ is a trifluoromethyl group, n is 0 or 1, $R^4$ is a phenyl group unsubstituted or substituted with 1 to 5 groups independently selected from the following

group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_5$ alkoxycarbonyl groups and benzoyl group.

17. A composition for controlling pests, which comprises an isoxazoline compound according to any one of items 1 to 16 as an active ingredient.
18. A method for controlling pests, which comprises applying an effective amount of an isoxazoline compound according to any one of items 1 to 16 to the pests or a locus where the pests inhabit .
19. Use of an isoxazoline compound according to ally one of items 1 to 1 G for controlling pests.
20. Use of an isoxazoline compound according to any one of items 1 to 16 for producing a composition for controlling pests.

Examples

The present invention is illustrated in further detail with the following production examples, formulation examples and test examples, which should not be construed as limiting the scope of the invention.
Firstly, production of the present compound is exemplified.

Production example 1

-In 1 ml of tetrahydrofuran were dissolved 99 mg of 3-(5-(3,5-dichlorophenyl)-4,5-dihydro-5-trifluoromethyl-3-isoxazolyl)aniline obtained according to Reference Production Example 2 and 47 mg of 3,3,3-trifluoropropionyl chloride, and 32 mg of triethylamine was added dropwise thereto at room temperature and stirred for 1 hour, after which the resulting- mixture was allowed to stand for 16 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 119 mg of N-(3-(5-(3,5-dichlorophenyl)-4,5-dihydro-5-trifluoromethyl-3-isoxazolyl)phenyl)-3,3,3-trifluoropropionylamide (hereinafter referred to as the present compound (1)).

The present compound (1):

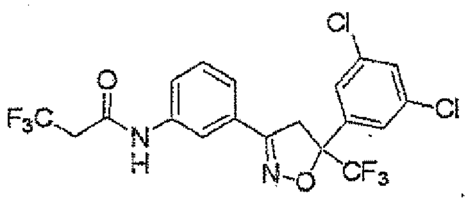

$^1$H-NMR (CDC$1_3$) δ: 7.92 (1H, t, J = 1.7 Hz), 7.50-7.44 (7H, m), 4.09 (1H, d, J = 17.4 Hz), 3.70 (1H, d, J = 17.4 Hz), 3.27 (2H, q, J= 10.3 Hz).

Production Example 2

In 1 ml of tetrahydrofuran were dissolved 99 mg of 3- (5- (3,5-dichlorophenyl) -4,5-dihydro-5-trifluoromethyl-3-isoxazolyl) aniline obtained according to Reference Production Example 2 and 52 mg of 4,4,4-trifluorobutanoyl chloride, and 32 mg of triethylamine was added dropwise thereto at room temperature and stirred for 1 hour, after which the resulting mixture ways allowed to stand for 16 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 95 mg of N-(3- (5- (3,5-diehlorophenyl) -4,5-dihydro-5-trifluoromethyl-3-isoxazolyl)phenyl) -3,3,3-trifluorobutanoylamide (hereinafter referred to as the present compound (2)).

The present compound (2):

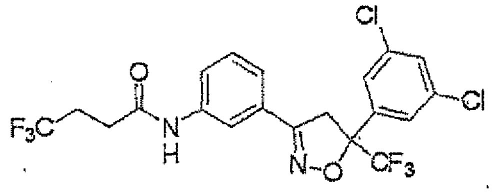

$^1$H-NMR ($CDC1_3$) δ: 7.93 (1H, s), 7.50-7.47 (4H, m), 7-42-7.38 8 (2H, m), 7.29 (1H, br s), 4.09 (1H, d, J = 17.4 Hz), 3.70 (1H, d, J = 17.4 Hz), 2.66-2.55 (4H, m).

Production Example 3

In 2 ml of tetrahydrofuran were dissolved 200 mg of 3-(5-(3,5-dichlorophenyl)-4,5-dihydro-5-trifluoromethyl-3-isoxazolyl)aniline obtained according to Reference Production Example 2 and 47 mg of 2,2,2-trichloroethyl chloroformate, and 54 mg of triethylamine was added dropwise thereto at room temperature and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 263 mg of N-(3-(5-(3,5-dichlorophenyl)-4,5-dihydro-S-trifluoromethyl-3-isoxazolyl)phenyl)-2,2,2-trichloroethyl carbamate (hereinafter referred to as the present compound (3)).

The present compound (3):

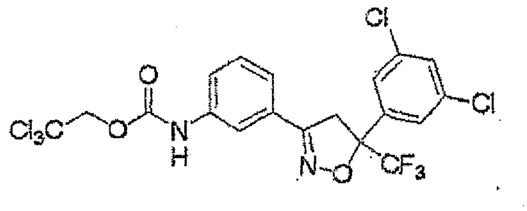

$^1$H-NMR ($CDC1_3$) δ : 7.83 (1H, s), 7.51-7.50 (2H, m), 7.47-7.38 (4H, m), 7.01 (1H, br s), 4.84 (2H, s), 4.09 (1H, d, J = 17.3 Hz), 3.70 (1H, d, J = 17.3 Hz).

Production Example 4

In 1 ml of tetrahydrofuran were dissolved 200 mg of 3- (5- (3,5-dichlorophenyl) -4,5-dihydro-5-trifluoromethyl-3-isoxazolyl)aniline obtained according to Reference Production Example 2 and 90 mg of benzoyl chloride, and 132 mg of triethylamine was added dropwise thereto at room temperature and stirred for 1 hour, after which the resulting mixture was allowed to stand for 16 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 238 mg of N-(3-(5-(3,5-dichlorophenyl) -4, 5-dihydro-5-trifluoromethyl-3-isoxazolyl)phenyl) benzamide (hereinafter referred to as the present compound (4)).

The present compound (4):

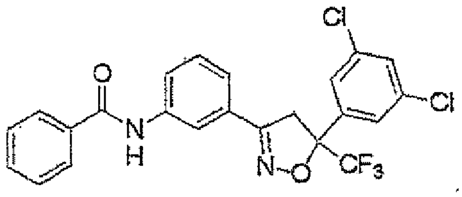

$^1$H-NMR ($CDC1_3$) δ: 8.08 (1H, s), 7.92 (1H, br s), 7.88 (2H, d, J = 8.0 Hz), 7.67 (1H, dt, J= 8.0, 1.0 Hz), 7.59-7.51 (6H, m), 7.44-7.42 (2H, m), 4.13 (1H, d, J = 17.3 Hz), 3.74 (1H, d, J = 17.3 Hz).

Production Example 5

In 1 ml of tetrahydrofuran were dissolved 200 mg of 3-(5-(3,5-dichlorophenyl)-4,5-dihydro-5-trifluoromethyl-3-isoxazolyl)aniline obtained according to Reference Production Example 2 and 105 mg of 4-cyanobenzoyl chloride, and 132 mg of triethylamine was added dropwise thereto at room temperature and stirred four 30 minutes. Water was added to the reaction mixture, followed by extraction with ethyl acetate. The organic layer was dried over anhydrous sodium sulfate and then concentrated under reduced pressure. The resultant residue was subjected to silica gel column chromatography to obtain 210 mg of N-(3-(5-(3,5-dichlorophenyl) -4, 5-dihydro-5-trifluoromethyl-3-isoxazolyl) phenyl) -4-cyanobenzamide (hereinafter referred to as the present compound (5)) .

The present compound (5):

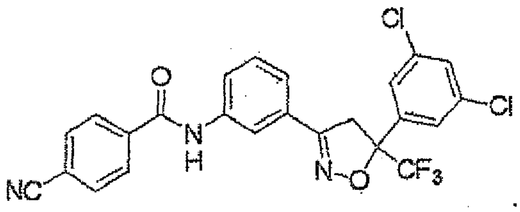

$^1$H-NMR ($CDC1_3$) δ: 8.06-8.05 (1H, m), 7.99-7.98 (3H, m), 7.82-7.80 (2H, m), 7.72-7.70 (1H, m), 7.50-7.44 (5H, m), 4.12 (1H, d, J = l7.4 Hz), 3.73 (1H, d, J = 17.4 Hz) .

Production Example 6

In 1 ml of tetrahydrofuran were dissolved 100 mg of 3-(5-(3,5-dichlorophenyl)-4,5-dihydro-5 - trifluoromethyl-3-isoxazolyl)aniline obtained according to Reference Production Example 2 and 114 mg of picolinoyl chloride hydrochloride, and 130 mg of triethylamine was added dropwise thereto at room temperature and stirred for 20 minutes. Water was added to the reaction mixture, followed by extraction with ethyl acetate. The organic layer was dried over anhydrous sodium sulfate and then concentrated under reduced pressure. The resultant residue was subjected to silica gel column chromatography to obtain 210 mg of N-(3- (-(3, 5-dichlorophenyl -4,5-dihydro-5-trifluoromethyl-3-isoxazolyl)phenyl)picolinoylamide (hereinafter referred to as the present compound (6)).

The present compound (6):

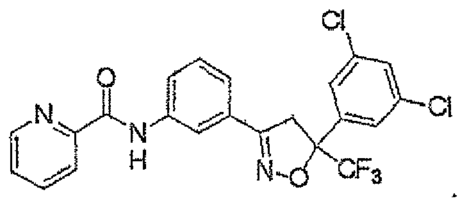

$^1$H-NMR ($CDC1_3$) δ : 10.16 (1H, s). 8.64-8.63 (1H, m), 8.30 (1H, d, J = 8.0 Hz), 8.21-8.21 (1H, m), 7.94 (1H, td, J = 7.7, 1.6 Hz), 7.78-7.76 (1H, m), 7.57-7.42 (6H, m), 4.16 (1H, d, J = 17.1 Hz) , 3.77 (1H, d, J = 17.1 Hz) .

Production Example 7

In 1 ml of tetrahydrofuran were dissolved 200 mg of 3-(5-(3,5-dichlorophenyl)-4,5-dihydro-5-trifluoromethyl-3-isoxazolyl)aniline obtained according to Reference Production Example 2 and 113 mg of 2-chloronicotinoyl chloride, and 130 mg of triethylamine was added dropwise thereto at room temperature and stirred for 10 minutes. Water was added to the reaction mixture, followed by extraction with ethyl acetate. The organic layer was dried over anhydrous sodium sulfate and then concentrated under reduced pressure. The resultant residue was subjected to silica gel column chromatography to obtain 251 mg of N-(3- (5- (3,5-dichlorophenyl) -4, 5-dihydro-5-trifluoromethyl-3-isoxa-zolyl) phenyl) -2 - chloronicotinamide (hereinafter referred to as the present compound (7)):

The present compound (7):

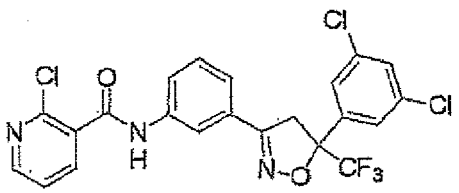

$^1$H-NMR ($CDC1_3$) δ: 8.54 (1H, dd, J = 4.8, 1.9 Hz), 8.38 (1H, br s), 8.21 (1H, dd, J = 7.7, 1.9 Hz), 8.06 (1H, t, J = 1.7 Hz), 7.70-7.68 (1H, m), 7.56-7.41 (6H, m), 4.13 (1H, d, J = 17.4 Hz), 3.74 (1H, d, J = 17.4 Hz).

Production Example 8

In 1 ml of tetrahydrofuran were dissolved 200 mg of 3-(5-(3,5-dichlorophenyl)-4,5-dihydro-5-trifluoromethyl-3-isoxazolyl)aniline obtained according to Reference Production Example 2 and 77 mg of pivaloyl chloride, and 64 mg of triethylamine, was added dropwise thereto at room temperature and stirred for 1 hour. Water was added to the reaction mixture, followed by extraction with ethyl acetate. The organic layer was dried over anhydrous sodium sulfate and then concentrated under reduced pressure. The resultant residue was subjected to silica gel column chromatography to obtain 215 mg of N-(3-(5-(3,5-dichlorophenyl) -4,5-dihydro-5-trifluoromethyl-3-isoxazolyl)phenyl)pivaloylamide (hereinafter referred to as the present compound (8)).

The present compound (8):

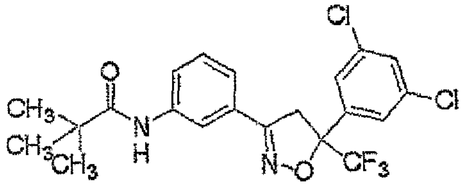

$^1$H-NMR (CDC$1_3$) δ: 8.02 (1H, t, J = 1.8 Hz), 7.50-7.49 (4H, m), 7.41-7.37 (3H, m), 4.12 (1H, d, J 17.4 Hz) , 3.72 (1H, d, J = 17.4 Hz), 1.33 (9H, s).

Production Example 9

In 1 ml of tetrahydrofuran were dissolved 200 mg of 3-(5-(3,5-dichlorophenyl)-4,5-dihydro-5-trifluoromethyl-3-isoxazolyl)aniline obtained according to Reference Production Example 2 and 58 mg of acryloyl chloride, and 65 mg of triethylamine was added dropwise thereto at room temperature and stirred for 1 hour. Water was added to the reaction mixture, followed by extraction with ethyl acetate. The organic layer was dried over anhydrous sodium sulfate and then concentrated under reduced pressure. The resultant residue was subjected to silica gel column chromatography to obtain 224 mg of N-(3-(5-(3,5-dichloropheryl)-4,5-dihydro-5-trifluoromethyl-3-isoxazolyl)phenyl)acrylamide (hereinafter referred to as the present compound (9)).

The present compound (9) :

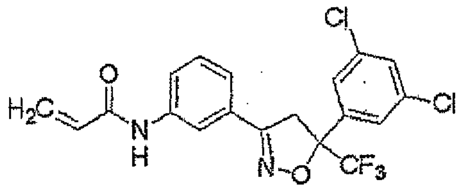

$^1$H-NMR (CDC$1_3$) δ : 8.04 (1H, s), 7.57-7.55 (1H, m), 7.52-7.49 (3H, m), 7.44-7.40 (1H, m), 7.38 (1H, d, J = 8.0 Hz),. 7.37 (1H, br s), 6.47 (1H, dd, J = 16.9, 1.2 Hz), 6.26 (1H, dd, J = 16.9, 10.1 Hz), 5.83 (1H, dd, J = 10.1, 1.2 Hz), 4.11 (1H, d, J = 17.4 Hz), 3.72 (1H, d, J = 17.4 Hz).

Production Example 10

In 3 ml of tetrahydrofuran was dissolved 200 mg of 3- (5- (3,5-dichlorophenyl) -4, 5-dihydro-5-trifluoromethyl-3-isoxazolyl)aniline obtained according to Reference Production Example 2, and 76 mg of phenyl isocyanate was added dropwise thereto at room temperature and stirred for 1 hour. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 215 mg of N-(3-(5-(3,5-dichloraphenyl)-4,5-dihydro-5-trifluoromethyl-3-isoxazolyl) phenyl) -N'-phenylurea (hereinafter referred to as the present compound (10)) .

The present compound (10):

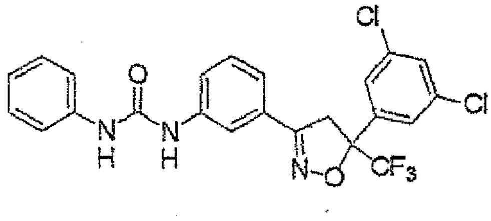

$^1$H-NMR ($CDC1_3$ δ: 7.73 (1H, t, J= 1.7 Hz), 7.48 (2H, d, J = 1.7 Hz), 7.42-7.28 (8H, m), 7.15-7.13 (1H, m), 6.98 (1H, br s), 6.82 (1H, br s), 4.04 (1H, d, J = 17.4 Hz), 3.64 (1H, d, J = 17.4 Hz).

Production Example 11

In 2 ml of tetrahydrofuran was dissolved 200 mg of 3 -(5 -(3,5-dichlorophenyl)-4,5-dihydro-5 - trifluoromethyl-3-isoxazolyl)aniline obtained according to Reference Production Example 2, and 87 mg of phenyl isothiocyanate was added dropwise thereto at room temperature and stirred for 1 hour. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 215 mg of N= (3- (5- (3,5-dichlorophenyl)-4,5-dihydro-5-trifluoromethyl-3-isoxazolyl)phenyl)-N'-phenylthiourea (hereinafter referred to as the present compound (11)).

The present compound (11):

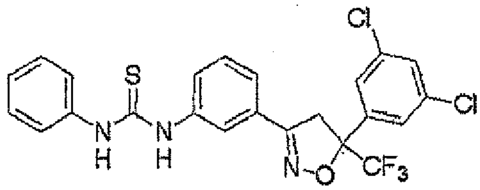

$^1$H-NMR ($CDC1_3$) δ : 7.85 (1H, br s), 7.81-7.78 (1H, m), 7.64 (1H, br s), 7.58-7.34 (11H, m), 4.07 (1H, d, J = 17.1 Hz), 3.69 (1H, d, J = 17.1 Hz).

Production Example 12

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 92 mg of benzoic acid, and 125 mug of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 178 mug of N-(5-(5-(3,5-dichlorophenyl)-4,5-dihydro-5-trifluoromethyl-3-isoxazolyl)-2-methylpheayl)benzamide (hereinafter referred to as the present compound (12)).

The present compound (12):

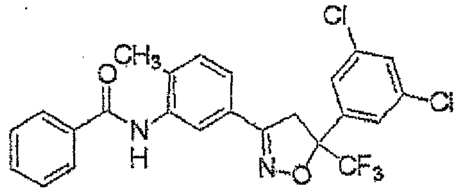

$^1$H-NMR ($CDCl_3$) 8.31-8.31 (1H, m), 7.90-7.89 (2H, m), 7.74 (1H, br s), 7.59-7.53 (6H, m), 7.42 (1H, t, J = 1.8 Hz), 7.30 (1H, d, J = 8.0 Hz), 4.14 (1H, d, J = 17.3 Hz) , 3.75 (1H, d, J = 17.3 Hz), 2.39 (3H, s).

Production Example 13

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 280 mg of 2-fluorobenzoic acid, and 125 mg of 1-[3-(diethylamino)propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 148 mg of N-(5-(5-(3,5-dichlorophenyl)-4,5-dihydro-5-trifluoromethyl-3-isoxazolyl) -2-methylphenyl) -2-fluorobenzamide (hereinafter referred to as the present compound (13)).

The present compound (13):

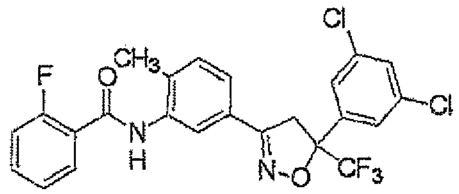

$^1$H-NMR ($CDCl_3$) δ: 8.53 (1H, d, J = 17.4 Hz), 8.44 (1H, d, J = 1.5 Hz), 8.22 (1H, td, J = 8.0, 1.9 Hz), 7.60-7.53 (4H, m), 7.41 (1H, t, J = 1.9 Hz), 7.37-7.35 (1H, m), 7.29 (1H, d, J = 8.1 Hz), 7.22 (1H, ddd, J = 12.6, 8.3, 1.0 Hz), 4.15 (1H, d, J = 17.3 Hz), 3.75 (1H, d, J = 17.3 Hz), 2.39 (3H, s).

Production Example 14

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 280 mg of 3-fluorobenzoic acid, and 125 mg of 1-[3-(diethylamino)propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 209 mg of N-(5-(5-(3,5-dichlorophenyl)-4,5-dihydro-5-trifluoromethyl-3-isoxazolyl)-2-methylphenyl)-3-fluorobenzamide (hereinafter referred to as the present compound (14)).

The present compound (14):

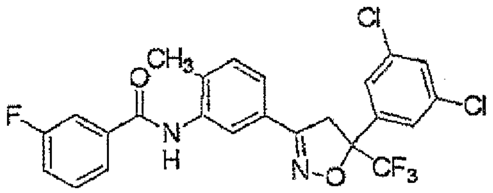

$^1$H-NMR ($CDCl_3$) δ : 8.23 (1H, d, J = 1.8 Hz), 7.74 (1H, br s), 7.66-7.56 (3H, m), 7.52-7.48 (3H, m), 7.42 (1H, t, J = 1.9 Hz), 7.32-7.26 (2H, m), 4.12 (1H, d, J = 17.3 Hz), 3.72 (1R, d, J = 17.3 Hz), 2.38 (3H, s).

Production Example 15

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 280 mg of 4-fluorobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 173 mg of N-(5-(5-(3,5-dichlorophenyl) -4,5-dihydro-5-trifluoromethyl-3-isoxazolyl)-2-methylphenyl)-4-fluorobenzamide (hereinafter referred to as the present compound (15)).

The present compound (15):

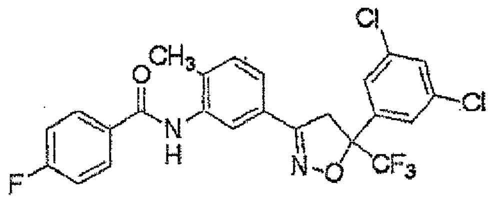

$^2$H-NMR ($CDCl_3$ ) δ . 8.21 (1H, d, J = 1.8 Hz), 7.91-7.89 (2H, m), 7.72 (1H, br s), 7.55 (1H, dd, J = 8.1, 1.8 Hz), 7.51 (2H, d, J = 1.5 Hz), 7.41 (1H, t, J = 1.8 Hz), 7.28 (1H, d, J = 8.1 Hz), 7.22-7,16 (2H, m), 4.11 (1H, d, J = 17.3 Hz), 3.72 (1H, d, J = 17.3 Hz), 2.37 (3H, s).

Production Example 16

In 1 ml of N,N-dimethylformamide were dissolved 195 mg of 5-(5-(3,5-dichlorophenyl)-4,5-dihydro-5-trifluoromethyl-3-isoxazolyl) -2-methylajiiline obtained according to Reference Production Example 5 and 313 mg of 2-chlorobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 69 mg of N-(5-(5-(3,5-dichlorophenyl)-4,5-dihydro-5-trifluoromethyl-3-isoxazolyl)-2-methylphenyl)-2-chlorobenzamide (hereinafter referred to as the present compound (16)).

The present compound (16):

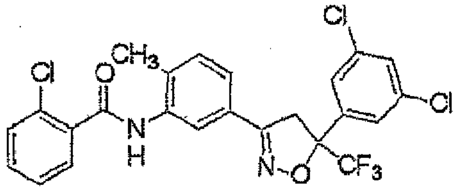

$^1$H-NMR ($CDCl_3$) δ: 8.35 (1H, s), 7.96 (1H, br s), 7.86 (1H, dd, J = 7.6, 1.6 HZ), 7.59 (1H, dd, J = 7.6, 1.6 Hz), 7.53-7.40 (6H, m), 7.29 (1H, d, J = 8.1 Hz), 4.14 (1H, d, J = 17.3 Hz) , 3.75 (1H, d, J = 17.3 Hz), 2.39 (3H, s).

Production Example 17

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 313 mg of 3-chlorobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 278 mg of N-(5-(5-(3,5-dichlorophenyl)-4,5-dihydro-5-trifluoromethyl-3-isoxazolyl)-2-methylphenyl)-3-chlorobenzamide (hereinafter referred to as the present compound (17)).

The present compound (17):

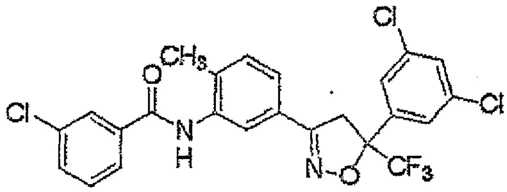

$^1$H-NMR ($CDC1_3$) δ: 8.19 (1H, d, J = 1.5 Hz), 7.87 (1H, t, J = 1.9 Hz), 7.76-7.74 (2H, m), 7.57-7.41 (6H, m), 7.29 (1H, d, J = 8.1 Hz), 4.11 (1H, d, J = 17.4 Hz), 3.72 (1H, d, J = 17.4 Hz), 2.38 (3H, s).

Production Example 18

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 313 mg of 4-chlorobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution, was added to the reaction mixture, followed by extraction with ethyl acetate. The organic layer was dried over anhydrous sodium sulfate and then concentrated under reduced pressure. The resultant residue was subjected to silica gel column chromatography to obtain 179 mg of N-(5-(5-(3,5-dichloropheayl)-4,5-dihydro-5-trifluoromethyl-3-isoxazolyl) -2-methylphenyl) -4-chlorobenzamide (hereinafter referred to as the present compound (18)).

The present compound (18) :

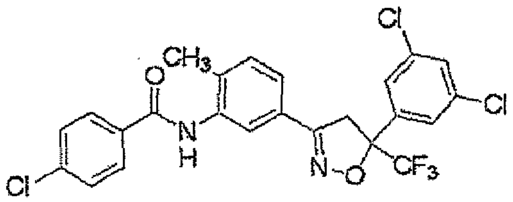

$^1$H-NMR ($CDC1_3$) δ : 8.23 (1H, d, J = 1.8 Hz), 7.84,-7.81. (2H, m), 7.69 (1H, br s), 7.57 (1H, dd, J = 7.8, 1.8 Hz), 7.51-7.48 (4H, m), 7.41 (1H, t, J = 1.9 Hz), 7.29 (1H. d, J = 8.1 Hz), 4,12. (1H, d, J = 17.4 Hz), 3.72 (1H, d, J = 17.4 Hz) , 2.37 (3H, s).

Production Example 19

In 1. ml of N,N-dimethylformamide were dissolved 195 mg of 5-(5-(3,5-dichlorophenyl)-4,5-dihydro-S-trifluoromethyl-3-isoxazolyl)-2-methylaniline obtained according to Reference Production Example 5 and 402 mg of 3-bromobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 228 mg of N-(5-(5-(3,5-dichlorophenyl)-4,5-dihydro-5-trifluoromethyl-3-isoxazolyl) -2-methylphenyl) -3-bromobenzamide (hereinafter referred to as the present compound (19)).

The present compound (19):

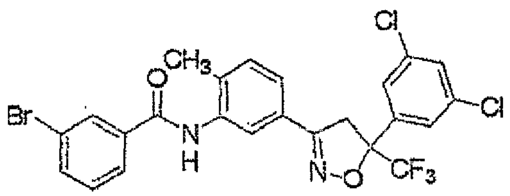

$^1$H-NMR ($CDC1_3$) δ : 8.17 (1H, d, J = 1.8 Hz), 8.03 (1H, t, J = 1.8 Hz), 7.80-7.78 (1H, m), 7.75 (1H, br s), 7.72-7.70 (1H, m), 7.56 (1H, dd, J = 7,8, 1.8 Hz), 7.51 (2H, d, J = 1.5 Hz), 7.41 (1H, t, J = 1.9 Hz) , 7.38 (1H, d, J = 7.8 Hz), 7.29 (1H, d, J = 8.1 Hz), 4.11 (1H, d, J = 17.1 Hz), 3.72 (1H, d, J = 17.1 Hz), 2.37 (3H, s).

Production Example 20

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 402 mg of 4-bromobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 192 mg of N-(5-(5-(3,5-dichlorophenyl)-4,5-dihydro- 5 -trifluoromethyl-3-isoxazolyl)-2-methylphenyl)-4-bromobenzamide (hereinafter referred to as the present compound (20)).

The present compound (20) :

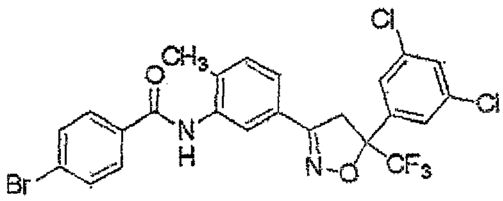

$^1$H-NMR ($CDC1_3$) δ : 8.23 (1H, d, J = 1. 8 Hz), 7.76-7.74 (2H, m), 7.69 (1H, br s), 7.67-7.65 (2H, m), 7.57 (1H, dd, J = 8.1, 1.8 Hz), 7.51 (2H, d, J = 1.8 Hz), 7.41 (1H, t, J = 1.8 Hz) , 7.29 (1H, d, J = 8.1 Hz), 4.12 (1H, d, J = 17.3 Hz), 3.72 (1H, d, J = 17.3 Hz), 2.37 (3H, s).

Production Example 21

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 334 mg of 2-nitrobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 220 mg of N-(5-(5-(3,5-dichlorophenyl)- 4,5 -dihydro-5 -trifluoromethyl- 3 - isoxazolyl)-2-methylphenyl) -2-nitrobenzamide (hereinafter referred to as the present compound (21)) .

The present compound (21) :

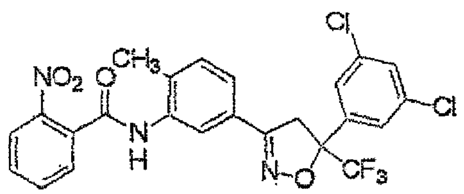

$^1$H-NMR ($CDC1_3$) δ : 8.11-8.09 (2H, m), 7.76-7.74 (1H, m), 7.67-7.51 (6H, m), 7.42 (1.H, t, J = 1.8 Hz), 7.27 (1H, d,J = 7.1 Hz), 4.12 (1H, d, J = 17.1 Hz), 3.73 (1H, d, J = 17.1 Hz), 2.33 (3H, s).

production Example 22

In 1 ml of N,N-dimetbylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 252 mg of 2-picolinic acid, and 125 mg of 1-[3-(diethylamino)propyl]-3-ethylcarbodiimide hydrochloride was added thereto at room temperature and stirred for 2 hours at room temperature. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 73 mg of N-(5-(5-(3,5-dichlorophenyl) -4,5-dihydro-5-trifluoromethyl-3-isoxasolyl)-2-methylphenyl)-2-picolylamide (hereinafter referred to as the present compound (22)).

The present compound (22):

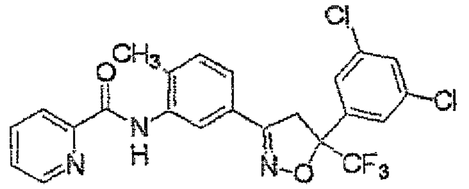

$^1$H-NMR ($CDCl_3$) δ : 10.23 (1H, br s), 8.65-8.64 (1H, m), 8.58 (1H, d, J= 1.8 Hz), 8.30 (1H, dt, J = 7.8, 1.4 Hz), 7.95 (1H, td, J = 7.8, 1.6 Hz) , 7.60 (1H, dd, J = 8.0, 1.8 Hz), 7.54-7.53 (2H, m), 7.51 (1H, dd, J = 4.8, 1.3 Hz), 7.41 (1H, t, J = 1.8 Hz), 7.29 (1H, d, J = 8.0 Hz), 4.17 (1H, d, J = 17.2 Hz), 3.77 (1H, d, J = 17.7 Hz), 2.47 (3H, s).

Production Example 23

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 294 mg of 3-cyanobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 214 mg of N- (5- (5- (3,5-dichlorophenyl)-4,5-dihydro-B-5-trifluoromethyl-3-isoxazolyl) -2-methylphenyl) -3-cyanobenzamide (hereinafter referred to as the present compound (23)).

The present compound (23):

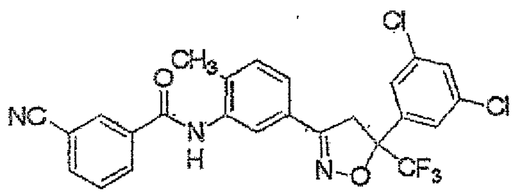

$^1$H-NMR ($CDCl_3$) δ : 8.17-8.13 (3H, m), 7.87-7.85 (2H, m) , 7.66 (1H, t, J= 7.8 Hz) , 7.55 (1H, dd, J = 7.8, 1.8 Hz), 7.50 (2H, d, J= 1.5 Hz), 7.42 (1H, t, J= 1.8 Hz), 7.31 (1H, d, J = 8.1 Hz), 4.10 (1H, d, J = 17.0 Hz), 3.72 (1H, d, J = 17.0 Hz), 2.38 (3H, s).

Production Example 24

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 294 mg of 4-cyanobenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 119 mg of N-(5-(5-(3,5-dichlorophenyl) -4,5-dihydro-5-trifluoromethyl-3-isoxazolyl)-2-methylphenyl)-4-cyanobenzamíde (hereinafter referred to as the present compound (24)).

The present compound (24):

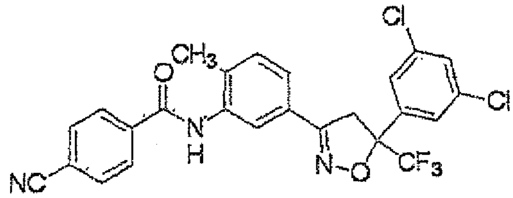

$^1$H-NMR ($CDCl_3$) δ: 8.23 (1H, d, J = 1.5 Hz), 8.00-7.98 (2H, m), 7.84-7.82 (2H, m), 7.70 (1H, br s), 7.58 (1H, dd, J = 7.8, 1.8 Hz), 7.51-7.51 (2H, m), 7.42 (1H, t, J = 1.8 Hz), 7.32 (1H, d, J= 8.1 Hz), 4.11 (1H, d, J = 17.3 Hz), 3.72 (1H, d, J= 17.3 Hz), 2.39 (3H, s).

Production Example 25

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 400 mg of 4-methylsulfonylbenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 247 mg of N-(5-(5-(3,5. dichlorophenyl)-4,5-dihydro-5-trifluoromethyl-3-isoxazolyl)-2-methylphenyl)-4-methylsulfonlbenzamide (hereinafter referred to as the present compound (25)).

The present compound (25):

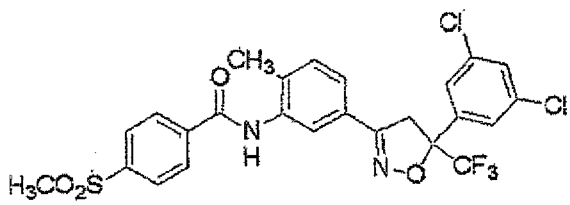

$^1$H-NMR ($CDCl_3$) δ: 8.17 (1H, d, J = 1.5 Hz), 8.08-8.03 (4H, m), 7.94 (1H, br s), 7.55 (1H, dd, J = 7.8, 1.8 Hz), 7.51-7.51 (2H, m), 7.42 (1H, t, J = 1.9 Hz), 7.32 (1H, d, J = 8.1 Hz), 4.12 (1H, d, J = 17.4 Hz), 3.73 (1H, d, J = 17.4 Hz), 3.10 (3H, s), 2.39 (3H, s).

Production Example 26

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 272 mg of 2-toluic acid, and 125 mg of 1-[3-(diethylamino)propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 76 mg of N-(5-(5-(3,5-dichlorophenyl)-4,5-dihydro-5-trifluoromethyl-3-isoxazolyl)-2-methylphenyl)-2-methylbenzamide (hereinafter referred to as the present compound (26)).

The present compound (26):

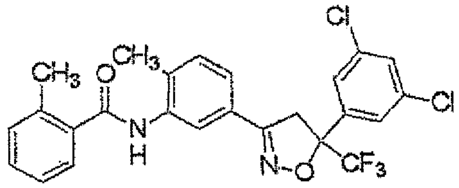

$^1$H-NMR ($CDCl_3$) δ : 8.32 (1H, br s), 7.57 (1H, dd, J = 8.1, 1.8 Hz), 7.52-7.52 (3H, m), 7.41-7.38 (3H, m), 7.31-7.28 (3H, m), 4.14 (1H, d, J = 17.3 Hz), 3.74 (1H, d, J = 17.3 Hz), 2.55 (3H, s), 2.33 (3H, s).

Production Example 27

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 272 mg of 3-toluic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 65 mg of N-(5-(5-(3,5-dichlorophenyl)-4,5-dihydro-5-trifluoromethyl-3-isoxazolyl)-2-methylphenyl)-3-methylbenzamide (hereinafter referred to as the present compound (27)).

The present compound (27):

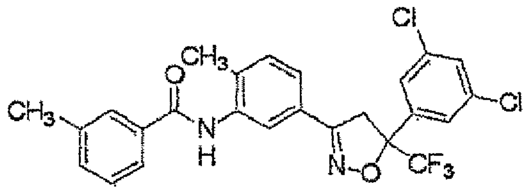

$^1$H-NMR ($CDCl_3$) δ : 8.29 (1H, d, J = 1.5 Hz), 7.73-7.71 (2H, m), 7.67-7.64 (1H, m), 7.58 (1H, dd, J = 7.8, 1.8 Hz), 7.52 (2H, d, J = 1.5 Hz), 7.42-7.40 (3H, m), 7.29 (1H, d, J = 8.1 Hz), 4.13 (1H, d, J = 17.2 Hz), 3.74 (1H, d, J = 17.2 Hz), 2.45 (3H, s), 2.38 (3H, s).

Production Example 28

In 1 ml of N,N-dimethylformamide were dissolved 195 mg of 5-(5-(3,5-dichlorophanyl)-4,5-dihydro-5-trifluoromethyl-3-isoxazolyl) -2-methylaniline obtained according to Reference Production Example 5 and 272 mg of 4-toluic acid, and 125 mg of 1-[3-(diethylamino)propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 64 mg of N- (5-(5-(3,5-dichlorophenyl)-4,5-dihydro-5-trifluoromethyl-3-isoxazolyl)-2-methylphenyl)-4-methylbenzamide (hereinafter referred to as the present compound (28)).

The present compound (28):

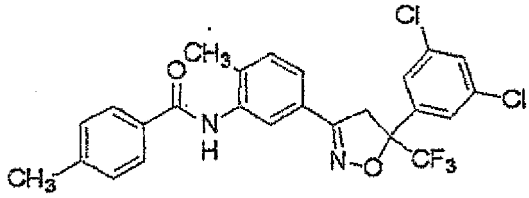

$^1$H-NMR ($CDCl_3$) δ : 8.30 (1H, d, J = 1.5 Hz), 7.80-7.77 (2H, m), 7.72 (1H, br s), 7.58 (1H, dd, J= 8.0, 1.9 Hz) , 7.52 (2H, d, J = 1.5 Hz) , 7.41 (1H, t, J = 1.8 Hz), 7.33-7.31 (2H, m), 7.28 (1H, d, J = 8.1 Hz), 4.13 (1H, d, J = 17.3 Hz), 3.74 (1H, d, J = 17.3 Hz), 2.45 (3H, s), 2.38 (3H, s).

Production Example 29

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 304 mg of 3-anisic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 74 mg of N-(5-(5-(3,5-dichlorophenyl) -4,5-dihydro-5-trifluoromethyl-3-isoxazolyl)-2-methylphenyl)-3-methoxybenzamide (hereinafter referred to as the present compound (29)).

The present compound (29):

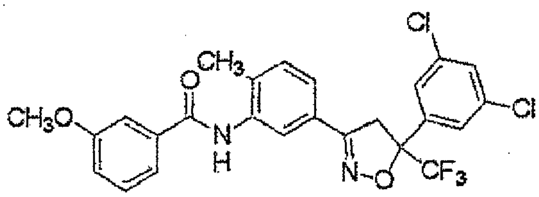

$^1$H-NMR ($CDCl_3$ ) δ: 8.30 (1H, d, J = 1.8 Hz), 7.76 (1H, br s), 7.58 (1H, dd, J = 7.8, 1.8 Hz), 7.52-7.51 (2H, m), 7.45-7.40 (4H, m), 7.29 (1H, d, J = 8.1 Hz), 7.14-7.11 (1H, m), 4.13 (1H, d, J = 17.2 Hz), 3.89 (3H, s), 3.74 (1H, d, J = 17.2 Hz), 2.38 (3H, s).

Production Example 30

In 1. ml of N,N-dimethylformamide were dissolved 195 mg of 5-(5-(3,5.=dichlorophenyl)-4,5-dihydro-5-trifluoromethyl-3-isoxazolyl)-2-methylaniline obtained according to Reference Production Example 5 and 304 mg of 4-anisic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 67 mg of N-(5-(5-(3,5-dichlorophenyl)-4,5-dihydro-5-trifluoromethyl-3-isoxazolyl)-2-methylphenyl)-4-methoxybenzamide (hereinafter referred to as the present compound (30)).

The present compound (30):

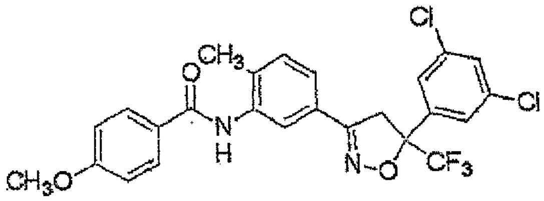

$^1$H-NMR ($CDCl_3$) δ: 8.26 (1H, d, J = 1.8 Hz;), 7.87-7.84 (2H, m), 7.71 (1H, br s), 7.56 (1H, dd, J = 8.1, 1.8 Hz), 7.51-7.51 (2H, m), 7.41 (1H, t, J = 1.8 Hz), 7.27 (1H, d, J = 8.1 Hz), 7.01-6.98 (2H, m), 4.13 (1H, d, J = 17.2 Hz), 3.89 (3H, s), 3.73 (1M, d, J= 17.2 Hz), 2.37 (3H, s).

Production Example 31

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 380 mg of 4-trifluoromethylbenzoic acid, and 125 mg of 1-[3-(diethylamino)propyll-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 204 mg of N-(5-(5-(3,5-dichlorophenyl)-4,5-dihydro-5-trifluoromethyl-3-isoxasolyl)-2-methylphenyl)-4-trifluoromethylbenzamide (hereinafter referred to as the present compound (31)).

The present compound (31):

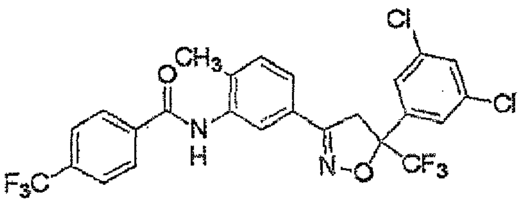

$^1$H-NMR ($CDCl_3$) δ: 8.21 (1H, d, J = 1.5 Hz), 8.00 (2H, d, J = 8.1 Hz), 7.83 (1H, br s), 7.78 (2H, d, J = 8.3 Hz), 7.55 (1H, dd, J = 7.8, 1.8 Hz), 7.51 (2H, d, J = 1.5 Hz), 7.42 (1H, t, J = 1.9 Hz), 7.29 (1H, d, J = 8.1 Hz), 4.11 (1H, d, J = 17.2 Hz), 3.72 (1H, d, J = 17.2 Hz), 2.38 (3H, s).

Production Example 32

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 452 mg of 4-benzoylbenzoic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 261 mg of N-(5-(5-(3,5-dichlorophenyl)-4,5.-dihydro-5-trifluoromethyl-3-isoxazolyl)-2-methylphenyl)-4-benzoylbenzamide (hereinafter referred to as the present compound (32)).

The present compound (32):

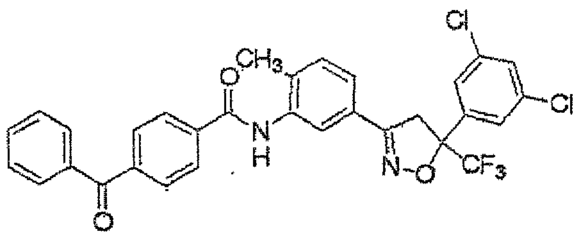

$^1$H-NMR (CDCl3 ) δ : 8.30 (1H, d, J = 1.5 Hz), 8.01-7.99 (2H, m), 7.95-7.92 (2H, m), 7.85-7.82 (2H, m), 7.79 (1H, br s), 7.67-7.59 (2H, m), 7.54-7.51 (4H, m), 7.43-7.41 (1H, m), 7.32 (1H, d, J = 8.3 Hz), 4.14 (1H, d, J = 17.3 Hz), 3.75 (1H, d, J = 17.3 Hz), 2.41 (3H, s).

Production Example 33

In 1 ml of N,N-dimethylformamide were dissolved 195 mg of 5-(5-(3,5-dichlorophenyl)-4,5-diliydro-5-trifluoromethyl-3-isoxazolyl)-2-methylaniline obtained according to Reference Production Example 5 and 315 mg of 2-chloronicotinic acid, and 125 mg of 1-[3-(diethylamino)propyll-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 174 mg of N.(5-(5-(3,5-dichlorophenyl)-4,5-dihydro-5-trifluoromethyl-3-isoxazolyl)-2-methylphenyl)-2-chloronicotinamide (hereinafter referred to as the present compound (33)).

The present compound (33):

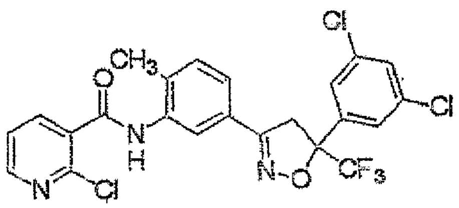

$^1$H-NMR ($CDCl_3$ ) δ : 8.55 (1H, dd, J = 4.7, 1.9 Hz), 8.31-8.30 (3H, m), 7.57 (1H, dd, J = 7.8, 1.5 Hz), 7.53-7.52 (2H, m), 7.45 (1H, dd, J = 7.7, 4.7 Hz), 7.42 (1H, t, J = 1.9 Hz), 7.31 (1H, d, J= 7.8 Hz), 4.13 (1H, d, J = 17.2 Hz), 3.74 (1H, d, J= 17.2 Hz), 2.41 (3H, s).

Production Example 34

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 315 mg of 6-chloronicotinic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 303 mg of N-(5-(5-(3,5-dichlorophenyl)-4,5-dihydro-5-trifluoromethyl-3-isoxazolyl)-2-methylphenyl)-6-chloronicotinamide (hereinafter referred to as the present compound (34)).

The present compound (34):

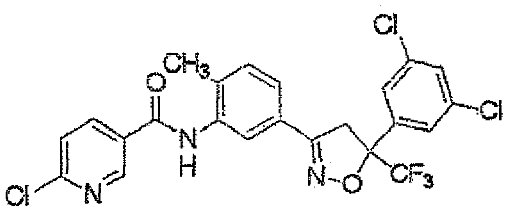

$^1$H-NMR ($CDCl_3$) δ : 8.88 (1H, d, J = 2.0 Hz), 8.18 (1H, dd, J = 8.3, 2.5 Hz), 8.13 (1H, d, J = 1.5 Hz), 7.83 (1H, br s), 7.54 (1H, dd, J = 8.1, 1.8 Hz), 7.50-7.48 (3H,m), 7.42 (1H, t, J = 1.9 Hz), 7.30 (1H, d, J = 8.3 Hz), 4.10 (1H, d, J = 17.3 Hz), 3.71 (1H, d, J = 17.3 Hz), 2.37 (3H, s).

Production Example 35

In 1 ml of N,N-dimethylformamide were dissolved 195 mg of 5-(5-(3,5-dichlorophenyl)-4,5-dihydro-5-trifluoromethyl-3 -isoxazolyl)-2-methylaniline obtained according to Reference Production Example 5 and 246 mg of nicotinic acid, and 125 mg of 1-[3-(diethylamino)propyll-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 211 mg of N-(5-(S-(3,5-dichlorophenyl)-4,5-dihydro-5-trifluoromethyl-3-isoxazolyl) - 2 -methylphenyl) nicotinamide (hereinafter referred to as the present compound (35)).

The present compound (35):

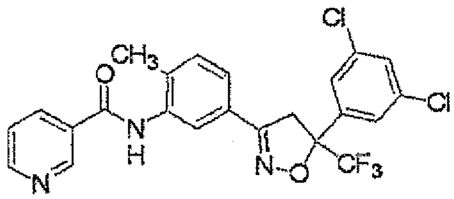

$^1$H-NMR ($CDCl_3$) δ 9.12 (1H, d, J = 1.8 Hz), 8.79 (1H, dd, J = 4.9, 1.6 Hz), 8.23 (1H, ddd, J = 8.1, 2.3, 1.8 Hz), 8.16 (1H, d, J = 1.5 Hz), 8.00 (1H, br s), 7.54 (1H, dd, J = 8.1, 1.8 Hz), 7.50 (2H, d, J = 1.8 Hz), 7.47 (1H, ddd, J = 7.8, 4.8, 0.8 Hz), 7.41 (1H, t, J = 1.9 Hz), 7.30 (1H, d, J 8.1 Hz), 4.11 (1H, d, J= 17.4 Hz), 3.72 (1H, d, J = 17.4 Hz), 2.38 (3H, s).

Production Example 36

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 92 mg of formic acid, and 125 mg of 1-[3-(diethylamino)propyll-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 144 mg of N-(5-(5-(3,5-dichlorophenyl)-4,5-dihydro-5-trifl-uoromethyl-3-isoxazolyl)-2-methylphenyl)formamide (hereinafter referred to as the present compound (36)).

The present compound (36):

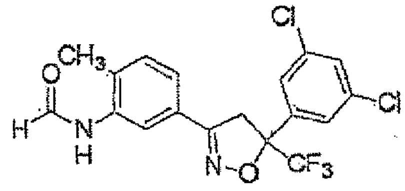

$^1$-H-NMR ($CDCl_3$) δ : 8.49 (1H, d, J= 1.5 Hz), 8.24 (1H, d, J = 1.8 Hz), 7.54 (1H, dd, J= 7.7, 1.9 Hz), 7.51 (2H, s), 7.41 (1H, t, J = 1.9 Hz), 7.26 (1H, d, J = 7.8 Hz), 7.13 (1H, br s), 4.07 (1H, d, J = 17.4 Hz), 3.70 (1H, d, J = 17.4 Hz), 2.33 (3H, s).

Production Example 37

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 120 mg of acetic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 181 mg of N-(5-(5-(3,5-dichloropheuyl)-4,5-dihydro-5-trifluoromethyl-3-isoxazolyl)-2-methylphenyl)acetamide (hereinafter referred to as the present compound (37)).

The present compound (37):

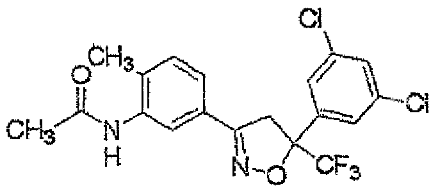

$^1$H-NMR ($CDCl_3$ ) δ : 8.09 (1H, br s), 7.52 (1H, d, J = 1.8 Hz), 7.50 (2H, d, J = 1.3 Hz), 7.41 (1H, t, J= 1.8 Hz), 7.24 (1H, d, J= 8.1 Hz), 7.01 (1H, br s), 4.08 (1H, d, J = 17.3 Hz), 3.69 (1H, d, J = 17.3 Hz), 2.30 (3H, s), 2.23 (3H, s).

Production Example 38

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 148 mg of propionic acid, and 125 mg of 1- [3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 98 mg of N-(5-(5-(3,5-dichlorophenyl)-4,5-dihydro-5-trifluoromethyl-3-isoxazolyl)-2-methylphenyl)propionylamide (hereinafter referred to as the present compound (38)).

The present compound (38):

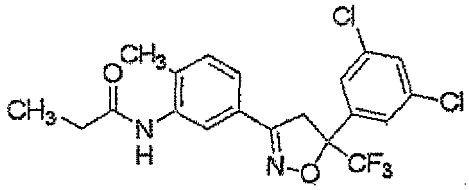

$^1$H-NMR ($CDCl_3$) δ 8.17 (1H, br s), 7.53 (1H, dd, J = 8.1, 1. 9 Hz), 7.50 (2H, d, J= 1.3 Hz), 7.41 (1H, t, J = 1.9 Hz), 7.23 (1H, d, J = 8.1 Hz), 7.01 (1H, s), 4.10 (1H, d, J = 17.2 Hz), 3.70 (1H, d, J = 17.2 Hz), 2.46 (2H, q, J = 7.5 Hz), 2.29 (3H, s), 1.28 (3H, t, J =7.5 Hz) .

Production Example 39

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 176 mg of n-butyric acid, and 125 mg of 1-[3-(diethylamino)propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 111 mg of N- (5- (5- (3,5-dichlorophenyl)-4,5-dihydro-5-trifluoromethyl-3-isoxazolyl) -2-methylphenyl) -n-butyramide (hereinafter referred to as the present compound (39)).

The present compound (39):

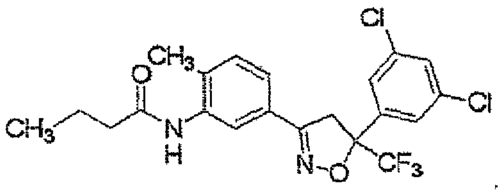

$^1$H-NMR ($CDCl_3$) δ : 8.16 (1H, br s), 7.54-7.50 (3H, m), 7.41 (1H, t, J = 1.9 Hz), 7.23 (1H, d, J = 7.8 Hz), 7.03 (1H, br s), 4.10 (1H, d, J= 17.3 Hz), 3.70 (1H, d, J = 17.3 Hz), 2.40 (2H, t, J= 7.3 Hz), 2.29 (3H, s), 1.81-1.77 (2H, m), 1.04 (3H, t, J = 7.3 Hz).

Production Example 40

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 204 mg of n-valeric acid, and 125 mg of 1-[3-(diethylamino) propyl]-3-etliylcarbodiimide hydrochloride, was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 129 mg of N-(5-(5-(3,5-dichlorophenyl)-4,5-dihydro-5-trifluoromethyl-3-isoxazolyl) -2-methylphenyl) -n-valeramide (hereinafter referred to as the present compound (40)).

The present compound (40):

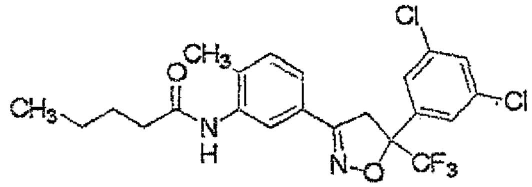

$^1$H-NMR CCDCl$_3$) δ : 8.18 (1H, br s), 7.54-7.52 (3H, m), 7.41 (1H, t, J = 1.9 Hz), 7.24 (1H, d, J = 7.8 Hz), 6.97 (1H, br s), 4.10 (1H, d, J = 17.3 Hz), 3.70 (1H, d, J = 17.3 Hz), 2.42 (2H, t, J = 7.5 Hz), 2.30 (3H, s), 1.78-1.70 (2H, m), 1.46-1.42 (2H, m), 0.97 (3H, t, J = 7.3 Hz).

Production Example 41

In 1 ml of N,N-dimethylformamide were dissolved 195 mg of 5- (5- (3, 5-dichlorophenyl) -4, 5-dihydro-5-trifluoromethyl-3-isoxazolyl) -2-methylaniline obtained according to Reference Production Example 5 and 176 mg of isobutyric acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 141 mg of N-(5-(5-(3,5-dichlorophenyl) -4, 5-dihydro-5-trifluoromethyl-3-isoxazolyl) -2-methylphenyl) isobutyramide (hereinafter referred to as the present compound (41)).

The present compound (41):

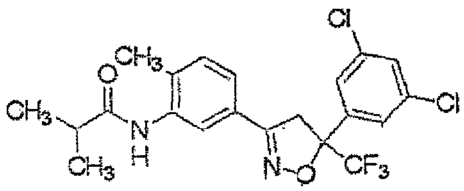

$^1$H-NMR (CDCl$_3$) δ: 8.19 (1H, br s), 7.54 (1M, dd, J = 8.1, 1.8 Hz), 7.51 (2H, d, J = 1.5 Hz), 7.41 (1H, t, J = 1.9 Hz), 7.23 (1H, d, J = 8.1 Hz), 7.07 (1H, br s), 4.10 (1H, d, J = 17.3 Hz), 3.70 (1H, d, J = 17.3 Hz), 2.64-2.54 (1H, m), 2.30 (3H, s), 1.30 (6H, dd, J = 6.8, 1.5 Hz).

Production Example 42

In 1 ml of.N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 204 mg of pivalic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixtures, followed by extraction with ethyl acetate. The organic layer was dried over anhydrous sodium sulfate and then concentrated under reduced pressure. The resultant residue was subjected to silica gel column chromatography to obtain 55 mg of N-(5-(5-(3,5-dichlorophenyl)-4,5-dihydro-5-trifluoromethyl-3-isoxazolyl)-2-methylphenyl)pivaloylamide (hereinafter referred to as the present compound (42)).

The present compounds (42):

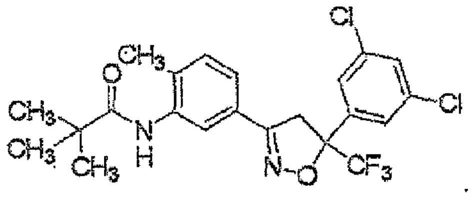

$^1$H-NMR ($CDCl_3$) δ :8.22 (1H, d, J = 1.8 Hz), 7.56 (1H, dd, J= 7.8, 1.8 Hz), 7.51 (2H, d, J = 1.5 Hz), 7.41 (1H, t, J = 1.8 Hz), 7.33 (1H, br s), 7.23 (1H, d, J = 8.1 Hz), 4.12 (1H, d, J = 17.3 Hz), 3.71 (1H, d, J = 17.3 Hz), 2.30 (3H, s), 1.36 (9H, s).

Production Example 43

In 1 ml of N,N-dimethylformamide were dissolved 195 mg of 5-(5-(3,5-dichlorophenyl)-4,5-dihydro- 5-trifluoromethyl- 3 -isoxazolyl) -2-methylaniline obtained according to Reference Production Example 5 and 204 mg of isovaleric acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 116 mg of N- (5- (5- (3, 5-dichlorophenyl)-4, 5-dihydro-5-trifluoromethyl-3-isoxazolyl)-2-methylphenyl)isovaleramide (hereinafter referred to as the present compound (43)).

The present compound (43):

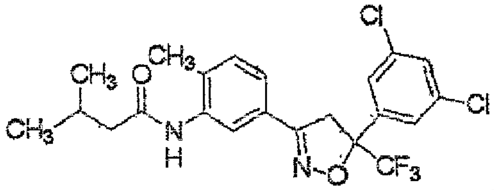

$^1$ H-NMR ($CDCl_3$) δ: 8.16 (1H, br s), 7.54-7.50 (3H, m), 7.41 (1H, t, J = 1.9 Hz), 7.23 (1H, d, J = 8.1 Hz), 7.00 (1H, br s), 4.10 (1H, d, J = 17.3 Hz), 3.70 (1H, d, J = 17.3 Hz), 2.29 (3H, s), 2.28-2.18 (3H, m), 1.05 (6H, d, J = 6.1 Hz).

Production Example 44

In 1. ml of N,N-dimethylformamide were dissolved 195 mg of 5- (5- (3, 5-dichlorophenyl)-4, 5-dihydro-5-trifluoromethyl-3-isoxazolyl)-2-methylaniline obtained according to Reference Production Example 5 and 284 mg of 4,4,4-trifluorobutyric acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 158 mg of N-(5-(5-(3,5-dichlorophenyl) -4, 5-dihydro-5-trifluoromethyl-3-isoxazolyl) -2-methylphenyl) -4,4,4-trifluorobutyramide (hereinafter referred to as the present compound (44)).

The present compound (44):

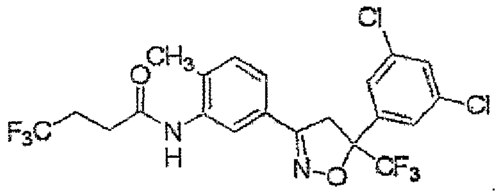

$^1$H-NMR ($CDCl_3$) δ: 8.05 (1H, br s), 7.52-7.49 (3H, m), 7.41 (1H, t, J = 1.8 Hz), 7.24 (1H, d, J = 7.8 Hz), 7.14 (1H, br s), 4.07 (1H, d, J = 17.3 Hz), 3.68 (1H, d, J = 17.3 Hz), 2.70-2.54 (4H, m), 2.29 (3H, s).

Production Example 45

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 312 mg of 5,5,5-trifluorovaleric acid, and 125 mg of 1- [3- (diethylamino) propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 164 mg of N-(5-(5-(3,5-dichlorophenyl) -4,5.dihydro-5-trifluoromethyl-3-isoxasolyl) -2-methylphenyl) -5, 5, 5-trifluorovaleramide (hereinafter referred to as the present compound (45)).

The present compound (45):

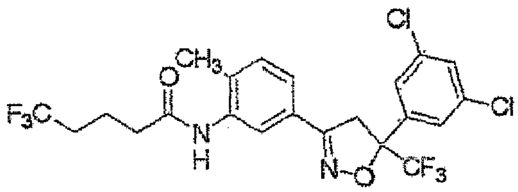

$^1$H-NMR, ($CDCl_3$) δ: 8.11 (1H, br s), 7.51-7.49 (3H, m), 7.41 (1H, t, J = 1.9 Hz), 7.24 (1H, d, J = 8.1 14z), 7.09 (1H, br s), 4.09 (1H, d, J = 17.2 Hz), 3.69 (1H, d, J = 17.2 Hz), 2.53 (2H, t, J = 7.2 Hz), 2.29 (3H, s), 2.24-2.19 (2H, m), 2.05-2.02 (2H, m).

Production Example 46

In 1 ml of N,N-dimethylformamide were dissolved 195 mg of 5- (5- (3, 5-dichlorophenyl) -4, 5-dihydro-5-trifluoromethyl-3-isoxazolyl)-2-methylaniline obtained according to Reference Production Example 5 and 144 mg of acrylic acid, and 125 mg of 1-[3-(diethylamino)propyl] --3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 106 mg of N-(5-(5-(3,5-dichlorophenyl)-4,5-dihydro-5-trifluoromethyl-3-isoxazolyl) -2-methylphenyl) -acrylamide (hereinafter referred to as the present compound (46)).

The present compound (46) :

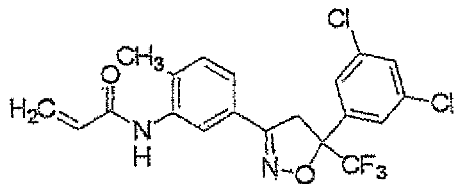

$^1$H-NMR ($CDCl_3$) δ: 8.21 (1H, br s), 7.54 (1H, dd, J = 7.8, 1.8 Hz), 7.50 (2H, d, J = 1.5 Hz), 7.41 (1H, t, J = 1.9 Hz), 7.24 (1H, d, J = 8.1 Hz), 7.21 (1H, br s), 6.47 (1H, dd, J = 16.9, 1.2 Hz), 6.31 (1H, dd, J = 16.9, 10.2 Hz), 5.82 (1H, dd, J = 10.2, 1.2 Hz), 4.10 (1H, d, J = 17.4 Hz), 3.70 (1H, d, J = 17.4 Hz), 2.31 (3H, s).

Production Example 47

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 280 mg of 2-trifluoromethylacrylic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 65 mg of N-(5-(5-(3,5-dichlorophenyl)-4,5-dihydro-5-trifluoromethyl-3-isoxazolyl) -2-methylphenyl) -2-(trifluoromethyl)acrylamide (hereinafter referred to as the present compound (47)).

The present compound (47):

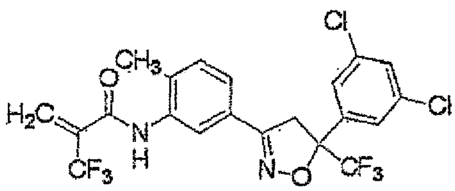

$^1$H-NMR ($CDCl_3$ ) δ: 8.19 (1H, d, J= 1.8 Hz), 7.58 (1H, dd, J = 8.1, 1.8 Hz), 7.56 (1H, br s), 7.51-7.51 (2H, m), 7.41 (1H, t, J = 1.8 Hz), 7.29 (1H, d, J = 8.1 Hz), 6.77-6.76 (1H, m), 6.42-6.42 (1H, m), 4.10 (1H, d, J = 17.3 Hz), 3.70 (1H, d, J = 17.3 Hz), 2.32 (3H, s).

Production Example 48

In 1 ml of N,N-dimethylformamide were dissolved 195 mg of 5- (5- (3, 5-dichlorophenyl) -4, 5-dihydro-5-trifluoromethyl-3-isoxazolyl) -2-methylaniline obtained according to Reference Production Example 5 and 172 mg of crotonic acid, and 125 mg of 1-[3-(diethylamino) propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 97 mg of N- (5- (5- (3, 5-dichlorophenyl)-4,5-dihydro-5-trifluoromethyl-3-isoxazolyl) -2-methylphenyl) crotonamide (hereinafter referred to as the present compound (48)).

The present compound (48):

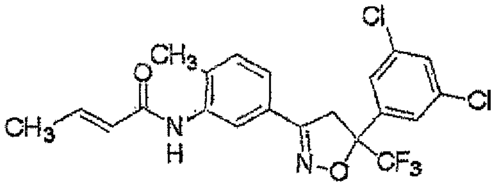

$^1$H-NMR ($CDCl_3$) δ : 8.23 (1H, br s), 7.55 (1H, dd, J = 8.0, 1.8 Hz), 7.51 (2H, d, J = 1.3 Hz), 7.41 (1H, t, J = 1.8 He), 7.24 (1H, d, J = 8.0 Hz), 7.06-7.01 (1H, m), 6.95 (1H, br s), 6.00 (1H, dd, J = 15.2, 1.7 Hz), 4.10 (1H, d, J = 17.3 Hz), 3.71 (1H, d, J = 17.3 Hz), 2.31 (3H, s), 1.95 (3H, dd, J = 6.8, 1.7 Hz).

Production Example 49

In 1 ml of N,N-dimethylformamide were dissolved 195 mg of 5-(5-(3,5-dichlorophenyl) -4, 5-dihydro-5-trifluoromethyl-3-isoxazolyl) -2-methylaniline obtained according to Reference Production Example 5 and 296 mg of cinnamic acid, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 163 mg of N-(5-(5-(3,5-dichlorophenyl)-4,5-dihydro-5-trifluoromethyl-3-isoxazolyl)-2-methylphenyl)cinnamamide (hereinafter referred to as the present compound (49)).

The present compound (49):

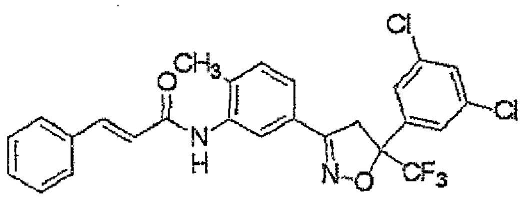

$^1$H-NMR (CDC$1_3$) δ: 8.26 (1H, br s), 7.78 (1H, d, J = 15.4 Hz), 7.54-7.53 (3H, m), 7.51 (2H, d, J = 1.5 Hz), 7.41 (1H, t, J= 1.9 Hz), 7.39-7.38 (3H, m), 7.34 (1H, br s), 7.24 (1H, d, J = 8.1 Hz), 6.61 (1H, d, J = 15.4 Hz), 4.11 (1H, d, J = 17.3 Hz), 3.70 (1H, d, J = 17.3 Hz), 2.34 (3H, s).

Production Example 50

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 180 mg of methoxyacetic acid, and 125 mg of 1-[3-(diethylainino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 98 mg of N-(5-(5-(3,5-dichlorophenyl)-4,5-dihydro-5-trifluoromethyl-3-isoxazolyl)-2-methylphenyl)methoxyacetamide (hereinafter referred to as the present compound (50)).

The present compound (50):

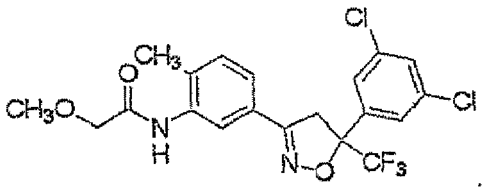

$^1$H-NMR ($CDCl_3$) δ: 8.31 (1H, br s), 8.28 (1H, d, J = 1.8 Hz) , 7.54 (1H, dd, J = 8.1, 1.8 Hz), 7.51 (2H, d, J = 1.5 Hz), 7.41 (1H, t, J 1. 8 Hz), 7.25 (1H, d, J = 8.1 Hz), 4.10 (1H, d, J= 17.3 Hz), 4.06 (2H, s), 3.71 (1H, d, J = 17.3 Hz), 3.54 (3H, s), 2.31 (3H, s).

Production Example 51

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 228 mg of trifluoroacetic acid, and 125 mg of 1-[3-(diethylamino)propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 163 mg of N-(5-(5-(3,5-dichlorophenyl)-4,5-dihydro-5-trifluoromethyl-3-isoxazolyl) -2-methylphenyl) trifluoroacetamide (hereinafter referred to as the present compound (51)).

The present compound (51) :

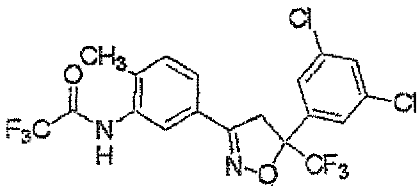

$^1$H-NMR ($CDC1_3$) δ: 8.05 (1H, d, J = 1.5 Hz), 7.79 (1H, br s), 7.61 (1H, dd, J = 8.0, 1.6 Hz), 7.50 (2H, d, J = 1.5 Hz), 7.42 (1H, t, J = 1.9 Hz), 7.33 (1H, d, J = 8.0 Hz), 4.08 (1H, d, J = 17.3 Hz), 3.69 (1H, d, J = 17.3 Hz), 2.35 (3H, s).

Production Example 52

In 1. ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 170 mg of cyanoacetic acid, and 125 mg of 1-[3-(diethylamino)propyl] -3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 218 mg of N-(5-(5-(3,5-dichlorophenyl)-4,5-dihydro-5-trifluoromethyl-3-isoxazolyl)-2-methylphenyl)cyanoacetamide (hereinafter referred to as the present compound (52)).

The present compound (52):

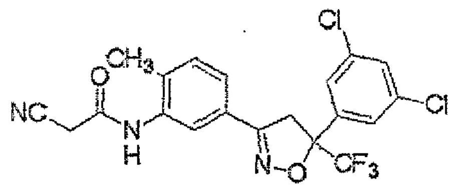

$^2$H-NMR ($CDCl_3$ ) *ð:* 8.03 (1H, d, J = 1.5 Hz) , 7.79 (1H, br s), 7.53 (1H, dd, J = 8.1, 1.8 Hz), 7.50 (2H, d, J = 1.5 Hz) , 7.42 (1H, t, J = 1.8 Hz) , 7.28 (1H, d, J = 8.1 Hz), 4.07 (1H. d, J = 17.3 Hz), 3.68 (1H, d, J = 17.3 Hz) , 3.61 (2H, s), 2.34 (3H, s).

Production Example 53

In 1 ml of N,N-dimethylformamide were dissolved 195 mg of 5-(5-(3,5-dichlorophenyl)-4,5-dihydro-5-trifluoromethyl-3-isoxazolyl) -2-methylaniline obtained according to Reference Production Example 5 and 279 mg of N,N-dimethylglycine hydrochloride, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 28 mg of N-(5- (5- (3,5-dichlorophenyl) -4,5-dihydro-5-trifluoromethyl-3-isoxazolyl) -2-methylphenyl) - (N,N-dimethylamino)acetamide (hereinafter referred to as the present compound (53)).

The present compound (53):

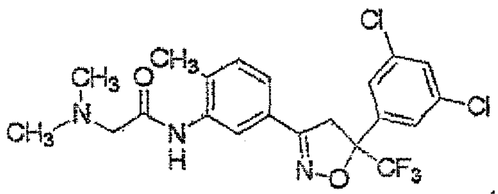

$^1$H-NMR ($CDCl_3$) δ : 9.34 (1H, br s), 8.33 (1H, d, J = 1.8 Hz), 7.54 (1H. dd, J = 8.0, 1.8 Hz), 7.51 (2H, d, J = 1.5 Hz), 7.41 (1H, t, J = 1.8 Hz), 7.24 (1H, d, J = 8.0 Hz), 4.11 (1H, d, J = 17.4 Hz), 3.71 (1H, d, J = 17.4 Hz) , 3.13 (2H, s), 2.43 (6H, s), 2.31 (3H, .s).

Production Example 54

In 1 ml of N,N-dimethylformamide were dissolved 195 mg of 5-(5-(3,5-dichlorophenyl)-4,5-dihydro-5-trifluoromethyl-3-isoxazolyl)-2-methylaniline obtained according to Reference Production Example 5 and 264 mg of monoethyl malonate, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 28 mg of N-(5-(5-(3,5-dichicrophenyl)-4,5-dihydro-5-trifluoromethyl-3-isoxazolyl) -2-methylphenyl) ethoxycarbonylacetamide (hereinafter referred to as the present compound (54)).

The present compound (54):

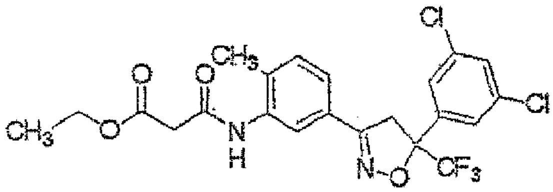

$^1$H-NMR ($CDCl_3$) δ. 9.52 (1H, br s), 8.28 (1H, d, J = 1.5 Hz), 7.54 (1H, dd, J = 8.0, 1.8 Hz) , 7.51 (2H, d, J= 1.5 Hz), 7.41 (1H, t, J = 1.8 Hz), 7.25 (1H, d, J = 8.0 Hz), 4.29 (2H, q, J = 7.2 Hz), 4.09 (1H, d, J = 17.2 Hz), 3.70 (1H, d, J = 17.2 Hz), 3.52 (2H, s), 2.37 (3H, s), 1.34 (3H, t, J = 7.2 Hz) .

Production Example 55

In 1 ml of N,N-dimethylformamide were dissolved 195 mg of 5-(S-(3,5-dichlorophenyl)-4,5-dihydro-5-trifluoromethyl-3-isoxazolyl)-2-methylaniline obtained according to Reference Production Example 5 and 347 mg of 2-pyridylacetic acid hydrochloride, and 125 mg of 1-[3-(diethylamino)propyl]-3-ethylcarbodiimide hydrochloride was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 165 mg of N-(5-(5-(3,5-dichlorophenyl)-4,5-dihydro-5-trifluoromethyl-3-isoxazolyl)-2-methylphenyl)-2-pyridylacetamide (hereinafter referred to as the present compound (55)).

The present compound (55):

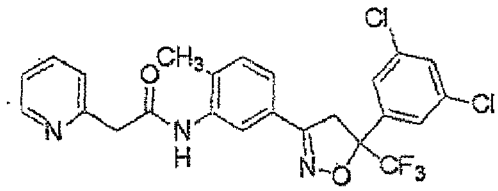

$^1$H-NMR ($CDC1_3$) 6: 10.36 (1H, br s), 8.62-8.61 (1H, m), 8.35 (1H, d, J = 1.8 Hz), 7.73 (1H, td, J = 7.7, 1.8 Hz), 7.52-7.50 (3H, m), 7.40 (1H, t, J = 1.9 Hz), 7.31-7.29 (2H, m), 7.21 (1H, d, J = 8.1 Hz) , 4.10 (1H, d, J = 17.2 Hz), 3.93 (2H, s), 3.71 (1H, d, J = 17.2 Hz) , 2.34 (3H, s).

Production Example 56

In 1 ml of tetrahydrofuran, were dissolved 195 mg of 5-(5-(3,5-dichlorophenyl)-4,5-dihydro-5-trifluoromethyl-3-isoxazolyl)-2-methylaniline obtained according to Reference Production Example 5 and 397 mg of monomethyl terephthalate chloride, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 180 mg of N-(S-(5-(3,5-dichlorophenyl)-4,5-dihydro-5-trifluoromethyl-3-isoxazolyl) -2-methylphenyl) -4-methoxycarbonylbensamide (hereinafter referred to as the present compound (56)).

The present compound (56):

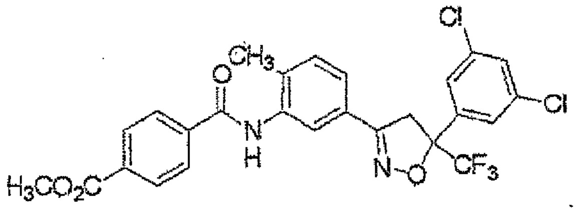

$^1$H-NMR ($CDCl_3$) δ: 8.25 (1H, d, J = 1.8 Hz), 8.19-8.17 (2H, m), 7.96-7.94 (2H, m), 7.79 (1H, br s), 7.58 (1H, dd, J = 8.0, 1.8 Hz), 7.51 (2H, d, J = 1.5 Hz), 7.42 (1H, t, J = 1. 8 Hz), 7.30 (1H, d, J = 8.0 Hz), 4.12 1H, d, J = 17.4 Hz), 3.97 (3H, s), 3.73 (1H, d, J = 17. 4 Hz), 2.39 (3H, s).

Production Example 57

In 1 ml of tetrahydrofuran were dissolved 195 mg of 5-(5-(3,5-dichlorophenyl)-4,5-dihydro-5-trifluoromethyl-3-isoxazolyl) -2-methylaniline obtained according to Reference Production Example 5 and 214 mg of dimethylcarbamoyl chloride, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 36 mg of 3-(5-(5-(3,5-dichlorophenyl)-4,5-dihydro-5-trifluoromethyl-3-isoxazolyl) -2-methylphenyl) -1, 1-dimethylurea. (hereinafter referred to as the present compound (57)).

The present compound (57):

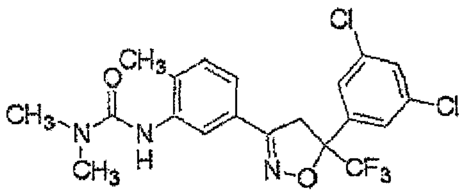

$^1$H-NMR ($CDCl_3$) δ: 8.08 (1H, d, J = 1.8 Hz), 7.50-7.48 (3H, m), 7.40 (1H, t, J = 1.9 Hz), 7.20 (1H, d, J = 8.1 Hz), 6.21 (1H, br s), 4.12 (1H, d, J = 17.2 Hz), 3.71 (1H, d, J = 17.7 Hz), 3.07 (6H, s), 2.28 (3H, s).

Production Example 58

In 1 ml of tetrahydrofuran were dissolved 195 mg of 5-(5-(3,5-dichlorophenyl)-4,5-dihydro-5-trifluoromethyl-3-isoxazolyl)-2-methylaniline obtained according to Reference Production Example 5 and 266 mg of 1-pyrrolidinecarbamoyl chloride, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 30 mg of 1-(N-(5-(5-(3,5-dichlorophenyl)-4,5-dihydro-5-trifluoromethyl-3-isoxazolyl) -2-methylphenyl) aminocarbonyl)pyrrolidine (hereinafter referred to as the present compound (58)).

The present compound (58):

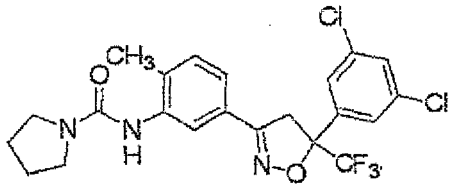

$^1$H-NMR ($CDCl_3$) δ : 8.20 (1H, d, J = 1.8 Hz), 7.50-7.49 (3H, m), 7.40 (1H, t, J = 1.8 Hz), 7.19 (1H, d, J = 8 .1 Hz) , 6.07 (1H, br s), 4.13 (1H, d, J = 17.4 Hz), 3.72 (1H, d, J = 17.4 Hz), 3.50-3.49 (4H, m), 2.28 (3H, s), 2.03-2.01 (4H, m).

Production Example 59

In 1 ml of tetrahydrofuran were dissolved 195 mg of 5-(5-(3,5-dichlorophenyl)-4,5-dihydro-5-trifluoromethyl-3-isoxazolyl)-2-methylaniline obtained according to Reference Production Example 5 and 189 mg of methyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 22 mg of N-(5-(5-(3,5-dichlorophenyl)-4,5-dihydro-5-trifluoromethyl-3-isoxazolyl)-2-methylphenyl)-O-methylcarbamate (hereinafter referred to as the present compound (59)).

The present compound (59):

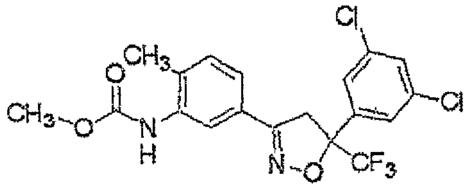

$^1$H-NMM ($CDC1_3$) δ: 8.08 (1H, br s), 7.51 (2H, d, J = 1.5 Hz), 7.48 (1H, dd, J = 7.9, 1.8 Hz), 7.41 (1H, t, J = 1.8 Hz), 7.21 (1H, d, J = 7.9 Hz), 6.47 (1H, br s) , 4.10 (1H, d, J = 17.3 Hz), 3.81 (3H, s), 3.70 (1H, d, J = 17.3 Hz), 2.28 (3H, s).

Production Example 60

In 1 ml of tetrahydrofuran were dissolved 195 mg of 5-(5-(3,5-dichlorophenyl)-4,5-dihydro-5-trifluoromethyl-3-isoxasolyl) -2-methylaniline obtained according to Reference Production Example 5 and 217 mg of ethyl chloroformate, and 101 mg of triethylamine was added thereto at room temperature and stirred for 2 hours at room temperature. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 83 mg of N-(5-(5-(3,5-dichlorophenyl)-4,5-dihydro-s-trifluoromethyl-3-isoxazolyl)-2-methylphenyl)-o-ethylcarbamate (hereinafter referred to as the present compound (60)).

The present compound (60):

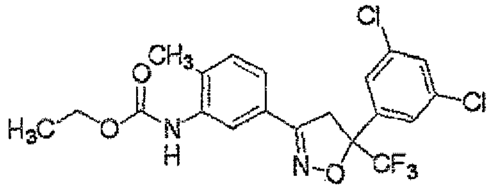

$^1$H-NMR ($CDCl_3$) δ: 8.11 (1H, br s), 7.51 (2H, d, J = 1.3 Hz), 7.48 (1H, dd, J= 8.0, 1.8 Hz), 7.41 (1H, t, J = 1.8 Hz), 7.21 (1H, d, J = 8.0 Hz), 6.46 (1H, brs), 4.25 (2H, q, J = 7.1 Hz) , 4.10 (1H, d, J = 17.4 Hz) , 3.70 (1H, d, J = 17.4 Hz), 2.29 (3H, s), 1.34 (3H, t, J = 7.1 Hz).

Production Example 61

In 1 ml of tetrahydrofuran were dissolved 195 mg of 5-(5-(3,5-dichlorophenyl)-4,5-dihydro-5-trifluoromethyl-3-isoxazolyl)-2-methylaniline obtained according to Reference Production Example 5 and 245 mg of propyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 72 mg of N-(5-(5-(3,5-dichlorophenyl)-4,5-dihydro-5-trifluoromethyl-3-isoxazolyl)-2-methylphenyl)-o-propylcarbamate (hereinafter referred to as the present compound (61)).

The present compound (61):

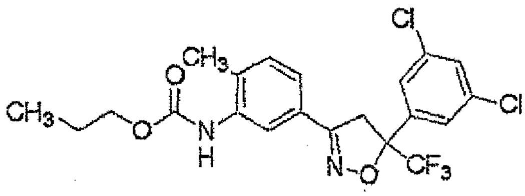

$^1$H-NMR ($CDCl_3$) δ: 8.10 (1H, br s), 7.51 (2H, d, J = 1.5 Hz), 7.48 (1H, dd, J = 8.0, 1.8 Hz), 7.41 (1H, t, J= 1.8 Hz), 7.21 (1H, d, J = 8.0 Hz), 6.46 (1H, br s), 4.15 (2H, t, J = 6.8 Hz), 4.10 (1H, d, J = 17.2 Hz), 3.70 (1H, d, J = 17.2 Hz), 2.29 (3H, s), 1.75-1.71 (2H, m), 1.00 (3H, t, J = 7.3 Hz).

Production Example 62

In 1 ml of tetrahydrofuran were dissolved 195 mg of 5- (5- (3,5-dichlorophenyl) -4,5-dihydro-5-trifluoromethyl-3-isoxazoly] -2-methylaniline obtained according to Reference Production Example 5 and 273 mg of butyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 49 mg of N- (5- (5- (3,5-dichlorophenyl) -4,5-dihydro-5-trifluoromethyl-3-isoxazolyl) -2-methylphenyl) -O-butylcarbamate (hereinafter referred to as the present compound (62)) .

The present compound (62):

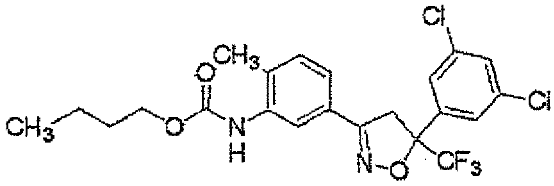

$^1$H-NMR ($CDCl_3$) δ: 8.10 (1H, br s), 7.51 (2H, d, J = 1.3 Hz), 7.48 (1H, dd, J = 8.1, 1.8 Hz), 7.41 (1H, t, J = 1.8 Hz), 7.21 (1H, d, J = 8.1 Hz), 6.45 (1H, br s), 4.20 (2H, t, J = 6.7 Hz), 4.10 (1H, d, J = 17.2 Hz), 3.70 (1H, d, J = 17.2 Hz), 2.29 (3H, s), 1.72-1.65 (2H, m), 1.48-1.39 (2H, m), 0.97 (3H, t, J = 7.5 Hz).

Production Example 63

In 1 ml of tetrahydrofuran were dissolved 195 mg of 5- (5" (3, 5-dichlorophenyl) -4,5-dihydro-5-trifluoromethyl-3-isoxazolyl)-2-methylaniline obtained according to Reference Production Example 5 and 245 mg of isopropyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 12 mg of N-(5-(5-(3,5-dichlorophenyl)- 4,5 -dihydro-5 -trifluoromethyl- 3-isoxazolyl) -2-methylphenyl) -o-isopropylcarbamate (hereinafter referred to as the present compound (63)).

The present compound (63):

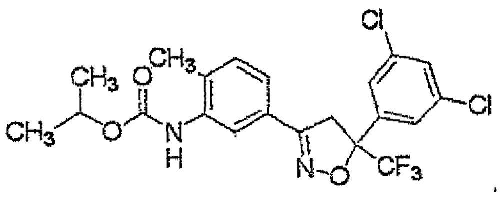

$^1$H-NMR ($CDC1_3$) δ. 8.13 (1H, br s), 7.51 (2H, d, J = 1.3 Hz), 7.47 (1H, dd, J = 8.0, 1.8 Hz), 7.41 (1H, t, J = 1.8 Hz) , 7.20 (1H, d, J = 8.0 Hz), 6.42 (1H, br s), 5.08-4.99 (1H, m), 4.11 (1H, d, J = 17.4 Hz), 3.70 (1H, d, J = 17.4 Hz), 2.29 (3H, s), 1.34 (3H, d, J = 2.3 Hz), 1.32 (3H, d, J = 2.3 Hz).

production Example 64

In 1 ml of tetrahydrofuran were dissolved 195 mg of 5-(S-(3,5-dichlorophenyl)-4,5-dihydro-5-trifluoromethyl-3-isoxazolyl)-2-methylaniline obtained according to Reference Production Example 5 and 273 mg of isobutyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 83 mg of N- (5- (5- (3, 5-dichlorophenyl) -4,5-dihydro-5-trifluoromethyl-3-isoxazolyl)-2-methylphenyl)-O-isobutylcarbamate (hereinafter referred to as the present compound (64)).

The present compound (64):

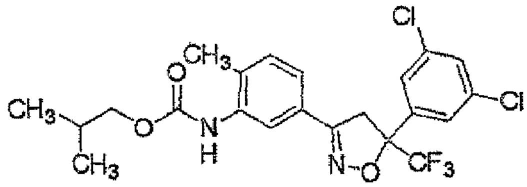

$^1$H-NMR ($CDC1_3$) $\delta$: 8.09 (1H, br s), 7.51 (2H,d, J= 1.3 Hz), 7.48 (1H, dd, J = 8.0, 1.8 Hz), 7.41 (1H, t, J = 1.8 Hz), 7.21 (1H, d, J = 8.0 Hz), 6 .47 (1H, br s), 4.10 (1H, d, J = 17.2 Hz), 3.98 (2H, d, J = 6.6 Hz), 3.70 (1H, d, J = 17.2 Hz), 3.29-3.27 (1H, m), 2.30 (3H, s), 0.98 (6H, d, J = 6.8 Hz).

Production Example 65

In 1 ml of tetrahydrofuran were dissolved 195 mg of 5-(5-(3,5-dichlorophenyl)-4,5-dihydro-5-trifluoromethyl-3-isoxazolyl) -2-methylaniline obtained according to Reference Production Example 5 and 241 mg of allyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 134 mg of N-(5-(5-(3,5-dichlorophenyl)-4,5-dihydro-5-trifluoromethyl-3-isoxasolyl) -2-methylphenyl) -0-allylca-rbamate (hereinafter referred to as the present compound (65)).

The present compound (65):

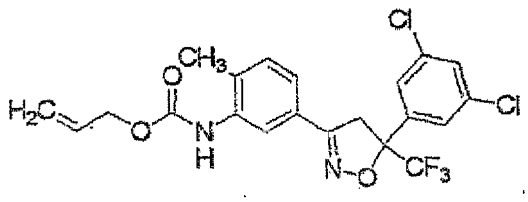

$^1$H-NMR ($CDCl_3$) δ: 8.09 (1H, br s), 7.51 (2H, d, J = 1.3 Hz), 7.48 (1H, dd, J = 8.0, 1. 8 Hz), 7.41 (1H, t, J = 1.8 Hz), 7.21 (1H, d, J = 8.0 Hz) , 6. 52 (1H, br s), 5.99 (1H, ddt, J = 17.2, 10.4, 5.8 Hz), 5.39 (1H, dq, J = 17.2, 1.4 Hz), 5.30 (1H, dq, J = 10.4, 1.4 Hz), 4.69 (2H, dt, J = 5.8, 1.4 Hz), 4.10 1H, d, J = 17.3 Hz), 3.70 (1H, d, J = 17.3 Hz), 2.29 (3H, s).

Production Example 66

In 1 ml of tetrahydrofuran were dissolved 195 mg of 5-(5-(3,5-dichlorophenyl)-4,5-dihydro-5-trifluoromethyl-3-isoxazolyl) -2-methylaniline obtained according to Reference Production Example 5 and 286 mg of 2-chloroethyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 144 mg of N-(5-(S-(3,5-dichlorophenyl)-4,5-dihydro-5-trifluoromethyl-3-isoxazolyl) -2-methylphenyl) -0- (2-chloroethyl)carbamate (hereinafter referred to as the present compound (66)) .

The present compound (66):

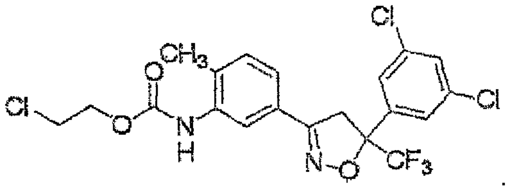

$^1$H-NMR ($CDCl_3$ ) δ 8.08-8.05 (1H, br m 7.51 (2H, d, J = 1.5 Hz), 7.48 (1H, dd, J = 8.0, 1.8 Hz), 7.41 (1H, t, J = 1.8 Hz), 7.23 (1H, d, J = 8.0 Hz), 6.57 (1H, br s), 4.47-4.44 (2H, m), 4.10 (1H, d, J = 17.3 Hz), 3.78-3.75 (2H, m), 3.70 (1H, d, J = 17.3 Hz), 2.31 (3H, s).

Production Example 67

In 1 ml of tetrahydrofuran were dissolved 195 mg of 5- (5- (3,5-dichlorophenyrl) -4,5.dihydro-5-trifluoromethyl-3 -isoxazolyl)- 2 -methylaniline obtained according to Reference Production Example 5 and 423 mg of (2,2,2-trichloroethyl) chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 293 mg of N-(5-(5-(3,5-dichlorophenyl) -4,5-dihydro-5-trifluoromethyl-3-isoxazolyl)-2-methylphanyl)-0-(2,2,2-trichloroethyl)carbamate (hereinafter referred to as the present compound (67)).

The present compound (67):

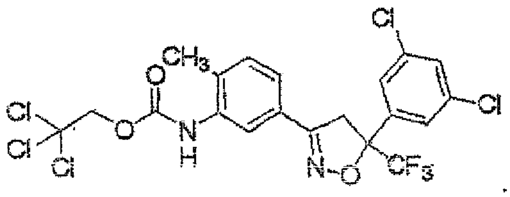

$^1$H-NMR ($CDCl_3$) δ : 8.04 (1H, br s), 7.51-7.49 (3H, m), 7.41 (1H, t, J = 1.9 Hz) , 7.26 (1H, d, J = 8.1 Hz), 6.72 (1H, br s), 4.85 (2H, s), 4.09 (1H, d, J = 17.3 Hz), 3.69 (1H, d, J = 17.3 Hz) , 2.34 (3H, s).

Production Example 68

In 1 ml of tetrahydrofuran were dissolved 195 mg of 5-(5-(3,5-dichlorophenyl)-4,5-dihydro-5-trifluoromethyl-3-isoxazolyl)-2-methylaniline obtained according to Reference Production Example 5 and 277 mg of (2-methoxyethyl) chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 142 mg of N-(5-(5-(3,5-dichlorophenyl)- 4,5 -dihydro-5 -trifluoromethyl-3-isoxazolyl) -2-1methylphenyl) -o- (2-methoxyethyl) carbamate (hereinafter referred to as the present compound (68)).

The present compound (68):

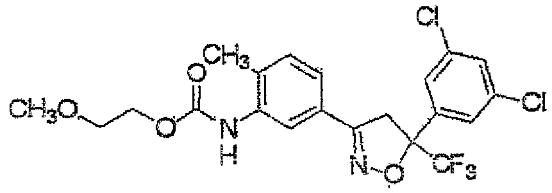

$^1H$ -NMR ($CDCl_3$) δ : 8.11 (1H, br s) , 7.51 (2H, d, J = 1.5 Hz), 7.48 (1H, dd, J = 8.0, 1.8 Hz), 7.41 (1H, t, J = 1.8 Hz), 7.21 (1H, d, J = 8.0 Hz), 6.58 (1H, br s), 4.37-4.35 (2H, m), 4.10 (1H, d, J = 17.3 Hz), 3.70 (1H, d, J = 17.3 Hz), 3.68-3.66 (2H, m), 3.43 (3H, s), 2.28 (3H, s).

Production Example 69

In 1 ml of tetrahydrofuran were dissolved 195 mg of 5-(5-(3,5-dichlorophenyl)-4,5-dihydro-5-trifluoromethyl-3 - isoxa-zolyl) - 2 -methylaniline obtained according to Reference Production Example 5 and 341 mg of benzyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 44 mg of N-(5-(5-(3,5-dichlorophenyl)-4,5-dihydro-5-trifluoromethyl-3-isoxazolyl) -2-methylphenyl) -0-benzylcarbamate (hereinafter referred to as the present compound (69)).

The present compound (69):

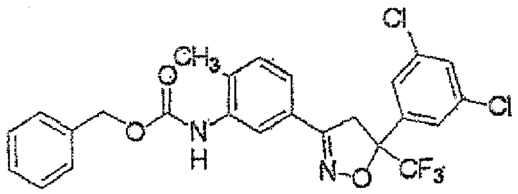

$^1H$-NMR ($CDCl_3$) δ: 7.50 (2H, d, J = 1.5 Hz), 7.41-7.29 (7H, m), 7.10 (1H, d, J = 7. 6 Hz), 7.01 (1H, d, J = 1.5 Hz), 6.83 (1H, dd, J = 7.6, 1.5 Hz), 4.39 (2H, d, J = 4.5 Hz), 4.02 (1H, d, J = 17.5 Hz), 3.62 (1H, d, J = 17.5 Hz), 2.18 (3H, s).

Production Example 70

In 1 ml of tetrahydrofuran were dissolved 195 mg of 5-(5-(3,5-dichlorophenyl)-4,5-dihydro-5-trifluoromethyl-3-isoxazolyl)-2-methylaniline obtained according to Reference Production Example 5 and 313 mg of phenyl chloroformate, and 101 mg of triethylamine was added thereto at room temperature and stirred for 2 hours. A saturated aqueous sodium hydrogencarbonate solution was added to the reaction mixture, followed by extraction with ethyl acetate. The organic layer was dried over anhydrous sodium sulfate and then concentrated under reduced pressure . The resultant, residue was subjected to silica gel column chromatography to obtain 69 mg of N-(5-(5-(3,5-dichlorophenyl)-4,5-dihydro-5-trifluoromethyl-3-isoxazolyl) -2-methylphenyl) -0-phenylcarbamate (hereinafter referred to as the present compound (70)).

The present compound (70):

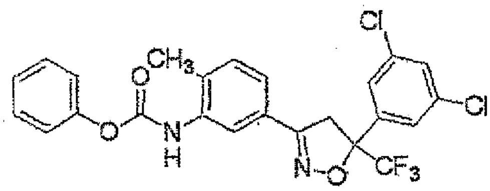

$^1$H-NMR ($CDCl_3$) δ: 8.15 (1H, br s), , 7.56 (1H, dd, J = 8.0, 1.5 Hz) , 7.48 (2H, d, J = 1.5 Hz), 7.45-7.39 (3H, m), 7.29-7.26 (2H, m), 7.22-7.19 (2H, m), 6.85 (1H, br *s),* 4.07 (1H, d, J = 17.3 Hz), 3.67 (1H, d, J = 17.3 Hz) , 2.38 (3H, s).

(Production Example 71

In 3 ml of tetrahydrofuran were dissolved 420 mg of 5- (5- (3,5-dichlorophenyl) -4,5-dihydro-5-trifluoromethyl-3-isoxazolyl)-2-ethylaniline obtained according to Reference Production Example 28 and 183 mg of 3,3,3-trifluoropropionyl chloride, and 126 mg of triethylamine was added thereto at room temperature and stirred four 1 hour. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 392 mg of N-(3-(5-(3,5-dichlorophenyl)- 4,5 -dihydro-5-trifluoromethyl- 3 - isoxazolyl) -6-ethylphenyl) -3,3,3-trifluoropropionylamide (hereinafter referred to as the present compound (71)).

The present compound (71):

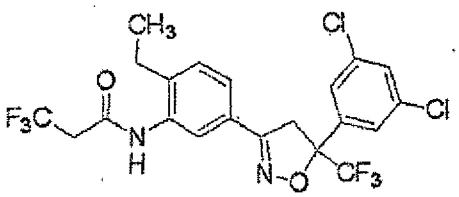

$^1$H-NMP, ($CDC1_3$) δ*:* 8.01 (1H, d, J = 1.8 Hz), 7.58 (1H, dd, J = 8.0, 1.8 Hz), 7.50 (2H, d, J = 1. 4 Hz), 7.42 (1H, t, J = 1.8 Hz), 7.38 (1H, br s), 7.29 (1H, d, J = 8.0 Hz), 4.08 (1H, d, J = 17.4 Hz), 3.68 (1H, d, J = 17.4 Hz), 3.31 (2H, q, J = 10.5 Hz), 2.62 (2H, q, J = 7.5 Hz), 1.24 (3H, t, J = 7.5 Hz).

Production Example 72

In 3 ml of N,N-dimethylformamide were dissolved 80 mg of N-(3,3,3-trifluoropropionyl)-2-mehyl-3-hydroxyiminomethylaniline obtained according to Reference Production Example 9 and 43 mg of N-chlorosuccinimide, and the resulting solution was stirred at 50°C for 30 minutes. The reaction solution was cooled to room temperature and 78 mg of 2-(3,5. dichlorophenyl)--3,3,3-trifluoro-l-propene and then 33 mg of triethylamine were added thereto and stirred for 15 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 80 mg of N-(3-(5-(3,5-dichlorophenyl) -4, 5-dihydro-5-trifluoromethyl-3-isoxazolyl)-2-methylphenyl)-3,3,3-trifluoropropionylamide (hereinafter referred to as the present compound (72)).

The present compound (72):

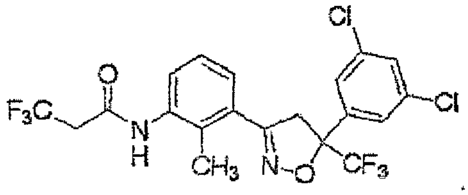

$^1$H-NMR ($CDC1_3$) δ: 7.71 (1H, d, J = 8.0 Hz), 7.50-7.50 (2H, m), 7.44-7.44 (1H, m), 7.38 (1H, br s), 7.29-7.27 (1H, m), 7.18 (1H, d, J = 7.5 Hz), 4.07 (1H, d, J = 17.4 Hz), 3.71 (1H, d, J = 17.6 Hz), 3.31 (2H, q, J = 10.5 Hz), 2.38 (3H, s).

Production Example 73

In 6 ml of N,N-dimethylformamide were dissolved 146 mg of N-(3,3,3-trifluoropropionyl)-2-chloro-3-hydroxyiminomethylaniline obtained according to Reference Production Example 12 and 98 mg of N-chlorosuccinimide, and the resulting solution was stirred at 50ºC for 30 minutes. The reaction solution was cooled to room temperature and 240 mg of 2-(3,5-dichlorophenyl)-3, 3, 3-trifluoro-1-propene and then 90 mg of triethylamine were added thereto and stirred for 15 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 245 mg of N-(3-(5-(3,5-dichlorophenyl) -4, 5-dihydro-5-trifluoromethyl-3-isoxazolyl)-2-chlorophenyl)-3,3,3-trifluoropropionylamide (hereinafter referred to as the present compound (73)).

The present compound (73):

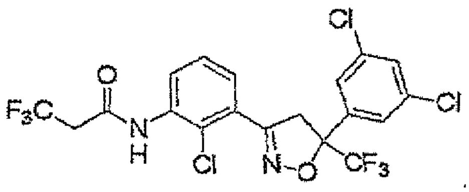

$^1$H-NMR ($CDC1_3$) δ : 8.44 (1R, dd, J = 7.6, 2.1 Hz), 7.94 (1H, s), 7.49-7.49 (2H, m), 7.45-7.45 (1H, m), 7.39-7.35 (2H, m), 4.19 (1H, d, J = 17.6 Hz), 3.78 (1H, d, J = 17.6 Hz), 3.34 (2H, q, J = 10.4 Hz).

Production Example 74

In 2.5 ml of N,N-dimethylformamide were dissolved 300 mg of N-(3,3,3-trifluoropropionyl)-5-methyl-3-hydroxyiminomethylaniline obtained according to Reference Production Example 17 and 154 mg of N-chlorosuccinimide, and the resulting solution was stirred at 50°C for 30 minutes. The reaction solution was cooled to room temperature and 277 mg of 2-(3,5-dichlorophenyl)-3,3,3-trifluoro-1-propene and then 116 mg of triethylamine were added thereto and stirred for 20 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 151 mg of N-(3-(5-(3,5-dichlorophenyl)-4,5-dihydro-5-trifluoromethyl-3-isoxazolyl)- 5-methylphenyl)- 3,3,3 - trifluoropropionylamide (hereinafter referred to as the present compound (74)).

The present compound (74):

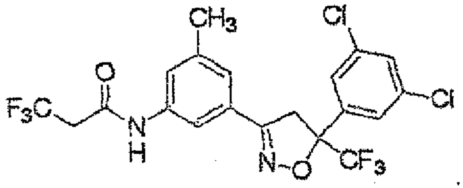

$^1H$-NMR ($CDC1_3$) δ : 7.71-7.70 (1H, m), 7.44-7.39 (6H, m) , 4.23-4.08 (1H, m), 3.83-3.69 (1H, m), 3.27-3.22 (2H, m), 2.39 (3H, s).

Production Example 75

To 1 ml of dimethylformamide were added 55 mg of N-(3,3,3-trifluoropropionyl)-2-methyl-5-hydroxyiminomethylaniline obtained according to Reference Production Example 21 and 30 mg of N-chlorosuccinimide, and the resulting mixture was stirred at 50ºC for 1 hour. The reaction solution was cooled to room temperature and 54 mg of 2-(3,5-dichlorophenyl)-3,3,3-trifluoro-1-propene and then 23 mg of triethylamine were added thereto and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 75 mg of N-(5-(5-(3,5-dichlorophenyl)-4,5-dihydro-5-trifluoromethyl-3-isoxazolyl)-2-methylphenyl)-3,3,3-trifluoropropionylamide (hereinafter referred to as the present compound (75)).

The present compound (75):

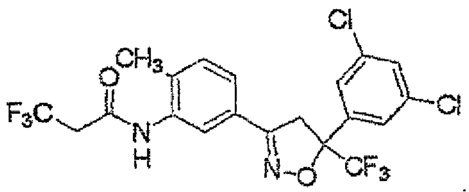

$^1H$-NMR ($CDC1_3$) δ: 8.05 (1H. s), 7.57-7.55 (1H, m), 7.50 (2H, s), 7.42-7.41 (1H, m), 7.29 (2H, s), 4.08 (1H, d, J = 17.1 Hz), 3.69 (1H, d, J = 17.1 Hz) , 3.31 (2H, q, J = 10.5 Hz), 2.31 (3H, s).

Production Example 76

In 2 ml of tetrahydrofuran were dissolved 250 mg of 5-(5-(3,5-dichlorophenyl)-4,5-dihydro-5-trifluoramethyl-3-isoxazolyl)-2-fluoroaniline obtained according to Reference Production Example 24 and 93 mg of 3,3,3-trifluoropropionyl chloride, and 64 mg of triethylamine was added thereto at room temperature and stirred for 6 hours. Water was added to the reaction mixture, followed by extraction with ethyl acetate,. The organic layer was dried over anhydrous sodium sulfate and then concentrated under reduced pressure. The resultant residue was subjected to silica gel column chromatography to obtain 265 mg of N-(3-(5-(3,5-dichlorophenyl) -4, 5-dihydro-5-trifluoromethyl-3 - isoxazolyl) -6-fluorophenyl) -3,3,3-trifluoropyronylamide (hereinafter referred to as the present compound (76)).

The present compound (76):

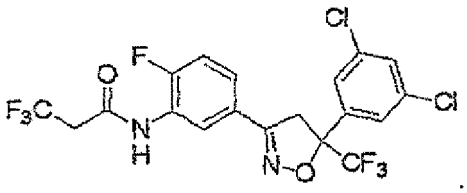

$^1$H-NMR ($CDC1_3$) δ: 8.53 (1H, dd, J = 7.4, 2.1 Hz), 7.66-7.64 (2H, m), 7.51-7.50 (2H, m), 7.42 (1S, t, J = 1.7 Hz), 7.20 (1H, dd, J = 10.4, 8.7 Hz), 4.09 (1H, d, J = 17.1 Hz), 3.70 (1H, d, J = 17.4 Hz), 3.32 (2H, q, J = 10. 2 Hz).

production Example 77

In 2 ml of tetrahydrofuran were dissolved 260 mg of 5-(5-(3,5-dichlorophenyl)-4, 5-dihydro-5-trifluoromethyl-3-isoxazolyl)-2-chloroaniline obtained according to Reference Production Example 26 and 93 mg of 3,3,3-trifluoropropionyl chloride, and 64 mg of triethylamine was added thereto at room temperature and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 79 mg of N-(3-(5-(3,5-dichlorophenyl) -4,5-dihydro-5-trifluoromethyl -3-isoxazolyl)-6-chlorophenyl)-3,3,3-trifluoropyronylamide (hereinafter referred to as the present compound (77)).

The present compound (77):

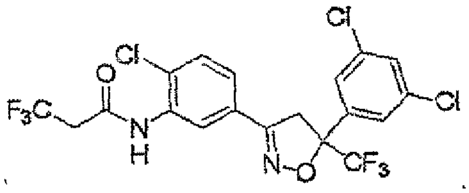

$^1$H-NMR ($CDCl_3$) δ*:* 8.55 (1H, d, J = 1.8 Hz), 7.93 (1H, S), 7.63 (1H, dd, J = 8.5, 1.8 HZ), 7.51-7.42 (4H, m), 4.09 (1H, d, J 17.3 Hz), 3.70 (1H, d, J = 17.3 Hz), 3.35 (2H, q, J = 10.1 Hz).

Production Example 78

In 13 ml of N,N-dimethylformamide was dissolved 390 mg of N-(5-hydroxyliminomethyl-2-methoxyphenyl)-3,3,3-trifluoropropionylamide obtained according to Reference Production Example 31, and 392 mg of N-chlorosuccinimide was added thereto at room temperature and stirred at 60°C for 1 hour. The reaction solution was cooled to room temperature and 342 mg of 2- (3,5-dichlorophenyl) -3,3,3-trifluoro-1-propene and then 144 mg of triethylamine were added thereto and stirred for 48 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 633 mg of N-(5-(5-(3, 5-dichlorophenyl)-4, 5-dihydro-5-trifluoromethyl-3-isoxazolyl)-2-methoxyphenyl)-3,3,3-trifluoropropionylamide (hereinafter referred to as the present compound (78)).

The present compound (78):

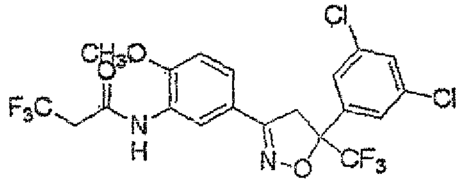

$^1$H-NME. ($CDC1_3$) δ: 8.55 (1H, d, J = 2.2 Hz), , 8.01 (1H, br s), 7.66-7.64 (1H, m), 7.51 (2H, d, J = 1.7 Hz), 7.41 (1H t. J = 1.9 Hz), 6.96 (1H, d, J = 8.7 Hz), 4.10 (1H, d, J = 17.3 Hz), 3.96 (3H, s), 3.70 (1H, d, J = 17.3 Hz), 3.29 (2H, q, J = 10.3 Hz).

Production Example 79

In 5 ml of toluene were dissolved 250 mg of 5-(5-(3,5-dichlorophenyl)-4,5-dihydro-5-trifluoromethyl-3-isoxazolyl)-2-chloroaniline obtained according to Reference Production Example 26 and 198 mg of triphosgene, and the resulting solution was stirred under reflux for 2 hours. The reaction solution was allowed to cool to room temperature, followed by adding thereto 4 ml of methanol, and the resulting mixture was stirred at the same temperature for 5 hours. A saturated aqueous sodium hydrogencarbonate solution was added to the reaction mixture, followed by extraction with ethyl acetate. The organic layer was dried over anhydrous sodium sulfate and then concentrated under reduced pressure. The resultant residue was subjected to silica gel column chromatography to obtain 218 mg of N-(5-(S-(3,5-dichlorophenyl)-4, 5-dihydro-5-trifluoromethyl-3-isoxazolyl)-2-chlorophenyl)-O-methylcarbamate.

The present compound (79):

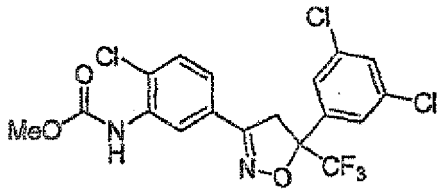

$^1$-H-NMR. ($CDCl_3$) δ: 8.38(1H, d, J= 2.0 Hz), 7.52-7.50 (3H, m), 7.42-7.40 (2H, m), 7.23 (1H, br s), 4.10 (1H, d, J = 17.3 Hz), 3.84 (3H, s), 3.70 (1H, d, J = 17.3 Hz).

Next, reference production examples are described below with regard to the production of intermediates for production.

Reference Production Example 1

In 15 ml of N,N-dimethylformamide was dissolved 249 mg of 3-nitrobenzaldoxime, and 201 mg of N-chlorosuccinimide was added thereto at room temperature and stirred at 60°C for 1 hour. The reaction solution was cooled to room temperature and 362 mg of 2-(3,5-dichlorophenyl)-3,3,3-trifluoro-l-propene and then 152 mg of triethylamine were added thereto and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 232 mg of 3-(5-(3,5-dichlorophenyl)-4,5-dihydro-5-trifluoromethyl-3-isoxazolyl)nitrobenzene.

3-(5-(3,5-Dichlorophenyl)-4,5-dihydro-5-trifluoromethyl-3-isoxazolyl)nitrobenzene:

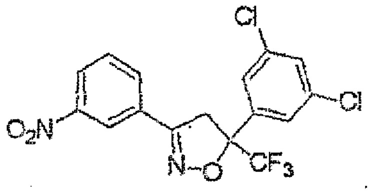

$^1$H-NMR ($CDC1_3$) δ: 8.43 (1H, br s), 8.34-8.32 (1H, m), 8.10 (1H, d, J = 8.0 Hz), 7.67-7.65 (1H, m), 7.52 (2H, s), 7.45-7.45 (1H, m), 4.14 (1H, d, J = 17.3 Hz), 3.76 (1H, d, J = 17.3 Hz) .

Reference Production Example 2

To 1 ml of a 2.5% aqueous acetic acid solution was added 192 mg of iron powder (10-to 20-mesh), and a suspension of 232 mg of the 3-(5-(3,5-dichlorophenyl)-4,5-dihydro-5-trifluoromethyl-3-isoxazolyl)nitrobenzene produced in Reference Production Example 1 in 1.5 ml of ethanol was added thereto at 75°C and stirred at the same temperature for 15 minutes. Then, 300 mg of iron powder was further added thereto and stirred for 1 hour. The reaction mixture was cooled to room temperature and filtered, and the precipitate was washed with ethyl acetate. Water and ethyl acetate was added to the filtrate to effect extraction. The organic layer was dried over anhydrous sodium sulfate and then concentrated under reduced pressure. The resultant residue was subjected to silica gel column chromatography to obtain 198 mg of 3-(5-(3,5-dichlorophenyl)-4,5-dihydro-5-trifluoromethyl-3-isoxazolyl) aniline.

3-(S-(3,5-Dichlorophenyl)-4,5-dihydro-5-trifluoromethyl-3-isoxazolyl)aniline:

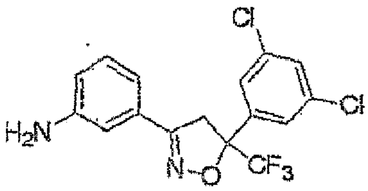

$^1$H-NMR ($CDCl_3$) δ: 7.51 (2H, br s), 7.42 (1H, t, J = 1.9 Hz), 7.20 (1H, t, J = 7.8 Hz), 7.04 (1H, t, J = 1.9 Hz), 6.95-6.93 (1H, m), 6.77-6.75 (1H, m), 4.05 (1H, d, J = 17.1 Hz), 3.77 (2H, br s), 3.66 (1H, d, J = 17.1 Hz).

Reference Production Example 3

2.24 Grams of sodium acetate was added to a mixture of 3.00 g of 4-methyl-3-nitrobenzaldehyde, 1.64 g of hydroxylamine hydrochloride, 30 ml of ethanol and 15 ml of water at room temperature and stirred at room temperature for 1. hour. The reaction mixture was added to water and extracted with ethyl acetate, and the organic layer was dried over anhydrous sodium sulfate and then concentrated under reduced pressure to obtain 2.77 g of 5-hydroxyiminomethyl-2-methylnitrobenzene.

5 -Hydroxyiminomethyl-2-methylnitrobenzene:

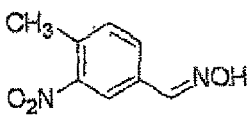

$^1$H-NMR (DMSO-$d_6$) δ: 11.53 (1H, s), 8.24 (1H, s), 8.17 (1H, d, J = 1.7 Hz), 7.84 (1H, dd, J = 8.0, 1.7 Ez), 7.54 (1H, d, J = 8.0 Hz), 2.52 (3H, s).

Reference Production Example 4

In 30 ml of N,N-dimethylformamide were dissolved 2.77 g of the 5-hydroxyiminomethyl-2-methylnitrobenzene obtained in Reference Production Example 3 and 2.06 g of N-chlorosuccinimide, and the resulting solution was stirred at 50°C for hours. The reaction solution was cooled to room temperature and 3.71 g of 2-(3,5-dichlorophenyl)-3,3,3-trifluoro-1-propene and then 1.56 g of triethylamine were added thereto and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 4.99 g of 5-(5-(3,5-dichlorophenyl)-4,5-dihydro-5-triflucromethyl-3 -isoxazolyl)-2-methylnitrobenzene.

5-(5-(3,5-Dichlorophenyl)-4,5-dihydro-5-trifluoromethyl-3-isaxazolyl)-2-methylnitrobenzene:

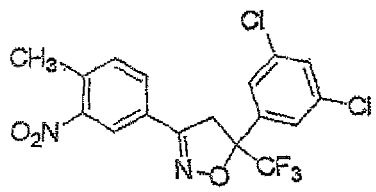

$^1$H-NMR CCDCl$_3$) δ: 8.16 (1H, d, J= 1.9 Hz), 7.90 (1H, dd, J = 8.1, 1.9 Hz), 7.51 (2H, d, J= 1.2 Hz) , 7.45-7.43 (2H, m), 4.11 (1H. d, J = 17.1 Hz), 3.73 (1H, d, J = 17.1 Hz), 2.66 (3H, s).

Reference Production Example 5

To 19 ml of a 2.5% aqueous acetic acid solution was added 1.89 g of iron powder (10-to 20-mesh), and a suspension of 4.62 of the 5-(5-(3,5-dichlorophenyl)-4,5-dihydro-5-trifluoromethyl-3-isoxazolyl)-2-methylnitrobenzene produced in Reference Production Example 4 in 29 ml of ethanol was added thereto at 75°C and stirred for 15 minutes. Then, 1.80 g of iron powder was further added thereto and stirred for 1 hour. The reaction mixture was cooled to room temperature and filtered, and the precipitate was washed with ethyl acetate. A saturated aqueous sodium hydrogencarbonate solution and ethyl acetate was added to the filtrate to effect extraction. The organic layer was dried over anhydrous sodium sulfate and then concentrated under reduced pressure. The resultant residue was subjected to silica gel column chromatography to obtain 3.18 g of 5-(5-(3,5-dichlorophenyl)-4,5-dihydro-5-trifluoromethyl-3-isoxazolyl)-2-methylaniline.

5-(5-(3,5-Dichlorophenyl)-4,5-dihydro-5-trifluoroiaethyl-3-isoxazolyl) -2-methylaniline:

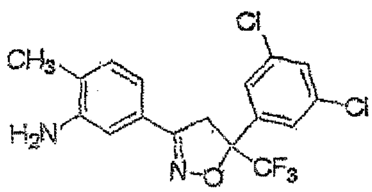

$^1$-H-NMR (CDCl$_3$) δ: 7.51 (2H, d, J = 1.4 Hz), 7.41 (1H, t, J = 1.8 Hz), 7.08 (1H, d, J = 7.8 Hz), 7.05 (1H, d, J = 1.8 Hz), 6.88 (1H, dd, J = 7.8, 1.7 HZ), 4.04 (1H, d, J = 17.1 Hz), 3.71 (2H, br s), 3.65 (1H, d, J = 17.1 Hz), 2.19 (3H, s).

Reference Production Example 6

In 12 ml of tetrahydrofuran was dissolved 1.00 g of methyl 3-amino-2-methylbenzoate, and 1.09 g of 3,3,3-trifluoropropionyl chloride was added thereto under ice-cooling and stirred at room temperature for 1 hours. The reaction mixture was added to water and extracted with ethyl acetate. The organic layer was dried over anhydrous sodium sulfate and then concentrated under reduced pressure. The resultant residue was recrystallized from hexane-ethyl acetate to obtain 1.23 g of methyl 3-(3,3,3-trifluoropropionylamino)-2-methylbenzoate.

Methyl 3-(3,3,3-trifluoropropionylamino)-2-methylbenzoate:

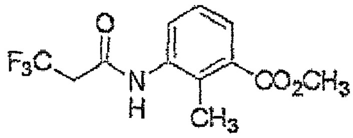

$^1$H-NMR (CDC$1_3$) δ: 7.79 (1H, d, J = 8.0 Hz), 7.72 (1H, d, J = 7.7 Hz), 7.33-7.25 (2H, m), 3.90 (3H, s), 3.30 (2H, q, J = 10.5 Hz), 2.45 (3H, s).

Reference Production Example 7

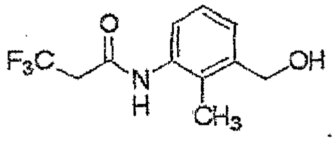

In 10 ml of tetrahydrofuran was dissolved 500 mg of the methyl 3-(3,3,3-trifluoropropionylamino)-2-methylbenzoate obtained in Reference Production Example 6, and 155 mg of lithium aluminum hydride was added thereto under ice-cooling and stirred under ice-cooling for 20 minutes. The reaction mixture was added to water and extracted with ethyl acetate. The organic layer was dried over anhydrous sodium sulfate and then concentrated under reduced pressure. The resultant residue was subjected to silica gel column chromatography to obtain 2-methyl-3-(3,3,3-trifluoropropionyl) aminobenzyl alcohol.

Reference Production Example 8

The 2-methyl-3-(3,3,3-trifluoropropionyl)aminobenzyl alcohol obtained in Reference Production Example 7 was dissolved in 10 ml of chloroform, and 5 g of manganese dioxide was added thereto at room temperature and stirred for 2 hours. Then, the resulting mixture was allowed to stand for 12 hours. The reaction mixture was filtered through Celite and the filtrate was concentrated under reduced pressure. The resultant residue was subjected to silica gel column chromatography to obtain 72 mg of 2-methyl-3- (3,3,3-trifluoropropionyl) aminobenzaldehyde.

2-Methyl-3- (3,3,3-trifluoropropionyl) aminobenzaldehyde:

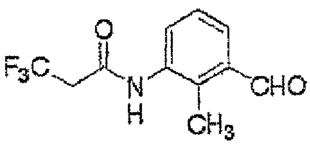

$^1$H-NMBL ($CDCl_3$) δ: 10.26 (1H, s), 7.87 (1H, d, J = 7.7 Hz), 7.72 (1H, d, J = 7.7 Hz), 7.43-7.41 (1H, m), 7.34 (1H, s), 3.33 (2H, q , J = 10.5 Hz), 2.57 (3H, s).

Reference Production Example 9

72 Milligrams of the 2-methyl-3-(3,3,3-trifluoropropionyl) aminobenzaldehyde obtained in Reference production Example 8, 27 mg of hydroxylamine hydrochloride, 36 mg of sodium acetate, 5 ml of ethanol and 2.5 ml of water were mixed, and the resulting mixture was stirred at room temperature for 20 minutes. The reaction mixture was added to a saturated aqueous sodium chloride solution and extracted with ethyl acetate, and the organic layer was dried over anhydrous sodium sulfate and then concentrated under reduced pressure to obtain 80 mg of N-(3,3,3-trifluoropropionyl)-2-methyl-3-hydroxyiminomethylaniline.

N-(3,3,3-trifluoropropionyl) -2-methyl-3-hydroxyiminomethylaniline:

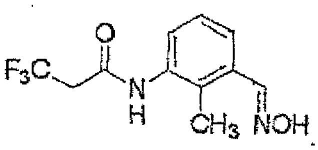

$^1$H-NMR ($CDC1_3$) δ: 10.32 (1H, s), 8.93 (1H, sr), 8.42 (1H, s), 7.61 (1H, d, J = 8.0 Hz), 7.44 (1H, d, J = 7.8 HZ), 7.20-7.18 (1H, M) , 3.32 (2H, q, J = 10.6 Hz), 2.27-2.24 (3H, br m).

Reference Production Example 10

In 20 ml of chloroform were dissolved 813 mg of 3-amino-2-chlorobenzyl alcohol and 8 g of manganese dioxide, and the resulting solution was stirred at room temperature for 14 hours. The reaction mixture was filtered through Celite and the filtrate was concentrated under reduced pressure. The resultant residue was subjected to silica gel column chromatography to obtain 284 mg of 3-amino-2-chlorobenzaldehyde.

3 -Amino-2-chlorobenzaldehyde;'

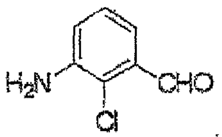

$^1$H-NMR ($CDC1_3$) (δ: 10.45 (1H, d, J = 0.7 Hz) , 7.32 (1H, dd, J = 7.6, 1.6 Hz), 7.20-7-18 (1H, m), 7.00 (1H, dd, J = 8.0, 1.4 Hz), 4.24 (2H, br s).

Reference Production Example 11

In 4 ml of tetrahydrofuran was dissolved 134 mg of the 3-amino-2-chlorobenzaldehyde obtained in Reference Production Example 10, and 126 mg of trifluoropropionyl chloride and then 87 mg of triethylamine were added thereto at room temperature and stirred for 1 hour. The reaction mixture was concentrated under reduced pressure and the resultant residue was subjected to silica gel chromatography to obtain 106 mg of 2-chloro-3-(3,3,3-trifluoropropionyl) amino-benzaldehyde.

2-Chloro-3-(3,3,3-trifluoropropionyl) aminobenzaldehyde:

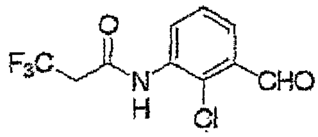

$^1$H-NMR (CDC$1_3$) δ: 10.46 (1H, s), 8.58 (1H, d, J = 7.3 Hz), 7.98 (1H, s), 7.75 (1H, dd, J = 7.7, 1.6 Hz), 7.45 (1H, t, J = 8.0 Hz), 3.37 (2H, q, J 10.4 Hz).

Reference Production Example 12

176 Milligrams of the 2-chloro-3-(3,3,3-trifluoropropiorryl) aminobenzaldehyde obtained in Reference production Example 11, 60 mg of hydroxylamine hydrochloride, 81 mg of sodium acetate, 7 ml of ethanol and 3.5 ml of water were mixed, and the resulting mixture was stirred at room temperature for 5 hours. The reaction mixture was added to water and extracted with ethyl acetate, and the organic layer was dried over anhydrous sodium sulfate and then concentrated under reduced pressure to obtain 143 mg of N-(3,3,3-trifluoropropionyl)-2-chloro-3-hydroxyiminomethylaniline.

N-(3;3;3-trifluoropropionyl)-2-chloro-3-hydroxyiminomethylaniline:

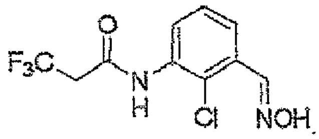

$^1$H-NMR (CDC$1_3$) δ: 8.55-8.54 (1H, m), 8.36 (1H, d, J = 8.7 Hz), 7.91 (1H, s), 7.72-7.65 (1H, m), 7.51 (1H, s), 7.38-7.30 (1H, m), 3.33 (2H, q, J = 10.4 Hz).

Reference Production Example 13

To 30 ml of toluene were added 2.57 g of 5-nitroisophthalaldehyde, 0.137 g of p-toluenesulfonic acid monohydrate and 1.49 g of 2,2-dimethyl-1,3-propanediol, and the resulting- mixture was heated under reflux for 2 hours while being dehydrated with a Dean-Stark trap. The reaction mixture was cooled to room temperature, added to a saturated aqueous sodium hydrogencarbonate solution and then extracted with ethyl acetate. The organic layer obtained was dried over anhydrous sodium sulfate and then concentrated under reduced pressure. The resultant residue was subjected to silica gel column chromatography to obtain 1.70 g of 2-(3-nitro-5-formylphenyl).5,5-dimethyl-1,3-l dioxane.

2-(3-Nitro-5-formylphenyl)-5,5-dimethyl-l,3-dioxane:

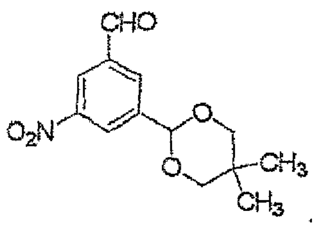

$^1$ H-NMR ($CDCl_3$) δ: 10.12 (11, s), 8.69-8-69 (1H, m), 8.63-8.62 (1H, m), 8.37-8.36 (1H, m), 5.53 (1H s), 3.93 (2H, d, J = 11.2 Hz), 3.71 (2H, d, J = 10. 5 Hz), 1.28 (3H, s), 0.84 (3H, s).

Reference Production Example 14

In 12 ml of diethylene glycol were dissolved 1.70 g of the 2-(3-nitro-5-formylphenyl)-5,5-dimethyl-1,3-dioxane obtained in Reference Production Example 13 and 1.93 g of hydrazine monohydrate, and the resulting solution was heated under reflux for 1 hour. A solution of 1.43 g of potassium hydroxide in 1.93 g of water was added thereto and the resulting mixture was heated under reflux for 1 hour. The reaction mixture was cooled to room temperature, added to water and then extracted with ethyl acetate. The organic layer was dried over anhydrous sodium sulfate and then concentrated under reduced pressure. The resultant residue was subjected to silica gel column chromatography to obtain 0.6 g of 2-(3-amino-5-methylphenyl)-5,5-dimethyl-1,3-dioxane.

2-(3-Amino-5-methylpheny)-5,5-dimethyl-l,3-dioxane:

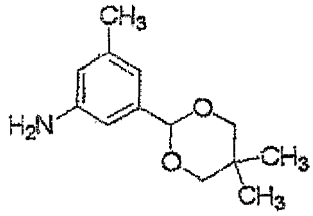

$^1$H-NMBL ($CDC1_3$) δ: 6.72 (1H, s), 6.66 (1H, s), 6.49 (1H, s), 5.27 (1H, s), 3.76 (2H, d, J = 11.2 Hz), 3.62 (4H, d, J = 10.5 Hz), 2.26 (3H, s), 1.29 (3H, s), 0.79 (3H, s).

Reference Production Example 15

In 4 ml of tetrahydrofuran was dissolved 400 mg of the 2- (3-amino-5-methylphenyl) -5,5-dimethyl-1,3-dioxane obtained in Reference Production Example 14, and 300 mg of 3,3,3-trifluoropropionyl chloride and then 183 mg of triethylamine were added thereto at room temperature and stirred for 15 hours. The reaction mixture was concentrated under reduced pressure and the resultant residue was subjected to silica gel column chromatography to obtain 0.40 g of 2-(3-trifluoropropionylairino-5-methylpherryl) -5, 5-dimethyl 1,3-dioxane.

2-(3-Trifluoropropionylamino-5-methylphenyl)-5,5-dimethyl-1,3-dioxane:

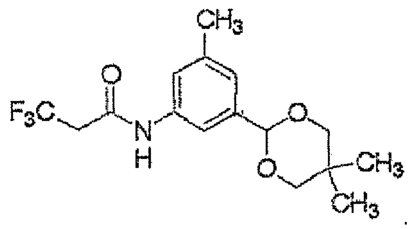

$^1$H-NMR ($CDC1_3$) δ: 7.43 (2H, br s), 7.35 (1H, s), 7.13 (1H, s), 5.34 (1H, s), 3.76 (2H, d, J = 11.1 Hz) , 3.64 (2H, d, J = 10.6 Hz), 3.18 (2H, q, J = 10.5 Hz), 2.34 (3H, s), 1.29 (3H, s), 0.80 (3H, s).

Reference Production Example 16

In a mixture of 8 ml of acetone and 15 ml of water were dissolved 0.40 g of the 2-(3- trifluoropropionylamino-5-methylphenyl)-5,5-dimethyl-1,3-dioxane obtained in Reference Production Example 15 and 46 mg of p-toluenesulfonic acid monohydrate, and the resulting solution was stirred at 60ºC for 11 hours. The reaction mixture was cooled to room temperature, added to a saturated aqueous sodium hydrogencarbonate solution and then extracted with ethyl acetate.

The organic layer was dried over anhydrous sodium sulfate and then concentrated under reduced pressure. The resultant residue was subjected to silica gel column chromatography to obtain 260 mg of 3-methyl-5- (3,3,3 -trifluoropropionyl) aminobenzaldehyde.

3-Methyl-5-(3,3,3-trifluoropropionyl)aminobenzaldehyde:

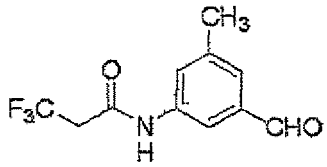

$^1$H-NMR ($CDCl_3$) δ: 9.95 (1H, s), 7.78-7.74 (3H, m), 7.49 (1H, s), 3.28 (2H, q, J = 10.4 Hz), 2.43 (3H, s).

Reference Production Example 17

In a mixture of 3 ml of ethanol and 1.5 ml of water was dissolved 380 mg of the 3-methyl-5-(3,3,3-trifluoropropionyl)aminobenzaldehyde obtained in Reference Production Example 16, and 140 mg of' hydroxylamine hydrochloride and 191 mg of sodium acetate were added thereto at room temperature and stirred for 1 hour. The reaction mixture was added to water and extracted with ethyl acetate, and the organic layer was dried over anhydrous sodium sulfate and then concentrated under reduced pressure to obtain 366 mg of N-(3,3,3-trifluoropropionyl)-5-methyl-3-hydroxyiminomethylaniline.

N-(3;3,3-trifluoropropionyl)-5-methyl-3-hydroxyiminomethylaniline:

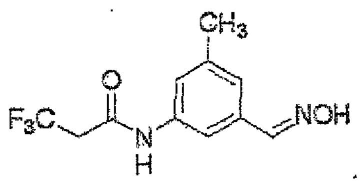

$^1$H-NMR (DMSO-$d_6$) δ: 11.22 (1H, s), 10.29 (1H, s), 8.05 (1H, s), 7.64 (1H, s), 7.38 (1H, s), 7.11 (1H, s), 3.4.8 (2H, q, J = 11.2 Hz), 2.28 (3H, s).

Reference Production Example 18

In 25 ml of tetrahydrofuran was dissolved 825 mg of methyl 3-amino-4-methylbenzoate, followed by adding thereto 735 mg of 3,3,3-trifluoropropionic acid chloride and then 505 mg of triethylamine, and the resulting mixture was heated under reflux for 9 hours. The reaction mixture was cooled to room temperature, added to water and then extracted with ethyl acetate. The organic layer was dried over anhydrous sodium sulfate and then concentrated under reduced pressure to obtain 1.3 g of methyl 3-(3,3,3-trifluoropropionylamino)-4-methylbenzoate.

Methyl 3-(3,3,3-trifluoropropionylamino)-4-methylbenzoate:

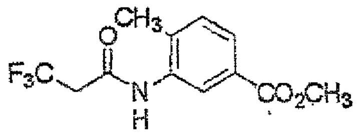

$^1$H-NMR ($CDCl_3$) δ : 8.30 (1H, s), 7.83 (1H, d, J = 8.0 Hz), 7.30 (2H, d, J = 8.0 Hz), 3.90 (3H, s), 3.31 (2H, q, J = 10.6 Hz), 2.31 (3 H, s).

Reference Production Example 19

In 20 ml of tetrahydrofuran was dissolved 1.3 g of the methyl 3-(3,3,3-trifluoropropionylamino)-4-methylbenzoate obtained in Reference Production Example 18, and 380 mg of lithium aluminum hydride was added thereto under ice-cooling and stirred for 1 hour. To the reaction mixture were added 380 mug of water, 1.2 g of a 15% aqueous sodium hydroxide solution and 380 mg of water in that order, and the resulting mixture was filtered. The filtrate was dried over anhydrous sodium sulfate and then concentrated under reduced pressure to obtain 3-(3,3,3-trifluoropropinloylamino)-4-methylbenzyl alcohol.

3-(3,3,3-Trifluoropropionylamino)-4-methylbenzyl alcohol:

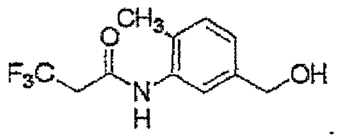

Reference production Example 20

The 3-(3,3,3-trifluoropropionylamino)-4-methylbenzyl alcohol obtained in Reference Production Example 19 was dissolved in 100 ml of chloroform, followed by adding thereto 12.3 g of manganese dioxide, and the resulting mixture was stirred at room temperature for 4 hours and allowed to stand for 2.5 days. The reaction mixture was filtered and the filtrate was concentrated under reduced pressure. The resultant residue was subjected to silica gel column chromatography to obtain 55 mg of 3-(3,3,3-trifluoropropionylamino)-4-methylbenzaldehyde.

3-(3,3,3-Trifluoropropionylamino)-4-methylbenzaldehyde:

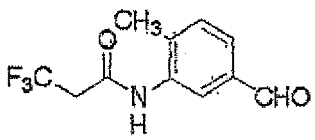

$^1$H-NMR ($CDC1_3$) δ: 9.97 (1H, s), 8.29 (1H, d, J=1.2 Hz), 7.68 (1H, dd, J = 7.8, 1.2 Hz), 7.39 (1H, d, J = 7.8 Hz), 7.28 (1H, br s), 3.33 (2H, q, J = 10.5 Hz), 2.36 (3H, s) .

Reference Production Example 21

In a mixture of 1 ml of ethanol and 0.5 ml of water were dissolved 55 mg of the 3-(3,3,3-trifluoropropionylamino) -4-methylbenzaldehyde obtained in Reference Production Example 20, 20 mg of hydroxylamine hydrochloride and 41

mg of sodium acetate, and the resulting solution was stirred at room temperature for 6 hours. The reaction mixture was added to water and extracted with ethyl acetate, and the organic layer was dried over anhydrous sodium sulfate and then concentrated under reduced pressure to obtain 55 mg of N-(3,3,3-trifluoropropionyl)-2-methyl-5-hydroxyiminomethylaniline.

N-(3,3,3-trifluoropropionyl)-2-methyl-5-hyd-roxyiminomethylaniline:

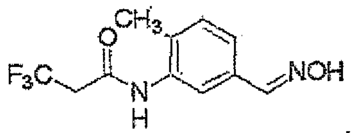

$^1$H-NMR (CDC$1_3$) δ: 9.37 (1H, s), 8.14 (1H, s), 8.08 (1H, s), 7.87 (1H, s), 7.36 (1H, d, J = 7.7 Hz), 7.21 (1H, d, J = 7.5 Hz), 3.31 (2H, q, J = 10.6 Hz), 2.27 (3H, s).

Reference Production Example 22

In a mixture of 20 ml of ethanol and 10 ml of water were dissolved 2 g of 4-fluoro-3-nitrobenzaldehyde, 1.07 g of hydroxylamine hydrochloride and 1.45 g of sodium acetate, and the resulting solution was stirred at room temperature for 6 hours. The reaction mixture was added to water and extracted with ethyl acetate, and the organic layer was dried over anhydrous sodium sulfate and then concentrated under reduced pressure to obtain 1.95 g of 2-fluoro-5-hydroxyiminomethylnitrobenzene.

2-Fluoro-5-hydraxyiminomethylnitrobenzene:

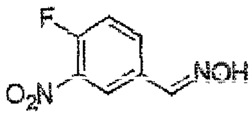

$^1$H-NMR (DMSO-$D_6$) δ: 11.63 (1H, s), 8.34 (1H, dd, J = 7.2, 2.2 Hz), 8.28 (1H, s), 8.04-8.03 (1H, m), 7.65-7.62 (1H, m).

Reference Production Example 23

In 20 ml of N,N-dimethylformamide were dissolved 1.95 g of the 2-fluoro-5-hydroxyiminomethylnitrobenzene obtained in Reference Production Example 22 and 1.42 g of N-chlorosuccinimide, and the resulting solution was stirred at 60°C for 1 hours. The solution was cooled to room temperature and 2.55 g of 2-(3,5-dichlorophenyl)-3,3,3-trifluoro-1-propene and then 1.07 g of triethylamine were added thereto and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 1.40 g of 5-(5-(3,5-dichlorophenyl)-4,5-dihydro-5-trifluoromethyl-3-isoxazolyl)-2-fluoronitrobenzene.

5-(5-(3,5-Dichlorophenyl)-4,5-dihydro-5-trifluoromethyl-3 - isoxazolyl) -2- fluoronitrobenzene:

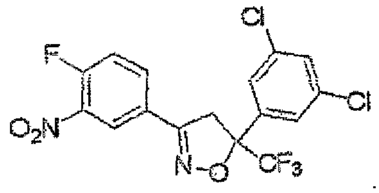

$^1$H-NMR ($CDCl_3$) δ- 8.25 (1H, dd, J = 6.9, 2.3 Hz), 8.08-8.05 (1H, m), 7.51 (2H, d, J = 1.2 Hz), 7.45 (1H, t, J = 1.9 Hz), 7.41 (1H, dd, J= 10.1, 8.7 Hz), 4.11 (1H, d, J = 17.4 Hz), 3.73 (1H, d, J = 17.9 Hz)

Reference Production Example 24

To a mixture of 0.2 g of acetic acid and 7 ml of water was added 1.85 g of iron powder, and a suspension of 1.40 g of the 5-(5-(3,5-dichlorophenyl)-4 ,5-dihydro-5-trifluoromethyl-3-isozazolyl) -2-fluoronitrobenzene obtained in Reference Production Example 23 in 15 ml of ethanol was added thereto at 75°C and stirred at 75°C for 20 minutes. The reaction mixture was filtered and the filtrate was concentrated under reduced pressure. The resultant residue was subjected to silica gel column chromatography to obtain 806 mg of 5-(5-(3,5-dichlorophenyl)-4,5-dihydro-5-trifluoromethyl-3-isoxazolyl) -2-fluoroaniline.

5-(5-(3,5-Dichlorophenyl)-4,5-dihydro-5-trifluoromethyl-3-isoxasolyl)-2-fluoroaniline:

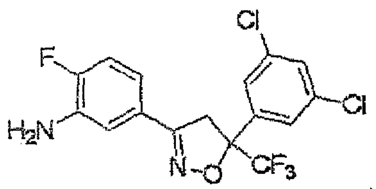

$^1$H-NMR ($CDCl_3$) δ: 7.50 (2H, d, J = 1.4 Hz), 7.42 (1H, t, J = 1.8 Hz) , 7.18 (1H, dd, J = 8.5, 2.2 Hz), 7.01 (1H, dd, J = 10.6, 8.5 Hz), 6.90-6.87 (1H, m), 4.03 (1H, d, J = 17.0 Hz), 3.85 (2H, br s), 3.64 (1H, d, J = 17.0 Fez) .

Reference Production Example 25

In 30 ml of N,N-dimethylformamide were dissolved 2.92 g of 2-chloro-5-hydroxyiminomethylnitrobenzene and 1.94 g of N-chlorosuccinimide, and the resulting solution was stirred at 60°c for 1 hour. The solution was cooled to room temperature and 3.50 g of 2-(3,5-dichlorophenyl)-3,3,3-trifluoro-1-propene and then 1.46 g of triethylamine were added thereto and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 4.42 g of 5-(5-(3,5-dichlorophenyl) -4,5-dihydro-5-trifluoromethyl-3-isoxazolyl) -2-chloronitrobenzene.

5-(5-(3,5-Dichlorophenyl)-4,5-dihydro-5-trifluoromethyl-3-isoxazolyl)-2-chloronitrobenzene:

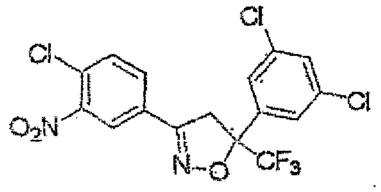

$^1$H.-NMR ($CDCl_3$) δ: 8.09 (1H d, J = 2.1 Hz), 7.89 (1H, dd, J = 8.5, 2.1 Hz), 7.65 (1H, d, J = 8.5 Hz), 7.50 (2H, d, J = 1.6 Hz), 7.45 (1H, t, J = 1.6 Hz), 4.09 (2H, d, J = 17.3 Hz), 3.71 (1H, d, J = 17.3 Hz).

Reference Production Example 26

To a mixture of 0.38 g of acetic acid, 15 ml of water and 30 ml of ethanol was added 3.46 g of iron powder, and 2.73 g of the 5- (5- (3, 5-dichlorophenyl) - 4, 5-dihydro-5-trifluoromethyl-3-isoxasolyl) -2-chloron-itrobenzene obtained in Reference Production Example 25 was added thereto at 75°C and stirred at 75°C for 50 minutes. The reaction mixture was filtered and the filtrate was concentrated under reduced pressure. The resultant residue was subjected to silica gel column chromatography to obtain 1.65 g of 5-(5- (3,5-dichlorophenyl) -4,5-dihydro-5-trifluoromethyl-3-isoxazolyl)-2-chloroaniline.

5 - (5- (3,5-Dichlorophenyl) -4,5-dihydro-5-trifluoromethyl-3-isoxazolyl)-2-chloroaniline:

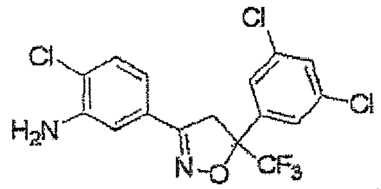

$^1$H-NMR ($CDCl_3$) δ: 7.49 (2H, d, J = 1.7 Hz), 7.42 (1H, t, J = 1.7 Hz), 7.29 (1H, d, J = 8.4 Hz), 7.13 (1H, d, J = 2.0 Hz), 6.89 (1H, dd, J = 8.4, 2.0 Hz), 4.18 (2H, br s), 4.03 (1H, d, J = 17.1 Hz) , 3.64 (1H, d, J = 16. 4 Hz).

Reference Production Example 27

In 40 ml of N,N-dimethylformamide were dissolved 4.30 g of 2-ethyl-5-hydroxyiminomethylnitrobenzene and 2.97 g of N-chlorosuccinimide, and the resulting- solution was stirred at 60°C for 1 hour. The reaction solution was cooled to room temperature and 3.5 g of 2-(3,5-dichlorophenyl)-3,3,3-trifluoro-1-propene and then 1.7 g of triethylamine were added thereto and stirred for 6 hours. Water was added to the reaction mixture, followed by extraction with ethyl acetate. The organic layer was dried over anhydrous sodium sulfate and then concentrated under reduced pressure. The resultant residue was subjected to silica gel column chromatography to obtain 5.74 g of 5-(5-(3,5-dichlorophenyl)-4,5-dihydro-5-trifluoromethyl-3-isoxazolyl)-2-ethylnitrobenzene.

5- (13,5-Dichlorophenyl) -4,5-dibydro-5-trifluoromethyl-3-isoxazolyl) -2-ethylnitrobenzene:

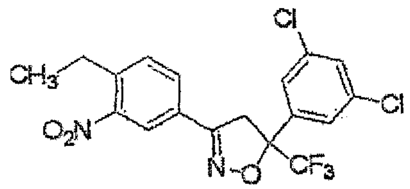

$^1$H-NMR ($CDC1_3$) δ: 8.07 (1H, d, J = 1.9 Hz) , 7.91 (1H, dd, J = 8.0, 1.9 Hz), 7.51 (2H, d, J = 1.6 Hz), 7.46 (1H, d, J = 8.0 Hz), '7.44 (1H, t, J = 1.6 Hz), 4.10 (1H, d, J = 17.3 Hz), 3.72 (1H, d, J = 17.3 Hz), 2.96 (2H, q, J = 7.5 Hz), 1.30 (3H, t, J = 7.5 Hz).

Reference Production Example 28

To a mixture of 0.5 m1 of acetic acid, 22 ml of water and 34 ml of ethanol was added 4.33 g of iron powder, and 5.60 g of the 5-(5-(3,5-dichlorophenyl)-4,5-dihydro-5-trifluoromethyl-3-isoxazolyl)-2-ethylnitrobenzene obtained in Reference Production Example 27 was added thereto at 75 °C and stirred for 3 hours. The reaction mixture was cooled to room temperature and filtered, and the precipitate was washed with ethyl acetate . A saturated aqueous sodium hydrogencarbonate solution and ethyl acetate were added to the filtrate to effect extraction. The organic layer was dried over anhydrous sodium sulfate and then concentrated under reduced pressure to obtain 5.20 g of 5 -(5 -(3,5 -dichlorophenyl)- 4,5 -dihydro-5 - trifluoromethyl- 3 -isoxazolyl)-2-ethylaniline.

5-(5-(3,5-Dichlorophenyl)-4,5-dihydro-5-trifluoromethyl-3-isoxazolyl)-2-ethylaniline:

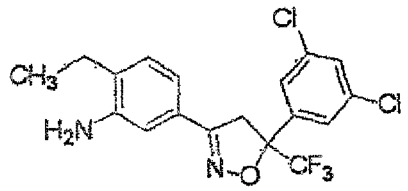

$^1$H-NMR ($CDCl_3$ ) δ: 7.51 (2H, d, J = 1.7 Hz), 7.41 (1H, t, J = 1.7 Hz), 7.10 (1H, d, J = 7.8 Hz), 7.04 (1H, d. J = 1.7 Hz) , 6.93 (1H, dd, J = 7.8, 1.7 Hz), 4.05 (1H, d, J = 17.1 Hz), 3.74 (2H, br s), 3.65 (1H, d, J = 17.1 Hz), 2.52 (2H, q, J = 7.5 Hz), 1.25 (3H, t, J = 7.5 Hz) .

Reference Production Example 29

To a mixture of 1.2 ml of acetic acid, 51 ml of water and 79 ml of ethanol was added 10.04 g of iron powder, and 5.44 g of 4-methoxy-3-nitrobenzaldehyde was added thereto at 75°C and stirred for 1 hour. The reaction mixture was filtered and the precipitate was washed with ethyl acetate. A saturated aqueous sodium hydrogencarbonate solution and ethyl acetate were added to the filtrate to effect extraction. The organic layer was dried over anhydrous sodium sulfate and then concentrated under reduced pressure to obtain 4.44 g of 3 -amino-4 -methoxybenzaldehyde.

3-Amino-4-methoxybenzaldehyde:

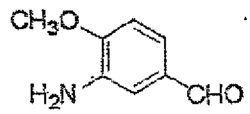

$^1$H-NMR ($CDC1_3$) δ: 9.80 (1H, s), 7.27 (1H, dd, J = 8.2, 2.0 Hz), 7.23 (1H, d, J = 2.0 Hz), 6.88 (1H, d, J = 8.2 Hz), 3.97 (2H, br s), 3.94 (3H, s).

Reference Production Example 30

In 3 ml of tetrahydrofuran was dissolved 302 mg of the 3-amino-4-methoxybenzaldehyde obtained in Reference Production Example 29, and 322 mg of 3,3,3-trifluoropropionyl chloride and then 223 mg of triethylamine were added thereto at room temperature and stirred for 15 minutes. The reaction mixture was concentrated under reduced pressure and the resultant residue was subjected to silica gel column chromatography to obtain 376 mg of N-(5-formyl-2-methoxyphenyl)-3,3,3-trifluoropropionylamide.

N- (5-Formyl-2-methoxyphenyl) -3,3,3-trifluoropropionylamide;

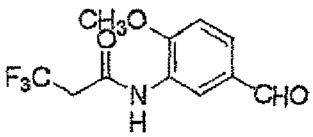

$^1$ H-NMR ($CDCl_3$) δ; 9.90 (1H, s), 8.86 (1H, d, J = 1.9 Hz), 8.05 (1H, br s), 7.71 (1H, dd, J = 8.5, 1.9 Hz) , 7.03 (1H, d, J = 8.5 Hz), 4.00 (3H, s), 3.31 (2H, q, J = 10.4 Hz).

Reference Production Example 31

In a mixture of 4.3 ml of ethanol and 2.2 ml of water were dissolved 370 mg of the N-(5-formyl-2-methoxyphenyl)-3,3,3-trifluoropropionylamide obtained in Reference Production Example 30, 131 mg of hydroxylamine hydrochloride and 174 mg of sodium acetate, and the resulting solution was stirred at room temperature for 30 minutes. The reaction mixture was added to water and extracted with ethyl acetate. The organic layer was dried over anhydrous sodium sulfate and then concentrated under reduced pressure to obtain 390 mg of N-(5-hydroxyliminomethyl-2-methoxyphenyl)-3,3,3-trifluoropropionylamide.

N-(5-hydroxyliminomethyl-2-methoxyphenyl)-3,3,3-trifluoropropionylamide:

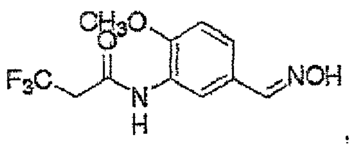

Next, formulation examples are described below. In the formulation examples, parts are all by weight.

Formulation Example 1

10% Emulsifiable concentrates of each of the present compounds (1) to (79) are obtained by dissolving 10

parts of each of the present compounds in a mixture of 35 parts of xylene and 35 parts of N,N-dimethylformamide, adding thereto 14 parts of polyoxyethylene styryl phenyl ether and 6 parts of calcium dodecylbenzenesulfonate, and thoroughly stirring and mixing the resultant mixture.

Formulation Example 2

20% Wettable powders of each of the present compounds (1) to (79) are obtained by adding 20 parts of each of the present compounds to a mixture of 4 parts of sodium lauryl sulfate, 2 parts of calcium lignosulfonate, 20 parts of fine powder of synthetic hydrated silicon dioxide and 54 parts of diatomaceous earth, and thoroughly stirring and mixing the resultant mixture.

Formulation Example 3

1 Part of fine powder of synthetic hydrated silicon dioxide, 2 parts of calcium lignosulfonate, 30 parts of bentonite and 65 parts of kaolin clay are added to 2 parts of each of the present compounds (1) to (79). The resultant mixture is thoroughly stirred and mixed. Thereafter, an appropriate quantity of water is added to the resulting mixture and further stirred. The thus stirred mixture is subjected to particle size regulation with a granulator and then through-flow drying, to obtain 2% granules of each of the present compounds.

Formulation Example 4

1% Dusts of each of the present compounds (1) to (79) are obtained by dissolving 1 part of each of the present compounds in an appropriate quantity of acetone, adding thereto 5 parts of fine powder of synthetic hydrated silicon dioxide, 0.3 part of PAP and 93.7 parts of fubasami clay, thoroughly stirring and mixing the resultant mixture, and then removing the acetone by evaporation.

Formulation Example 5

10% Flowable concentrates of each of the present compounds (1) to (79) are obtained by mixing 10 parts of each of the present compounds, 35 parts of white carbon containing 50 parts of polyoxyethylene alkyl ether sulfate ammonium salt, and 55 parts of water, and finely grinding the resultant mixture by a wet grinding method.

Formulation Example 6

0,1% Oil formulations of each of the present compounds (1) to (79) are obtained by dissolving 0.1 part of each of the present compounds in a mixture of 5 parts of xylene and 5 parts of trichloroethane and mixing the resulting solution with 89.9 parts of deodorized kerosene.

Formulation Example 7

Ten milligrams of each of the present compounds (1) to (79) is dissolved in 0.5 ml of acetone. The resulting solution is uniformly mixed with 5 g- of solid feed powder for animals (solid feed powder for breeding CE-2, a trade name, Oriental Kobo Co.). The resulting mixture was dried to remove the acetone by evaporation, to obtain poisonous baits of each of the present compounds.

Formulation Example 8

Mixed wettable powders are obtained by adding 10 parts of each of the present compounds (1) to (79) to a mixture of 10 parts of any of other agents for controlling pests, such as insecticides, acaricides, nematicides or fungicides, plant hormone preparations, plant growth regulators and herbicides (these agents include their isomers and salts), synergists and phytotoxity-reducing agents, 4 parts of sodium lauryl sulfate, 2 parts of calcium lignosulfonate, 20 parts of fine powder of synthetic hydrated silicon dioxide and 54 parts of diatomaceous earth, and thoroughly stirring and mixing the resultant mixture.

The following test example demonstrates the controlling effect of the present compounds on pests.

Test Example 1

Each of the formulations of the present compounds (1) to (3), (5), (7), (13), (15), (17), (19) to (25), (30), (31),

(33) to (37), (43) to (45), (48), (57), (60), (64), (71), (72), (75) to (77) and (78), obtained according to Formulation Examples 5 was diluted with water so as to have an active ingredient concentration of 500 ppm. Thus, test spray liquids were prepared.

On the other hand, each cabbage plant was planted in a polyethylene cup and grown until its third true leaf or fourth true leaf was developed. Then, each of the above-mentioned test spray liquids was sprayed on the cabbage plant in a volume of 20 ml per cup.

After the liquid chemical sprayed on the cabbage plant dried up, 10 third-instar larvae of diamondback moth (Plutella xylostella) were made parasitic on the cabbage plant. After 5 days, diamondback moths on the cabbage plant were counted and the control efficacy was calculated by the following equation:

$$\text{Control efficacy (\%)} = \{1 - (Cb \times Tai) / (Cai \times Tb)\} \times 100$$

In the equation, the symbols have the following meanings:

Cb: Number of insects before spraying in untreated group,
Cai: Number of insects after spraying in untreated group,
Tb: Number of insects before spraying in treated group,
Tai: Number of insects after spraying in treated group.

As a result, it was found, that the present compounds (1) to (3), (5), (7), (13), (15), (17), (19) to (25), (30), (31), (33) to (37), (43) to (45), (48), (57), (60), (64), (71), (72), (75) to (77) and (78) had a control efficacy of 80% or more in the groups treated with the test spray liquids, respectively.

Test Example 2

The formulation of the present compound,(79) obtained according to Formulation Example 5 was diluted with water so as to have an active ingredient concentration of 2,500 ppm. Thus, a test dilution was prepared.

The bottom of a polyethylene cup having a diameter of 5.5 cm was covered with a filter paper having the same diameter. Artificial feed (Silkmate 2S; Nihon Nosan Kogyo K.K.) sliced in a thickness of 2 mm was placed on the filter paper, and 1 mL of the above-mentioned test dilution was irrigated on the artificial feed. After air-drying of the test dilution, a filter paper having a diameter of 5.5 cm was placed on the artificial feed, and 30 first-instar larvae of summer fruit tortrix (Adoxophyes orana) were released on this filter paper, after which the cup was closed with a lid. After 7 days, larvae of summer fruit tortrix were counted and the mortality was calculated by the following equation:

$$\text{Mortality (\%)} = (\text{Number of dead larvae} / \text{Number of released larvae}) \times 100$$

As a result, the mortality in a group treated with the test dilution of the present compound (79) was found to be 80% or more.

In addition, the compound identified by compound number 1-2 and described in International Publication No. WO2005-51932 pamphlet:

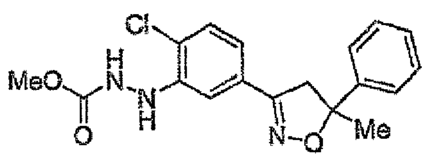

was tested in the same manner as in Test Example 1 and Test Example 2 to find that said compound had a control

efficacy of 0% in Test Example 1 and had a mortality of 0% in Test Example 2.

## Claims

1. An isoxazoline compound represented by the formula (1):

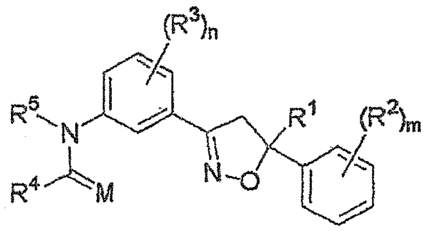

(1)

wherein $R^2$ is a $C_1$-$C_4$ haloalkyl group,
$R^2$is a $_{C1}$-$C_6$ alkyl group unsubstituted or substituted with one or more halogen atoms, a $C_1$-$C_6$ alkoxy group unsubstituted or substituted with one or more halogen atoms, a halogen atom, a $C_1$-$C_6$ alkylsulfenyl group, a $C_1$-$C_6$ alkylsulfinyl group, a $C_1$-$C_6$ alkylsulfonyl group, a nitro group or a cyano group,
$R^3$ is a $C_1$-$C_6$ alkoxy group,
m is an integer of 0 to 5,
n is 1,
M is an oxygen atom or a sulfur atom,
$R^4$ is hydrogen atom; a $C_1$-$C_{12}$ alkyl group unsubstituted or substituted with one or more halogen atoms; a ($C_2$-$C_5$ alkoxycarbonyl) $C_1$-$C_2$ alkyl group; a $C_2$-$C_{12}$ cyanoalkyl group; a $C_3$-$C_{12}$ cycloalkyl group unsubstituted or substituted with one or more halogen atoms; a ($C_3$-$C_6$ cycloalkyl)$C_1$-$C_6$ alkyl group; a ($C_1$-$C_6$ alkoxy)$C_2$-$C_6$ alkyl group; a $C_2$-$C_{12}$ alkenyl group unsubstituted or substituted with one or more halogen atoms; a $C_2$-$C_{12}$ alkynyl group unsubstituted or substituted with one or more halogen atoms; a $C_1$-$C_6$ alkoxy group unsubstituted or substituted with one or more halogen atoms; a ($C_1$-$C_6$ alkoxy) $C_1$-$C_6$; alkoxy group; a $C_3$-$C_6$ alkenyloxy group unsubstituted or substituted with one or more halogen atoms; a benzyloxy group; a phenyl($C_2$-$C_6$)alkenyl group; a ($C_1$-$C_6$ alkylamino)$C_1$-$C_6$ alkyl group; a (di($c_1$-$C_6$ alkyl)amino)$C_1$-$C_6$ alkyl group; a group represented by the following formula:

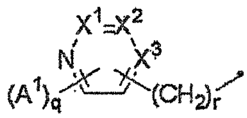

[wherein $A^1$ is a $C_1$-$C_4$ alkyl group unsubstituted or substituted with one or more halogen atoms, a $C_1$-$C_4$ alkoxy group unsubstituted or substituted with one or more halogen atoms, a halogen atom or a cyano group,
q is an integer of 0 to 3,
r is an integer of 0 to 2, and
$X^1$, $X^2$ and $X^3$ represent a combination of one nitrogen atom and two CH groups, or a combination of three CH groups]; a group represented by the following formula:

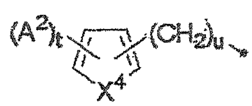

[wherein $_A{}^2$ is a $C_1$-$C_4$ alkyl group, a halogen atom or a nitro group,
t is an integer of 0 to 3,

u is an integer of 0 to 2, and
$X^4$ is an oxygen atom, a sulfur atom or NH]; a group represented by the following formula:

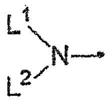

[wherein $L^1$is a C1-$C_6$ alkyl group unsubstituted or substituted with one or more halogen atoms, or a $C_3$-$C_8$ cycloalkyl group, and
$L^2$ is a hydrogen atom or a $C_1$-$C_6$ alkyl group unsubstituted or substituted with one or more halogen atoms, or $L^1$ and $L^2$, when taken together, represent a $C_2$-$C_9$ alkanediyl group, a $CH_2CH_2CH(CH_3)CH_2CH_2$ group or a $CH_2CH_2OCH_2CH_2$ group] ; a phenyl group; a phenyl ($C_1$.-$C_4$)alkyl group; a phenoxy group; a phenoxy($C_1$-$C_4$) alkyl group; a phenyl amino group or a phenyl ($C_1$-$C_2$)alkylamino group, wherein the benzene ring of any of said phenyl group, said phenyl ($C_1$-$C_4$) alkyl group, said phenoxy group, said phenoxy ($C_1$-$C_4$) alkyl group, said phenylamino group and said phenyl ($C_1$-$C_2$)alkylamino group may be substituted with 1 to 5 groups independently selected from the group consisting of $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups and benzoyl group, and
$R^5$ is a hydrogen atom, a $C_1$-$C_{12}$ alkyl group, a $C_3$-$C_{12}$ cycloalkyl group, a ($C_3$-$C_6$ cycloalkyl) $C_1$-$C_6$ alkyl group, a $C_2$-$C_6$ alkoxyalkyl group, a $C_3$-$C_{12}$ alkenyl group, a $C_3$-$C_{12}$ alkynyl group, a $C_1$-$C_6$ acyl group, a $C_2$-$C_8$ cyanoalkyl group, a nitromethyl group, a $C_2$-$C_6$ alkoxycarbonyl group, a $C_3$-$C_8$ alkoxycarbonylalkyl group, a phenyl ($C_1$-$C_4$) alkyl group or a benzoyl group, wherein the benzene ring of said phenyl ($C_1$-$C_4$) alkyl group or said benzoyl group may be substituted with 1 to 5 groups independently selected from the group consisting of $C_1$-$C_6$alkyl groups unsubstituted or substituted with one or more halogen atoms, $C_1$-$C_6$ alkoxy groups unsubstituted or substituted with one or more halogen atoms, halogen atoms, nitro group and cyano group.

**2.** The isoxazoline compound according to claim 1, wherein in the formula (1) , n is 1 and $R^3$ is a $C_1$-$C_4$ alkoxy group unsubstituted or substituted with one or more halogen atoms which is a substituent at the 6-position.

**3.** The isoxazoline compound according to any one of claims 1 to 2, wherein in the formula (1), $R^4$ is a $C_1$-$C_{12}$ alkyl group unsubstituted or substituted with one or more halogen atoms .

**4.** The isoxazoline compound according to any one of claims 1 to 2, wherein in the formula (1), $R^4$ is a $C_2$-$C_{12}$ alkenyl group unsubstituted or substituted with one or more halogen atoms.

**5.** The isoxazoline compound according to any one of claims 1 to 2, wherein in the formula (1), $R^4$ is a $C_1$-$C_6$ alkoxy group unsubstituted or substituted with one or more halogen atoms.

**6.** The isoxazoline compound according to any one of claims 1 to 2 , wherein in the formula (1), $R^4$ is a phenyl group unsubstituted or substituted with 1 to 5 groups independently selected from the following group X:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfenyl groups, $C_1$-$C_6$ alkylsulfenyl groups, $C_2$-$C_6$ alkoxycarbonyl groups and benzoyl groups.

**7.** The isoxazoline compound according to claim 1, wherein in the formula (1), $R^1$ is a trifluoromethyl group, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atom.

**8.** The isoxazoline compound according to claim 1, wherein in the formula (1), $R^1$ is a trifluoromethyl group, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfenyl groups, $C_1$-$C_6$ alkylsulfinyl groups, $C_1$-$C_6$ alkylsulfonyl groups, $C_2$-$C_6$ alkoxycarbonyl groups benzyl group.

**9.** The isoxazoline compound according to claim 1, wherein in the formula (1), $R^1$ is a trifluoromethyl group, m is 2, $R^2$s are chlorine atoms as substituents at the 3-position and 5-position, respectively, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atoms.

**10.** The isoxazoline compound according to claim 1, wherein in the formula (1), $R^1$ is a trifluoromethyl group, m is 2, $R^2$S are chlorine atoms as substituents at the 3-position and 5-position, respectively, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfonyl groups, $C_1$-$C_6$ alkylsulfinyl groups, $C_1$-$C_6$ alkylsulfonyl groups, $C_2$-$C_6$ alkoxycarbonyl groups and benzoyl group.

**11.** The isoxazoline compound according to claim 1, wherein in the formula (1), $R^1$ is a trifluoromethyl group, n is 0 or 1, $R^4$ is a hydrogen atom, a $C_1$-$C_{12}$ alkyl group unsubstituted or substituted with one or more halogen atoms, a $C_2$-$C_{12}$ alkenyl group unsubstituted or substituted with one or more halogen atoms, or a $C_1$-$C_6$ alkoxy group unsubstituted or substituted with one or more halogen atoms, and $R^5$ is a hydrogen atoms.

**12.** The isoxazoline compound according to claim 1, whereon in the formula (1), $R^1$ is a trifluoromethyl group, n is 0 or 1, $R^4$ is a phenyl group unsubstituted or substituted with 1 to 5 groups independently selected from the following group X, and $R^5$ is a hydrogen atom:

group X: $C_1$-$C_6$ alkyl groups unsubstituted or substituted with one or more halogen atoms, $C_1$-$C_6$ alkoxy groups unsubstituted or substituted with one or more halogen atoms, halogen atoms, nitro group, cyano group, $C_1$-$C_6$ alkylsulfonyl groups, $C_1$-$C_6$ alkylsulfinyl groups, $C_1$-$C_6$ alkylsulfonyl groups, $C_2$-$C_6$ alkoxycarbonyl groups and benzoyl group.

**13.** A composition for controlling pests, which comprises an isoxazoline compound according to any one of claims 1 to 12 as an active ingredient.

**14.** A method for controlling pests, which comprises applying an effective amount of an isoxazoline compound according to any one of claims 1 to 12 to the pests or a locus where the pests inhabit.

**15.** Use of an isoxazoline compound according to any one of claims 1 to 12 for controlling pests.

**16.** Use of an isoxazoline compound according to any one of claims 1 to 12 for producing a composition for controlling pests.

Europäisches Patentamt
European Patent Office
Office européen des brevets

# EUROPEAN SEARCH REPORT

Application Number
EP 10 19 5739

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/066617 A1 (MITA TAKESHI [JP] ET AL) 22 March 2007 (2007-03-22)<br>* pages 100,107; compounds 5-333,5-336,5-337,5-682 *<br>----- | 1-16 | INV.<br>C07D261/04<br>C07D413/12<br>A01N43/80<br>A01P7/02 |
| X | WO 2007/026965 A (NISSAN CHEMICAL IND LTD [JP]; MITA TAKESHI [JP]; FURUKAWA YUKI [JP]; T) 8 March 2007 (2007-03-08)<br>* abstract; compound ALL *<br>----- | 1-16 | |
| X,P | WO 2007/075459 A (DU PONT [US]; LAHM GEORGE PHILIP [US]; PATEL KANU MAGANBHAI [US]; PAHU) 5 July 2007 (2007-07-05)<br>* claim 1; compound ALL *<br>----- | 1-16 | |
| X,P | WO 2007/105814 A (NISSAN CHEMICAL IND LTD [JP]; MITA TAKESHI [JP]; MAEDA KAZUSHIGE [JP];) 20 September 2007 (2007-09-20)<br>* abstract; compound ALL *<br>----- | 1-16 | |
| X,P | WO 2008/019760 A (BAYER CROPSCIENCE AG [DE]; MIHARA JUN [JP]; MURATA TETSUYA [JP]; YAMAZ) 21 February 2008 (2008-02-21)<br>* claim 1; compound ALL *<br>----- | 1-16 | TECHNICAL FIELDS SEARCHED (IPC)<br>C07D<br>A01N<br>A01P |

The present search report has been drawn up for all claims

1

| Place of search | Date of completion of the search | Examiner |
|---|---|---|
| Munich | 1 March 2011 | Bareyt, Sébastian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 19 5739

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2011

| Patent document cited in search report | Publication date | Patent family member(s) | Publication date |
|---|---|---|---|
| US 2007066617 A1 | 22-03-2007 | AU 2005219788 A1 | 15-09-2005 |
| | | AU 2010212450 A1 | 09-09-2010 |
| | | BR PI0508140 A | 24-07-2007 |
| | | CA 2558848 A1 | 15-09-2005 |
| | | CN 101768129 A | 07-07-2010 |
| | | EA 200601639 A1 | 27-02-2007 |
| | | EA 200900197 A1 | 30-06-2009 |
| | | EP 1731512 A1 | 13-12-2006 |
| | | WO 2005085216 A1 | 15-09-2005 |
| | | KR 20070010145 A | 22-01-2007 |
| | | US 2009312330 A1 | 17-12-2009 |
| WO 2007026965 A | 08-03-2007 | AU 2006285613 A1 | 08-03-2007 |
| | | BR PI0617076 A2 | 04-08-2009 |
| | | CA 2621228 A1 | 08-03-2007 |
| | | EP 1932836 A1 | 18-06-2008 |
| | | KR 20080049091 A | 03-06-2008 |
| WO 2007075459 A | 05-07-2007 | AR 058358 A1 | 30-01-2008 |
| | | AU 2006329856 A1 | 05-07-2007 |
| | | CA 2626839 A1 | 05-07-2007 |
| | | EP 1966195 A2 | 10-09-2008 |
| | | JP 2009519953 T | 21-05-2009 |
| | | KR 20080080189 A | 02-09-2008 |
| | | NZ 567427 A | 24-12-2010 |
| | | US 2009133319 A1 | 28-05-2009 |
| | | UY 30013 A1 | 31-07-2007 |
| WO 2007105814 A | 20-09-2007 | AT 466846 T | 15-05-2010 |
| | | CN 101400662 A | 01-04-2009 |
| | | EP 1997813 A1 | 03-12-2008 |
| | | ES 2344027 T3 | 16-08-2010 |
| | | KR 20080110610 A | 18-12-2008 |
| | | US 2009156643 A1 | 18-06-2009 |
| WO 2008019760 A | 21-02-2008 | AR 062358 A1 | 05-11-2008 |
| | | AU 2007283819 A1 | 21-02-2008 |
| | | CA 2660576 A1 | 21-02-2008 |
| | | CL 23502007 A1 | 14-03-2008 |
| | | CN 101522672 A | 02-09-2009 |
| | | CR 10611 A | 30-06-2009 |
| | | EP 2069338 A1 | 17-06-2009 |
| | | JP 2008044880 A | 28-02-2008 |
| | | JP 2010500981 T | 14-01-2010 |
| | | KR 20090040469 A | 24-04-2009 |
| | | PE 05452008 A1 | 13-07-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 10 19 5739

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2011

| Patent document cited in search report | Publication date | Patent family member(s) | Publication date |
|---|---|---|---|
| WO 2008019760 A | | RU 2009109160 A | 27-09-2010 |
| | | SV 2009003170 A | 19-01-2010 |
| | | US 2010249191 A1 | 30-09-2010 |
| | | UY 30540 A1 | 31-03-2008 |
| | | ZA 200901033 A | 28-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200551932 A
- EP 0374753 A
- WO 930727 B
- WO 9534656 A
- EP 0427529 A
- EP 451878 A
- WO 03052073 A
- EP 0392225 A
- WO 9533818 A
- EP 0353191 A
- WO 03000906 A